# EUROPEAN PATENT APPLICATION

(11) **EP 4 431 090 A1**
(43) Date of publication of application: **18.09.2024**
(21) Application number: 23162131.9
(22) Date of filing: 15.03.2023
(51) Int. Cl.: A61K 31/18, A61K 31/277, A61K 31/4418, A61K 31/445, A61K 31/10, A61P 11/00, A61P 19/00, A61P 19/02, A61P 29/00, A61P 35/00, A61P 37/00, A61P 43/00

(54) **SOLFONAMIDES ACTING AS MITOCHONDRIAL COMPLEX I MODULATOR COMPOUNDS**

(71) Applicant: Istesso 1 Limited, London N1C 4AG (GB)
(72) Inventor: FOSTER, Martyn Leslie, Essex, SS7 5JQ (GB); MAZANETZ, Michael Philip, Glasgow, G43 2NX (GB); PATEL, Lisa, London, N1C 4AG (GB); VINK, Paul, Nijmegen (NL)
(74) Representative: Mewburn Ellis LLP

(57) **Abstract**

The invention provides mitochondrial complex I modulator (MCIM) compounds, which find utility in the medical field of inflammatory and progressive disorders such as IBD, idiopathic pulmonary fibrosis, interstitial lung disease, MS and RA.

## Description

### Field of the Invention

The present invention relates to a class of therapeutic agents that can have reparative effects on a range of progressive and/or degenerative diseases. In particular instances, the therapeutic agents of the invention can act by binding mitochondrial Complex I. Collectively, the therapeutic agents of the invention are referred to herein as Mitochondrial Complex I Modulator compounds (or MCIM compounds).

### Background

Mitochondrial Complex I (also known as NADH:ubiquinone oxidoreductase, Type I NADH dehydrogenase, respiratory Complex I, or simply 'Complex I') is the first enzyme complex of the respiratory chain (Yoga *etal,* 2021, which is hereby incorporated by reference in its entirety). Complex I is a very large protein complex comprising 45 subunits (Gutiérrez-Fernández, 2020) which is highly conserved in eukaryotes and prokaryotes. In eukaryotes, it couples the transfer of electrons from NADH to the coenzyme ubiquinone (Q) with the translocation of protons across the inner mitochondrial membrane, although the mechanism linking the spatially distinct proton translocation and electron transfer remains unknown (Gutiérrez-Fernández, 2020).

In humans, the most common form of ubiquinone is known as `Q10' because it has ten isoprenyl subunits in its `tail' region. Q10 is hydrophobic and it enters the Complex I enzyme from the mitochondrial inner membrane through a long binding channel (Bridges *etal,* 2020), which is often called the `Q tunnel'. The Q-tunnel is long and heterogenous in nature. Various compounds are known to bind within the Q tunnel, but there is no single consensus site for compound binding. For example, piericidin A is reported to bind within the Q-tunnel as a `short-form' ubiquinone, with interactions at multiple residues from the top of the Q-tunnel to its midway point (Gutiérrez-Fernández, 2020; Bridges *etal,* 2020; Chung *et al,* 2021, each of which is hereby incorporated by reference in its entirety). Aureothin and pyridaben, which are also quinone-like compounds, are also observed to bind at a similar site in *T. thermophilus* (Gutiérrez-Fernández, 2020; Chung *et al,* 2021). IACS-2858 and BAY-87-2243 act like a "cork in a bottle", binding a cluster of residues of subunits ND1 and NDUFS7 in the central charged region of the ubiquinone-binding pocket of mouse Complex I (Chung *et al,* 2021; Kurelac *et al,* 2022). Biguanides such as metformin are similarly thought to interact with Phe244 of ND1 and Arg77 of NDUFS7 in the Q-tunnel with a mode dependent on the active or inactive state of the enzyme. Furthermore, evidence indicates that the biguanides may be non-selective in their binding, with a putative additional binding site on Complex IV, another subunit of the electron transport chain (LaMoia *et al,* 2021). The binding sites of several Complex I inhibitors are reviewed by Schiller and Zickermann (2022).

Classical Complex I inhibitors, such as those mentioned above, often cause cytotoxicity and cell death. For instance, piercidin A is insecticidal and antibacterial, pyridaben is an acaricide (killing ticks and mites) and aureothin exhibits antitumor, antifungal and insecticidal activity. IACS-010579 and IM156 (also known as HL156A) have been reported to possess anti-tumour effects due to profound impacts on cancer cell viability (Tsogbadrakh *et al,* 2018 and Izreig *et al,* 2020). However, despite these properties, the known, classical, C1 inhibitors have not found use as approved therapeutics. Indeed, as well as showing mechanism-based toxicity (Yap, T *et* al, 2023), these agents have been used to elicit disease in mouse models of neurodegenerative conditions such as Parkinson disease (PD; Xiong *et al,* 2012, which is hereby incorporated by reference in its entirety) and Alzheimer's disease (AD; Joh *et al,* 2017, which is hereby incorporated by reference in its entirety). As such, for classical complex I inhibitors, identifying a suitable approach for therapeutic use that provides a benefit without toxicity has proved to be a challenge. An alternative approach which might elicit a benefit without adverse effects may have potential benefits for the treatment of a variety of progressive diseases.

***

The goal of therapeutic tissue repair is to restore the tissue to its original state of structure and function (Krafts, 2010 and Paul and Sharma, 2021). Attaining this goal has proved elusive, with the only effective examples being organ transplantation or surgical implants using natural or biomimetic structures such as aortic valves or joint replacement). Further, whilst there has been a significant increase in research into strategies to achieve repair in different organ systems, these approaches aim to remove the primary driver to tissue injury (e.g., the calcified heart valve) or to replace the dysfunctional matrix environment with an environment which favours homing of repair cells or local augmentation of soluble pro-repair factors (e.g., fibrin). Such examples include biomimetic scaffolds in orthopaedics, which simulate normal structural matrix for cell homing, and cellular products to simulate the soluble matrix microenvironment for cutaneous and ocular wound repair. As such, the identification of a therapeutic agent(s) which both attenuates the drive to tissue injury and concurrently promotes a pro-repair microenvironment, has proved elusive.

Tissue repair (healing) is a highly orchestrated and complex process involving a sequence of overlapping events that are precisely timed. The process has three broad phases which were first delineated in the 19^{th} Century (Virchow, 1859) and have been since augmented by the addition of data on their cellular genotypes, phenotypes, and molecular mediators (Liehn, 2011, Takeo, 2015, and Somer *et al,* 2021). However, there are limited, if any, data available regarding the mechanisms underpinning repair in most chronic disease settings (Peyrin-Biroulet, 2020) and current understanding of tissue repair processes has not led to substantial improvements in the clinical care of tissue damage (Eming *et al,* 2014).

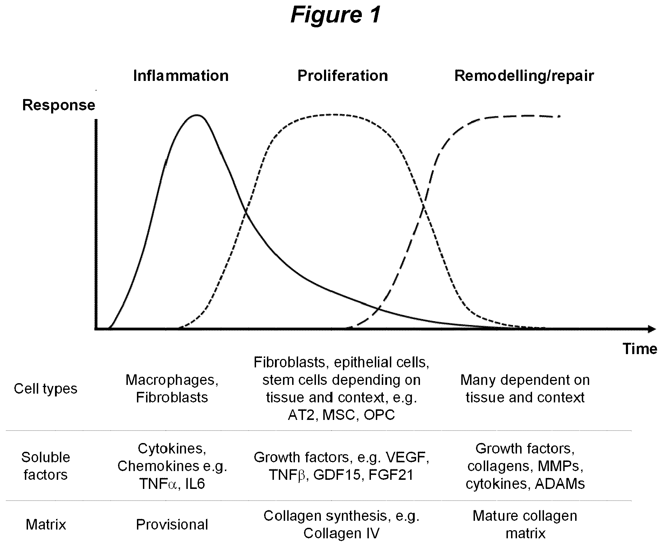

As shown in Figure 1, the three phases of tissue repair are:
1. Inflammation
2. Proliferation (fibrogenesis and angiogenesis)
3. Tissue Remodelling
   (Lokmic *et al,* 2012.)

In the healthy, functional healing/repair process, these phases proceed in an overlapping sequence, with resolution of inflammation progressing alongside proliferation and matrix remodelling to restore tissue architecture. In conditions of chronic disease, this process is interrupted or dysregulated, resulting in persistent non-healing tissue damage.

Functional repair involves the resolution of the primary inflammatory response to injury and the simultaneous activation of the inflammatory response consequent upon repair. These phases show different qualitative and kinetic properties; the first phase induces a proliferative response in both infiltrating and resident cells whilst the second involves repolarisation of the infiltrating cells. Furthermore, the cells involved in the repair process show different responses to stress dependent on the progressing phase of repair. For example, immune cells of the myeloid lineage (short-lived cells with high bioenergetic demand) respond to stress by reducing proliferation and activating apoptosis pathways such that the overall phenotype of the myeloid lineage is anti-inflammatory, and in the context of repair, a reduced population showing immunomodulatory effects. By contrast, mesenchymal and epithelial cells respond to similar microenvironmental stressors by activating effector pathways. For example, mesenchymal cells typically respond to low oxygen tension (hypoxia) by the expansion and activation of a repair phenotype.

There is an urgent need for therapeutics which exert a dual pharmacology of reducing disease progression whilst supporting tissue repair in chronic progressive disease, medical conditions that worsen over time without medical intervention. Prominent examples are autoimmune diseases such as rheumatoid arthritis (RA), psoriasis and inflammatory bowel disease (IBD) and progressive fibrosis such as idiopathic pulmonary fibrosis (IPF), non-alcoholic fatty liver disease (NAFLD)/ non-alcoholic steatohepatitis (NASH) and chronic kidney disease. Without treatment these conditions typically progress in severity over time and may, as in the case of pulmonary fibrosis, be fatal.

The current treatments for these conditions inhibit the progression of the diseases. However, in general these agents typically fail to promote tissue repair by exerting a direct effector response on repair processes. Indeed, their suppression of the pro-inflammatory effector cell and molecular response proceeds cyclically, perhaps for months or years. For example, in rheumatoid arthritis, persistent inflammation within the bone erosion prevents erosions from healing (Berardi *et al,* 2021) whilst a similar unresolved inflammation preventing repair is seen in chronic skin wounds (Li *et al,* 2021), ulcerative colitis and Crohn's disease. Examples of conditions where effective anti-inflammatory effects by a therapeutic do not result in effective repair include:
- rheumatoid arthritis, in which suppression of synovitis and pannus formation does not result in complete suppression of erosions and bone loss;
- inflammatory bowel disease, in which suppression of mucosal inflammation does not result in complete suppression of ulceration;
- neurodegeneration, such as multiple sclerosis (MS), in which suppression of the inflammatory drive to de-myelination does not result in concomitant increase in oligodendrocyte or Schwann cell-induced re-myelination;
- pathology of the distal lungs, such as idiopathic pulmonary fibrosis, in which suppression of inflammation does not halt the drive to fibroblastic expansion and emphysematous destruction, or result in the conservation of alveolar stem cells.

Furthermore, in other chronic diseases, the kinetic interplay between the drive to injury and sequential activation of repair, often results in 'futile' repair, i.e., creation of maladapted restitution of tissue remodelling to a normal state. Such futile repair is seen in settings such as osteoarthritis and fibrosis.

The result of either failure to repair or futile repair is that most patients with chronic disease experience ongoing symptoms, and disease progression despite treatment. As such, there is a pressing need for therapeutics with a dual pharmacology that can act on both the inflammatory and repair arms of a lesion. Such a therapeutic would:
- display a pharmacology-dependent, differential transduction of microenvironmental stress signals.
- mitigate the drive to injury and simultaneously, augment and capitalise upon the stressor events that constitute the inflammatory to proliferative phase of tissue repair.
- orchestrate repair, in an anatomically appropriate manner *to restore key aspects of tissue function* (Eming *et al,* 2014).

In addition, the pharmacological intervention might elicit cellular changes consistent with those required to orchestrate a controlled repair response. For example, the approach might elicit production of the key basement membrane collagen IV in a controlled manner, alongside production of growth factors important for angiogenesis, epithelialisation and matrix remodelling, such as VEGF, FGF21 and GDF15. Were this approach to be successful it would elicit repair in a pathology agnostic manner, i.e. the repair response would be seen in multiple settings regardless of the nature of the original injury. In addition, it might alter the activation response of resident cells and de-sensitize' the microenvironment to the effects of a pro-inflammatory cell infiltrate, resulting in a 'permission' to repair.

One proposed approach to eliciting a tissue repair response has been to repair the cell first (Fu, 2021). The induction of an integrated stress response (ISR) may be an effective means of achieving this goal. The ISR is a cytoprotective mechanism that maintains cellular proteostasis (i.e., protein homoeostasis) in response to stress conditions. The ISR is highly conserved across cell types, and is triggered in response to changes in mitochondrial function (Savu and Moisoi, 2022). Activation of a controlled ISR has been shown to have beneficial effects in multiple disease settings. For example, in mouse models of multiple sclerosis the ISR can be harnessed to protect oligodendrocytes and myelin during inflammation (Way and Popko, 2016). In addition, the ISR has been shown to regulate the health of cardiac progenitor cells by removing unhealthy cells to prevent their differentiation and self-renewal (Searfoss *et al,* 2019), a property that may be shared across progenitor cells in other settings such as oligodendrocyte progenitors in the brain and spinal cord, alveolar type II epithelial cells (AT2) in the lung and mesenchymal stem cells and bone marrow progenitor cells. Roles for the ISR in obesity, neurodegeneration and heart failure have also been proposed. Importantly, to achieve these outcomes the ISR should be moderate and tightly regulated, like a rheostat, in order to avoid pushing cells towards apoptosis (Kaspar *et al,* 2021).

An agent which could both control symptoms and elicit tissue repair/healing may have greater benefits for patients than existing therapy and lead to improved treatment outcomes.

The present invention has been devised in light of the above considerations.

### Summary of the Invention

Current treatments for chronic, progressive conditions inhibit disease progression but fail to promote tissue repair. Consequently, patients with chronic disease experience ongoing symptoms and progression, and poor quality of life. Despite the attractiveness of restoring normal tissue architecture as a means to treat chronic disease, pharmacological interventions to achieve this have not been studied, and as a result practical applications of this approach do not yet exist. One potential approach to achieve pharmacological tissue repair is through changes in mitochondrial function. However, the literature currently teaches that eliciting changes in mitochondrial function suppresses repair and promotes inflammation (Cai *et al,* 2022), and indeed, alterations in mitochondrial function to alter disease progression through control of inflammation, or tissue remodelling, have not been extensively studied. The mitochondrial Complex I modulator (MCIM) compounds of this invention modulate the activity of mitochondrial Complex I in a manner that differs from that of conventional complex I inhibitors. This elicits an adaptive phase which directs cell fate choices and mimics a wound repair-like microenvironment. Phenotypically, this can control inflammation, alter the activation response of resident cells and desensitizes the microenvironment to the effects of a pro-inflammatory cell infiltrate, and concurrently initiate repair signals in affected tissues, such as the lung and joint. In addition, the compounds can stimulate the production of key growth factors such as VEGF, and the basement collagen IV. Together, these mechanisms support a reduction in inflammation and restoration of tissue architecture in multiple organ/tissue settings. Thus, the invention provides a multi-organ applicable approach to eliciting tissue repair in humans.

At its broadest, the invention provides MCIM compounds that can achieve reparative effects when used for treating inflammatory and/or progressive diseases. Preferably, the compounds bind Complex I and are able to modulate Complex I function.

Accordingly, in a first aspect, the invention provides a mitochondrial Complex I modulator (MCIM) compound for use in treating inflammatory and/or progressive diseases, wherein the treatment comprises administering the compound to the subject. In some embodiments, the treatment elicits tissue repair and/or disease control or regression. In some embodiments, the treatment achieves tissue repair and/or disease control or regression. In some embodiments, the treatment initiates an adaptive response in certain cell types that leads to pharmacodynamic evidence of tissue repair and/or disease control or regression. In some embodiments, the adaptive response leads to tissue repair and/or disease regression. In some embodiments, the tissue repair and/or disease regression induces a restoration of tissue architecture towards its healthy state, which is characterised by anatomically normal architecture. In a related aspect, the invention provides methods of treating an inflammatory and/or progressive disease in a subject in need thereof, comprising administering a mitochondrial Complex I modulator (MCIM) compound to the subject. In some embodiments, the treatment elicits tissue repair and/or disease control or regression. In some embodiments, the treatment initiates an adaptive response in certain cell types that leads to pharmacodynamic evidence of tissue repair and/or disease regression. In some embodiments, the adaptive response leads to tissue repair and/or disease regression. In some embodiments, the tissue repair and/or disease regression induces a restoration of tissue architecture towards its healthy state, which is characterised by anatomically normal architecture. In some embodiments, the MCIM compounds bind Complex I at a binding site at the top of, or outside, the Q tunnel.

The inflammatory and/or progressive disease may be any condition in which an imbalance is seen between cellular pathology and cellular repair. In some embodiments, the disease or disorder may be an autoimmune disorder such as rheumatoid arthritis (RA), inflammatory bowel disease (IBD), ulcerative colitis, Crohn's disease, a fibrotic condition such as interstitial lung disease, pulmonary fibrosis or liver fibrosis, a neurological disorder such as multiple sclerosis (MS) or amyotrophic lateral sclerosis (ALS), or a skeletal disorder such as osteoarthritis or osteoporosis. In some embodiments, the tissue repair and/or disease regression is characterised by an increased clinical repair score and/or comprises increased wound healing. Clinical repair scores can be used to objectively assess clinical repair in inflammatory and/or progressive diseases. Increased clinical repair score can be indicated by a decreased disease score. In some embodiments, the tissue repair and/or disease regression comprises an increased cell count of reparative cells. In some embodiments, the tissue repair and/or disease regression comprises an increased count of mesenchymal and/or epithelial cells. In some embodiments, the adaptive response is characterised by an increased count of mesenchymal and/or epithelial cells. In some embodiments, the adaptive response is characterised by an increase in the differentiation of mesenchymal and/or epithelial cells. For instance, when the inflammatory and/or progressive disease is an interstitial lung disease or pulmonary fibrosis, the reparative cells may comprise alveolar type 1 and/or type 2 cells and/or club cells. When the inflammatory and/or progressive disease is an IBD, the reparative cells may comprise epithelial cells and/or mucus cells. For instance, in the gastrointestinal tract the cells may comprise fibroblasts or epithelial cells, or alternatively repair may be indicated by an increase in the overall number of mucus cells which indicate that epithelial differentiation is proceeding normally. In the bone, an increase in the number or activity of osteoblasts and/or myeloid cells, such as M2 macrophages, may be seen.

In some embodiments, the adaptive response comprises a change in the function of mesenchymal or epithelial cells. For instance, in IBD the cells may comprise PAS positive cells, or surfactant producing epithelial cells. In some embodiments, the tissue repair and/or disease regression comprises a decreased cell count or decreased function of activated immune cell subtypes. For instance, when the inflammatory and/or progressive disease is RA, the tissue repair and/or disease regression may comprise a decrease in the number of myeloid cells such as macrophages or osteoclasts, lymphocytes such as T-, B-, or Th17 cells, or fibroblasts such as FLS cells. Preferably, the decrease in immune cell activity or activated immune cell numbers is not accompanied by a decrease in the activity, function or numbers of adapting mesenchymal or epithelial cells. When the inflammatory and/or progressive disease is MS, the reparative cells may comprise oligodendrocyte precursor cells (OPCs).

In some embodiments, the tissue repair and/or disease regression induces a restoration of tissue architecture towards its healthy state, which is characterised by anatomically normal architecture. For example, in some embodiments, the inflammatory and/or progressive disease is RA and the tissue repair and/or disease regression comprises increased bone formation and/or decreased bone resorption, optionally alongside reduced oedema and/or erythema. In some embodiments, the tissue repair and/or disease regression comprises a reduction in inflammatory cytokine production from pro-inflammatory myeloid cells. In some embodiments, the tissue repair and/or disease regression comprises an increase in growth factors that are important for angiogenesis, epithelialisation and matrix remodelling, such as VEGF, FGF21 and/or GDF15. Cytokine and growth factor levels can be measured by any suitable method, e.g. via ELISA as described herein, or via ELISpot. In some embodiments, the tissue repair and/or disease regression comprises an increase in basement collagen IV. Collagen IV levels can be measured by any suitable method, e.g. via ELISA as described herein and/or by immunohistochemical analysis.

Relatedly, the invention also provides compounds for use in increasing reparative cells and/or decreasing destructive cells in a subject with an inflammatory and/or progressive disease, to achieve tissue repair and, as a result, disease, regression or resolution, or improved symptom control and quality of life. In some embodiments, the compounds trigger an adaptive response in a subject with an inflammatory and/or progressive disease. The invention also provides methods for increasing reparative cells and/or decreasing destructive cells in a subject with an inflammatory and/or progressive disease, comprising administering the compound to achieve tissue repair and/or disease regression or resolution, or improved symptom control and quality of life. In some embodiments, the method increases the adaptive response of mesenchymal or epithelial cells and/or decreases the activation of or numbers of pro-inflammatory/fibrotic/erosive cells in a subject with an inflammatory and/or progressive disease. Relatedly, the invention also provides compounds for use in reducing cytokine production from pro-inflammatory myeloid cells in a subject with an inflammatory and/or progressive disease, to achieve tissue repair and/or disease control or regression. The invention also provides methods for reducing cytokine production from pro-inflammatory myeloid cells in a subject with an inflammatory and/or progressive disease, comprising administering the compound to achieve tissue repair and/or disease regression. The compounds and inflammatory and/or progressive diseases are defined herein.

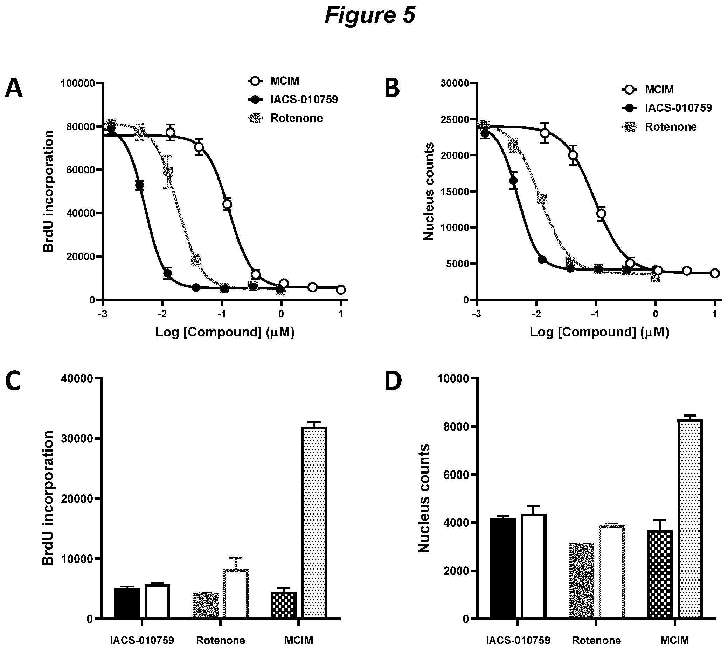

Preferably, the compound binds to Complex I and modulates Complex I activity. The modulation of Complex I activity may be determined by detecting a reduction in cellular O₂ consumption. In some embodiments, the reduction in cellular O₂ consumption is not associated with a reduction of cell viability. O₂ consumption may be measured by any standard technique known in the art, for example, using a real-time cell metabolic analyser (e.g. a Seahorse Analyzer). The modulation of Complex I activity may also lead to a reversible reduction of cell proliferation. The reversibility of the reduction of cell proliferation means that the reduction of proliferation is reversed when the compound is removed. This contrasts with the reduction of proliferation that is observed following treatment of the same cell type with a classical complex I binder, where reduced cell proliferation is not reversed by removal of the classical complex I binder. To assess the reversibility of the reduction of cell proliferation, an MCIM is applied to a cell culture, at a concentration to substantially reduce cell proliferation, for 24 hours at 37°C / 5% CO₂. After 24 hours, the cell culture is washed and cultured under conditions conducive to cell growth and proliferation. Recovery of cell proliferation is measured after 24-hours incubation in these 'growth' conditions (37°C / 5% CO₂, without MCIM present). Recovery of cell proliferation is observed. This contrasts to observations made on the same cells that have undergone the same culture/wash/grow cycle with a classical complex I binder in the culture step. The reversable reduction of cell proliferation may be assessed using human primary lung fibroblasts, e.g. as described in Example 4 (as illustrated in Figure 5).

Preferably, the compound interacts with Complex I at a binding site at the top of or outside the Q tunnel. In some embodiments, the binding site comprises one or more amino acid residues from NDUSF2 (SEQ ID NO: 1) and/or NDUSF7 (SEQ ID NO2). In some embodiments, the compound interacts with at least one amino acid residues in NDUFS2 (SEQ ID NO: 1), for instance His92, Gly85, Tyr141, His88, Leu95, Asp193, or Phe458. In some embodiments, the compound interacts with one or more amino acid residues in NDUFS2 (SEQ ID NO: 1) selected from Tyr141, His92 and Asp193.

In some embodiments, the response may include promotion of a repair phenotype during the same time course as control of inflammation. The control of inflammation induced by compounds of the invention may be distinct from the control of inflammation induced by other anti-inflammatory drugs which rely on the suppression of inflammation before activation of tissue repair as a secondary effect.

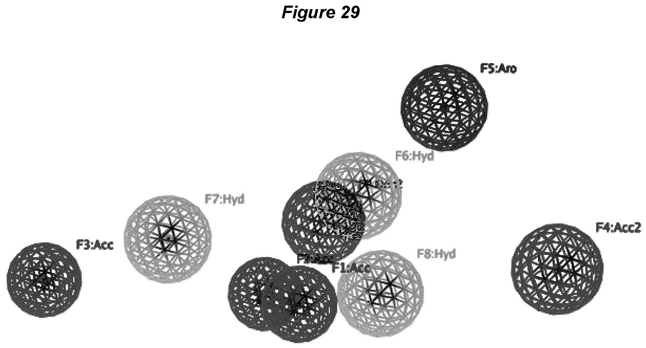

In some embodiments, the compound comprises four or more of the pharmacophore features of the pharmacophore model represented in Figure 29. The three-dimensional arrangement of the pharmacophore features may be as described in Tables 8-A, 8-B and 8-C.

In some embodiments, the compound is a compound, or a pharmaceutically acceptable salt, hydrate, or solvate thereof, of the following formula: 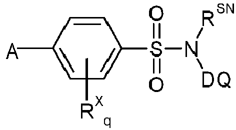 as defined in claim 1 of WO2010/032009. Claim 1 of WO2010/032009 is hereby incorporated by reference. Furthermore, WO2010/032009 is hereby incorporated by reference in its entirety.

In one embodiment, the compound is a compound, or a pharmaceutically acceptable salt, hydrate, or solvate thereof as defined in WO2014/207445 A1, which is incorporated herein by reference in its entirety. For example, in some embodiments, the compound is a compound selected from compounds of the following formulae, or a pharmaceutically acceptable salt, hydrate, or solvate thereof: 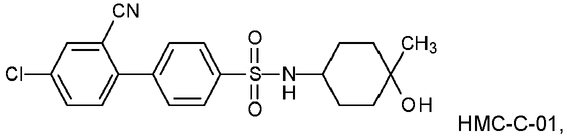 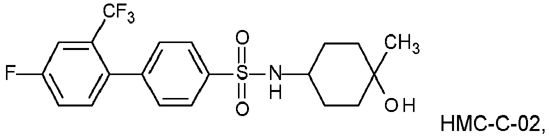 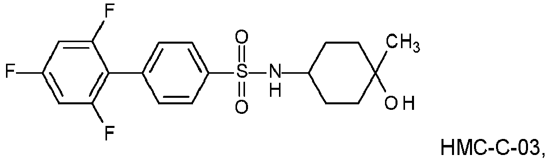 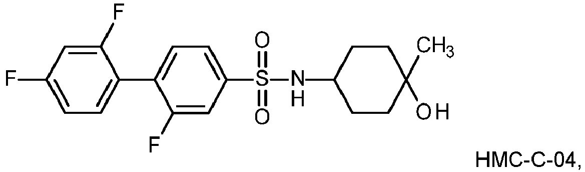 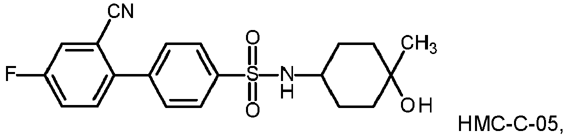 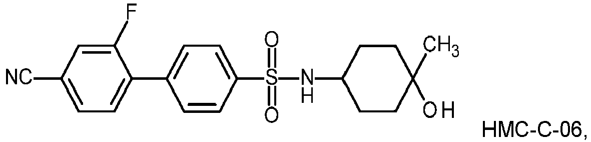 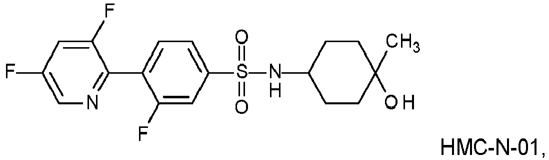 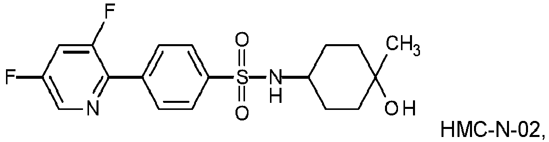 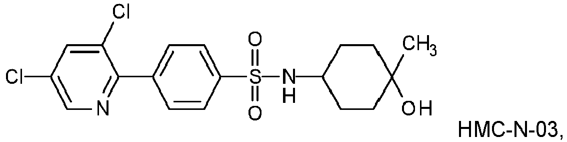 and

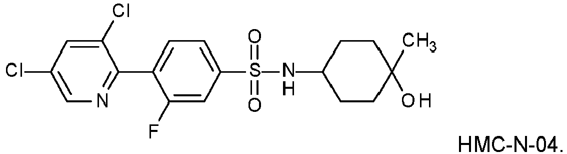

In some embodiments, the compound is a compound of the following formula, or a pharmaceutically acceptable salt, hydrate, or solvate thereof:

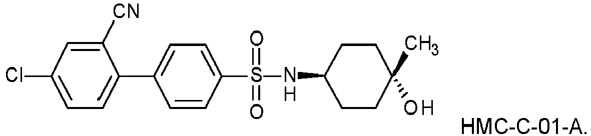

In one embodiment, the compound is a compound, or a pharmaceutically acceptable salt, hydrate, or solvate thereof as defined in WO2016/097001 A1, which is incorporated herein by reference in its entirety. For example, in some embodiments, the compound is a compound selected from compounds of the following formulae, or a pharmaceutically acceptable salt, hydrate, or solvate thereof: 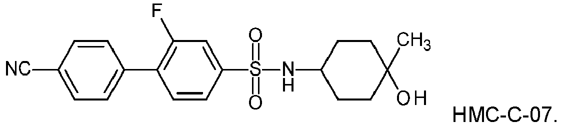 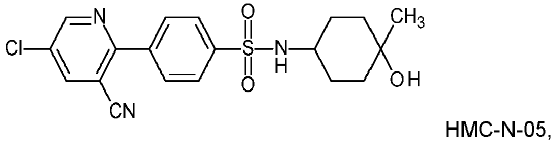 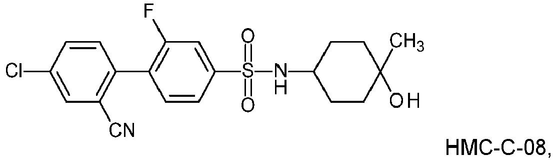 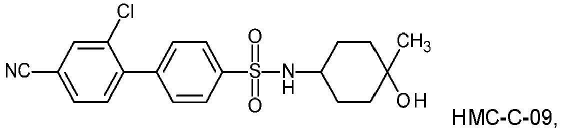 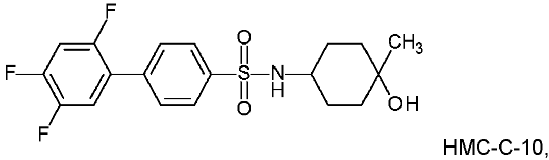 and

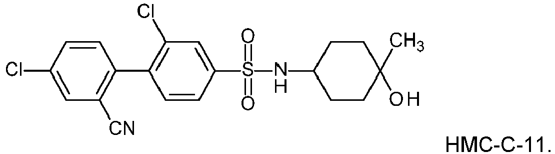

In some embodiments, the compound is a compound, or a pharmaceutically acceptable salt, hydrate, or solvate thereof, of the following formula: 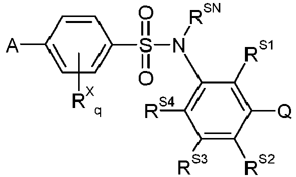 as defined in claim 1 of WO2010/032010. Claim 1 of WO2010/032010 is hereby incorporated by reference. Furthermore, WO2010/032010 is hereby incorporated by reference in its entirety.

In some embodiments, the compound is a compound, or a pharmaceutically acceptable salt, hydrate, or solvate thereof, of the following formula: 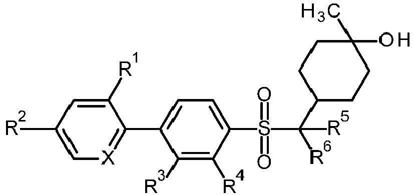 as defined in claim 1 of WO2020/035560 A1. Claim 1 of WO2020/035560 A1 is hereby incorporated by reference. Furthermore, WO2020/035560 A1 is hereby incorporated by reference in its entirety.

In some embodiments, the compound is a compound, or a pharmaceutically acceptable salt, hydrate, or solvate thereof, of the following formula: 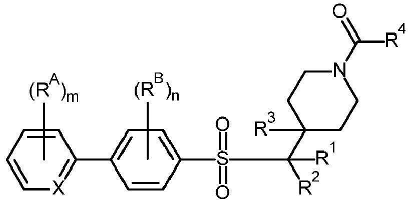 as defined in claim 1 of WO2020/212581 A1. Claim 1 of WO2020/212581 A1 is hereby incorporated by reference. Furthermore, WO2020/212581 A1 is hereby incorporated by reference in its entirety.

In some embodiments, the compound is a compound of the following formula, or a pharmaceutically acceptable salt, hydrate, or solvate thereof:

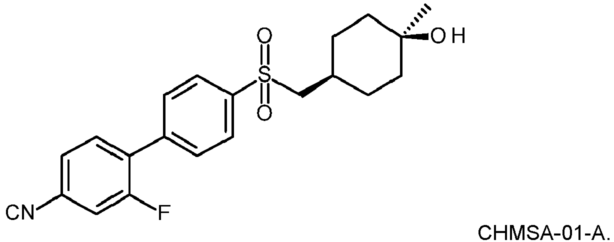

In some embodiments, the compound is a compound of the following formula, or a pharmaceutically acceptable salt, hydrate, or solvate thereof:

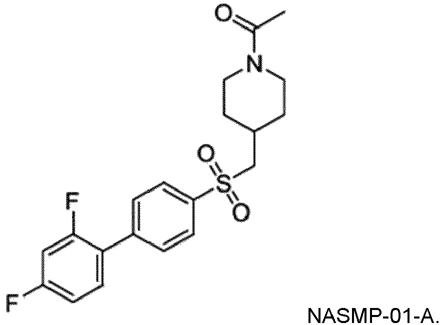

In some embodiments, the compound is a compound of the following formula, or a pharmaceutically acceptable salt, hydrate, or solvate thereof:

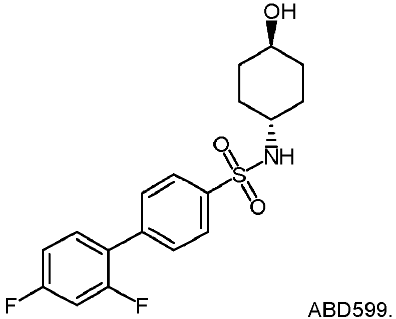

In some embodiments, the compound is a compound of the following formula, or a pharmaceutically acceptable salt, hydrate, or solvate thereof:

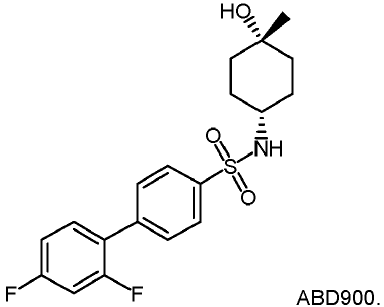

In some embodiments, the compound is a compound of the following formula, or a pharmaceutically acceptable salt, hydrate, or solvate thereof:

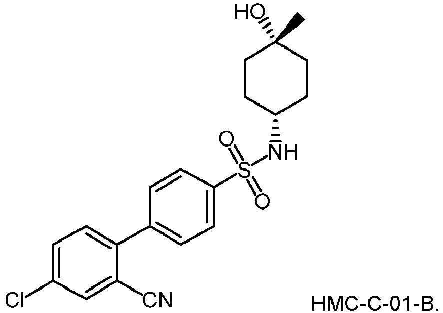

In some embodiments, the compound is a compound of the following formula, or a pharmaceutically acceptable salt, hydrate, or solvate thereof:

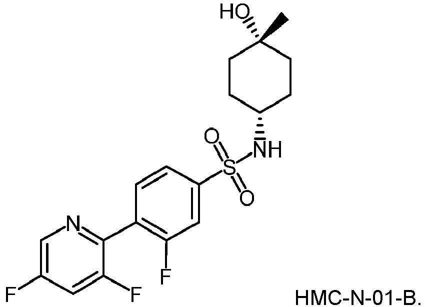

In some embodiments, the compound is a compound of the following formula, or a pharmaceutically acceptable salt, hydrate, or solvate thereof:

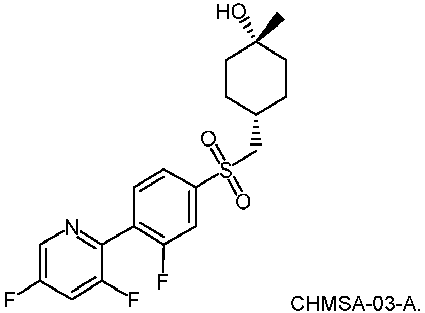

In some embodiments, the compound is a compound of the following formula, or a pharmaceutically acceptable salt, hydrate, or solvate thereof:

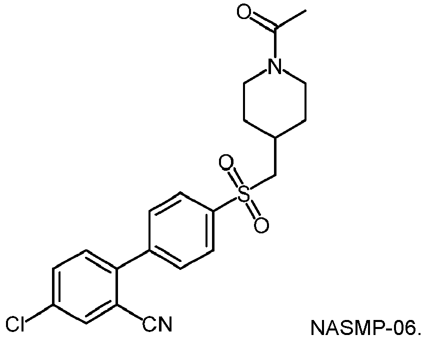

The invention includes the combination of the aspects and preferred features described except where such a combination is clearly impermissible or expressly avoided.

### Summary of the Figures

Embodiments and experiments illustrating the principles of the invention will now be discussed with reference to the accompanying figures in which:
**Figure 1****.** A graph of response over time of each of the three phases of repair (Inflammation, proliferation and tissue remodelling) which are proposed to be broadly similar across tissues. Cell types and soluble factors involved in each phase are shown below the graph along with changes in the extracellular matrix.

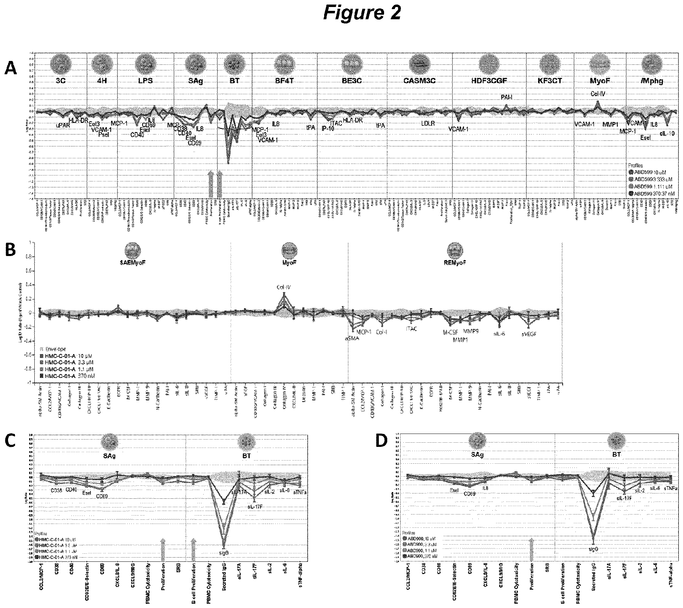

**Figure 2****.** High throughput integrative biology platform (BioMAP^{®}) profile of the effects of MCIM compounds of the invention on multiple disease-associated regulatory pathways identified that these compounds have the potential to regulate inflammatory responses and the tissue remodelling collagen, collagen type IV for ABD599 (A), HMC-C-01-A (B) and (C), and ABD900 (D).

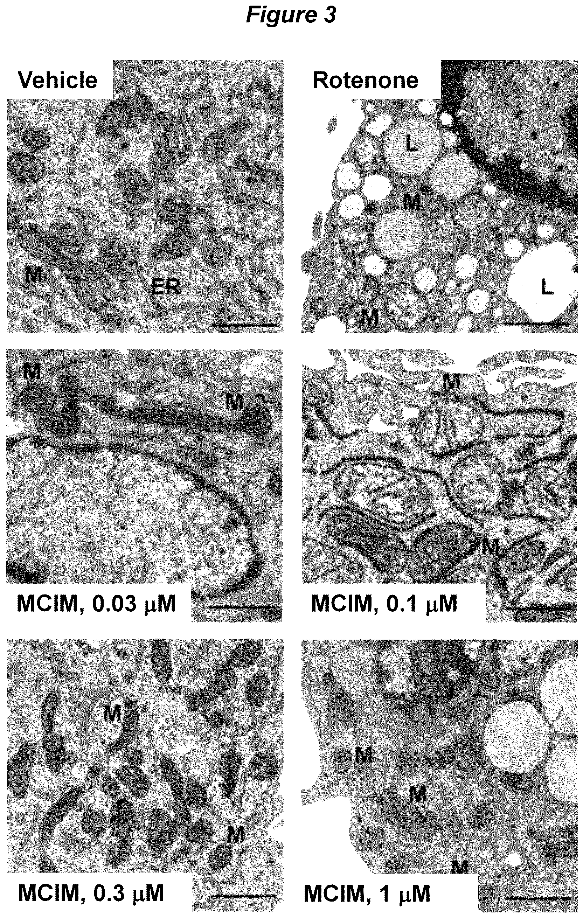

**Figure 3****.** Electron micrographs of human primary myeloid cells (osteoclasts) treated with Complex I binders of the invention show an adaptive response by changing mitochondrial morphology, with an increase in mitochondrial area without an overt increase in mitochondrial mass.

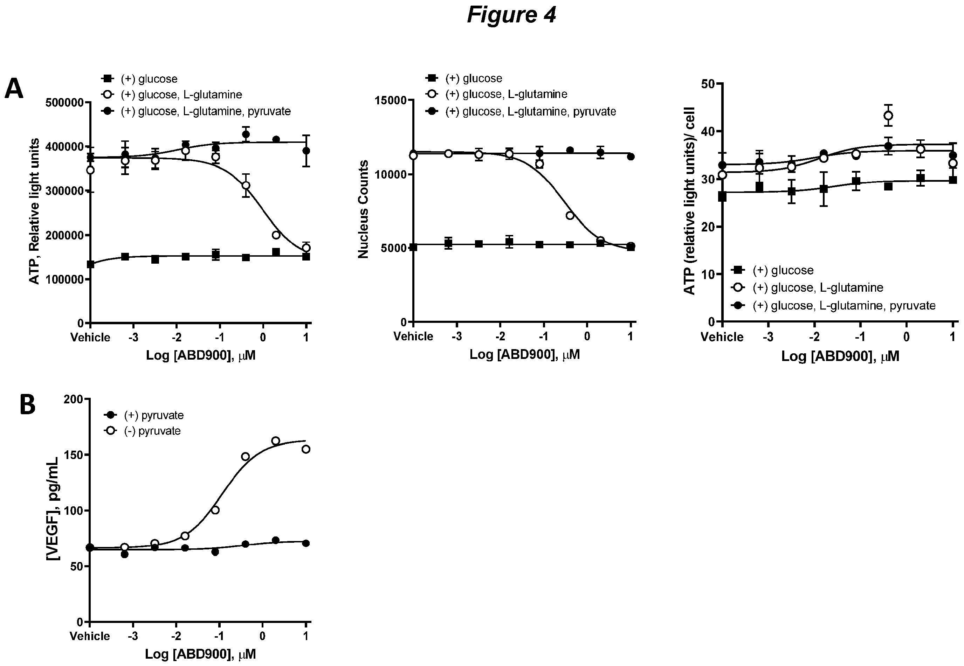

**Figure 4****.** A) Three graphs showing the change in intracellular ATP levels (top left), nuclei count (top middle) and ATP readout per cell (top right) in response to increasing MCIM compound dose in standard, glucose supplemented media (squares), media supplemented with glucose and L-glutamine (open circles) or media supplemented with glucose, L-glutamine, and pyruvate (close circles). B) A graph showing the change in VEGF secretion in response to increasing MCIM compound concentration in standard, glucose and L-glutamine supplemented media containing pyruvate (closed circles), or medium containing glucose and L-glutamine but without pyruvate (open circles). MCIM compounds of the invention promote an adaptive response by increasing VEGF secretion in metabolically restricted human primary lung fibroblasts without a change in cell number.
**Figure 5****.** Cell proliferation inhibition measured by BrdU incorporation (A) and nucleic count (B) as a function of increasing dosage of either Rotenone (squares), IACS-010759 (closed circles) or MCIM compound (open circles). Comparison of cells without washout vs washout of test compounds on cell proliferation measure by BrdU incorporation (C) and nuclei count (D). Non-washout and washout of rotenone are shown in grey and open grey, respectively. Non-washout and washout of IACS-010759 are shown in black and open black, respectively. Non-washout and washout of MCIM compound are shown in checkboard fill and open fill pattern, respectively. MCIM compounds of the invention reversibly inhibit cell proliferation whereas cells treated with typical Complex I inhibitors IACS-010759 and rotenone do not recover cell proliferative capacity after compound washing out.

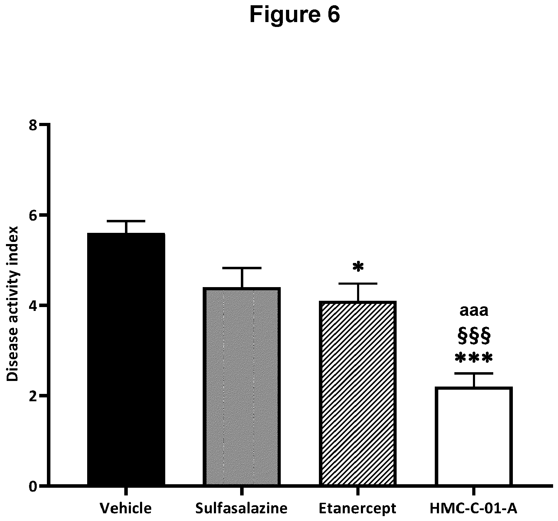

**Figure 6****.** A graph showing a comparison of disease activity index in mice with DSS-induced colitis treated with vehicle control, sulfasalazine, etanercept and MCIM compound. MCIM compounds of the invention reduce the severity of symptoms of mice with DSS-induced colitis compared to mice treated with vehicle control, sulfasalazine or etanercept. Data are mean ± s.e.m.. * p<0.05, *** p<0.005 vs vehicle, §§§ p <0.005 vs sulfasalazine, ^{aaa} p<0.005 vs etanercept.

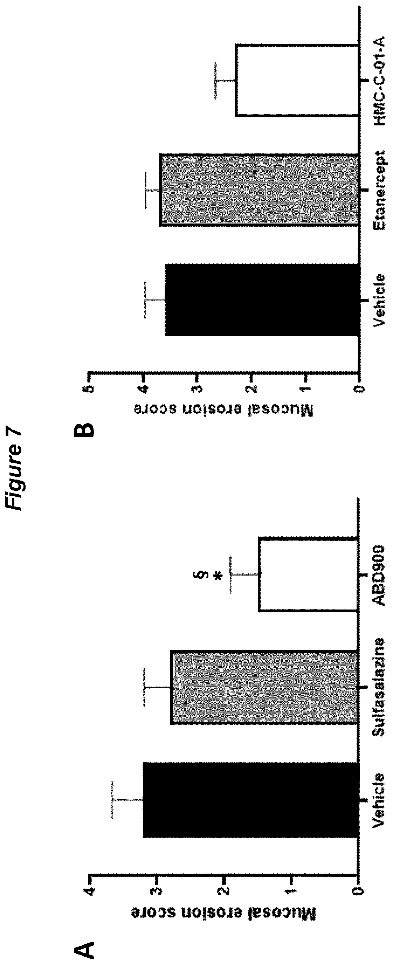

**Figure 7****.** Two graphs showing a comparison of mucosal erosion in mice with DSS-induced colitis treated with (A) vehicle control, sulfasalazine or ABD900 and (B) vehicle control, sulfasalazine or HMC-C-01-A. MCIM compounds of the invention inhibit mucosal erosion to a greater extent in mice with established DSS-induced colitis compared to mice treated with vehicle control, Sulfasalazine (A), or Etanercept (B). Data are mean ± s.e.m.. * p<0.05 vs vehicle § p<0.05 vs sulfasalazine.

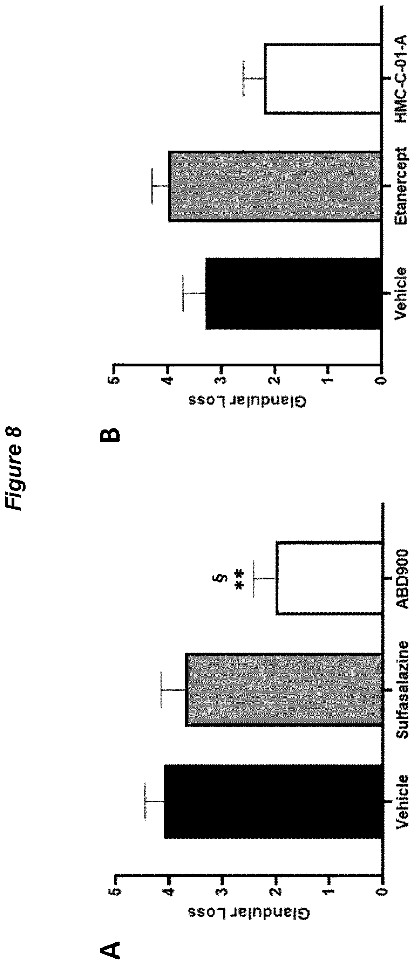

**Figure 8****.** Two graphs showing a comparison of glandular loss in mice with DSS-induced colitis treated with (A) vehicle control, sulfasalazine or ABD900 and (B) vehicle control, sulfasalazine or HMC-C-01-A. MCIM compounds of the invention reduce glandular loss to a greater extent in mice with established DSS-induced colitis compared to mice treated with vehicle control, Sulfasalazine (A) or Etanercept (B). Data are mean ± s.e.m.. ** p<0.01 vs vehicle, § p<0.05 vs sulfasalazine.

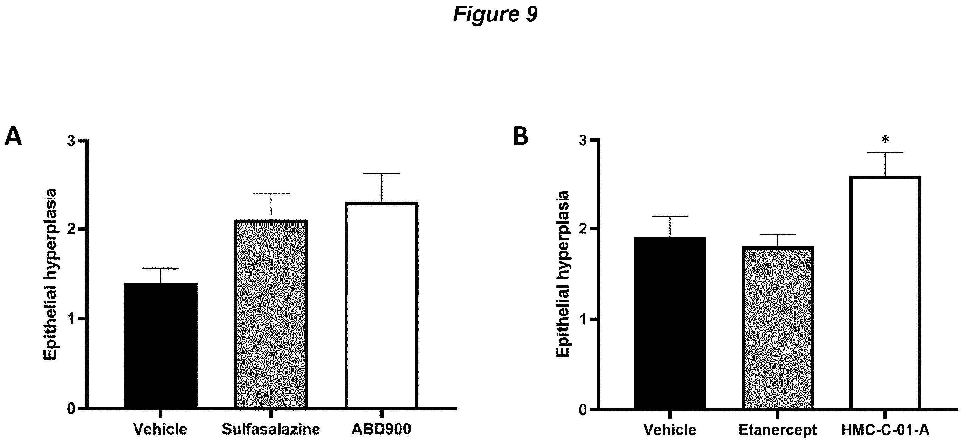

**Figure 9****.** Two graphs showing a comparison of epithelial hyperplasia in mice with DSS-induced colitis treated with (A) vehicle control, sulfasalazine or ABD900 and (B) vehicle control, sulfasalazine or HMC-C-01-A. MCIM compounds of the invention can promote epithelial hyperplasia in mice with established DSS-induced colitis at a comparable level compared to mice treated with Sulfasalazine (A) but to a greater extent than mice treated with Etanercept (B). Data are mean ± s.e.m.. * p<0.05 vs vehicle.

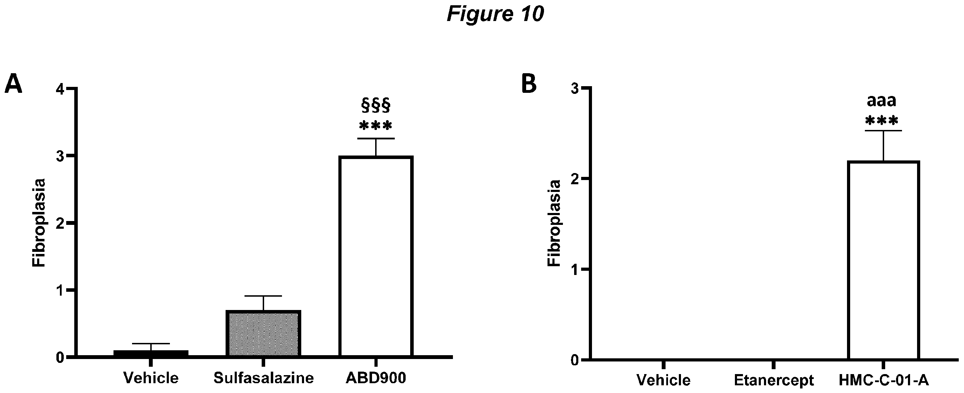

**Figure 10****.** Two graphs showing a comparison of fibroplasia, which is indicative of tissue or wound repair, in mice with DSS-induced colitis treated with (A) vehicle control, sulfasalazine or ABD900 and (B) vehicle control, sulfasalazine or HMC-C-01-A. MCIM compounds of the invention promote increased 'healthy' fibroplasia in mice with established DSS-induced colitis compared to mice treated with vehicle control, Sulfasalazine (A) or Etanercept (B). Data are mean ± s.e.m.. *** p<0.005 vs vehicle, §§§ p <0.005 vs sulfasalazine, ^{aaa} p<0.005 vs etanercept.

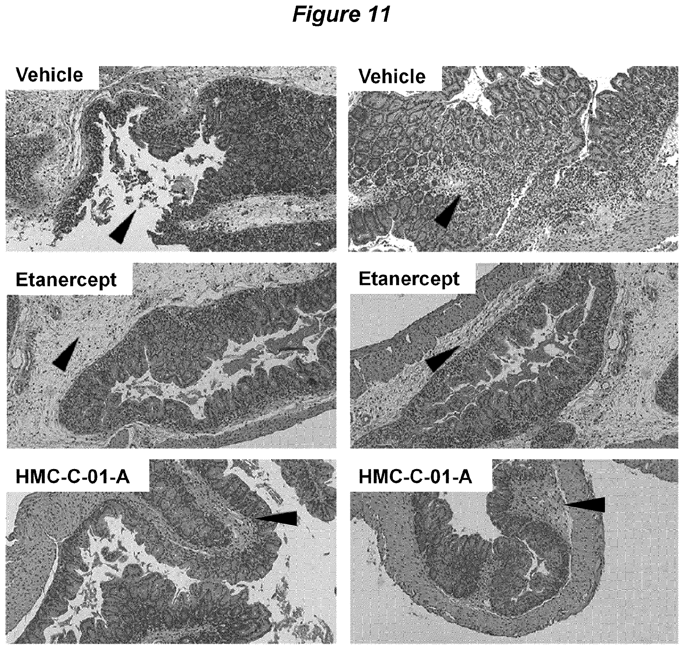

**Figure 11****.** Histological sections from mice with DSS-induced colitis treated with either vehicle control (top panels), etanercept (middle panels) or an HMC-C-01-A (bottom panels). Ulceration, loss of architecture, oedema/inflammation and erosion is seen in the vehicle control (arrows). The positive control, etanercept, shows general conservation of tissue architecture but with underlying inflammation and oedema (arrows). The MCIM of the invention shows a general conservation of architecture, with no inflammation or oedema and with radial distribution of repair (arrows).

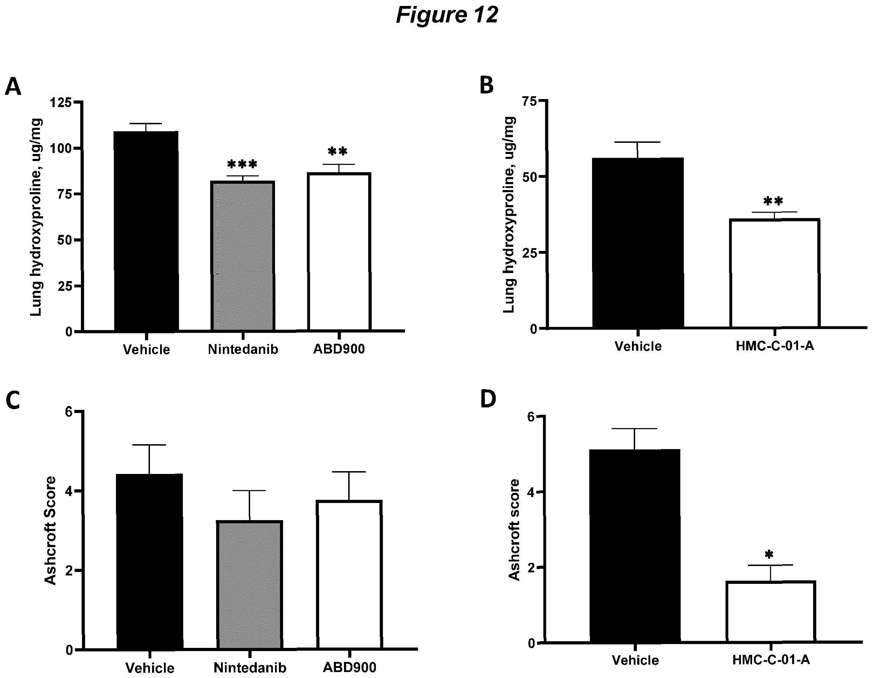

**Figure 12****.** Four graphs showing the change in (A and B) lung hydroxyproline levels and (C and D) Ashcroft score following treatment of mice with bleomycin-induced lung fibrosis with vehicle control, nintedanib or MCIM compounds of the invention. MCIM compounds of the invention reduce lung hydroxyproline levels in mice (panels A and B) which is indicative of reduced collagen and fibrosis in lung. MCIM compounds of the invention also reduce the histopathological presentation of fibrosis (as measured by the Ashcroft scoring system) compared to vehicle controls (panels C and D), with HMC-C-01-A reducing the Ashcroft score from ~5 to <2 indicating MCIM compounds reduce the pathological presentation of lung fibrosis. Data are mean ± s.e.m.. * p<0.05, ** p<0.01, *** p<0.005 vs vehicle

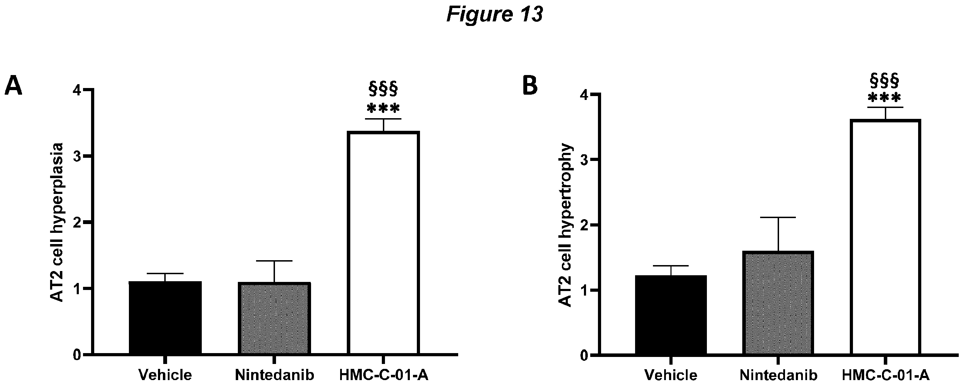

**Figure 13****.** Two graphs showing changes in (A) AT2 cell hyperplasia and (B) AT2 cell hypertrophy in mice with bleomycin-induced lung fibrosis treated with vehicle control, nintedanib or MCIM compounds. MCIM compounds of the invention increase alveolar AT2 cell number (AT2 cell hyperplasia, first panel) and cell size (AT2 cell hypertrophy, second panel). AT2 cells maintain alveolar homeostasis by secreting pulmonary surfactant and serve as alveolar stem cells with the potential to self-renew and differentiate into AT1 cells. Loss of these cells contributes to the development of lung fibrosis. No significant increase is seen following treatment with nintedanib at 30 mg/kg/ given twice daily. Increased AT2 cell size and cell number is seen with the MCIM compound of the invention, with highly significant increases seen at 20 mg/kg/day indicating mobilisation of a repair response. Data are mean ± s.e.m.. *** p<0.005 vs vehicle, §§§ p <0.005 vs nintedanib.

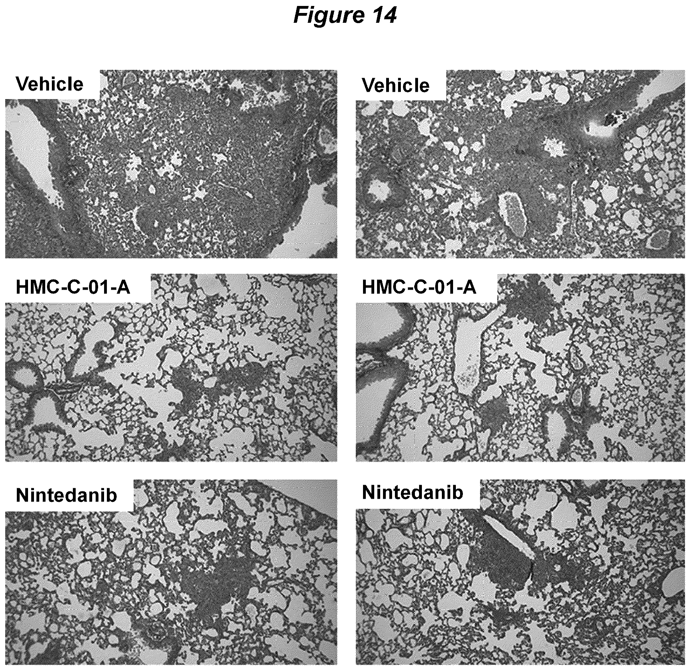

**Figure 14****.** H&E stained bleomycin-induced fibrotic lung tissue from mice treated with Vehicle, nintedanib and MCIM compounds of the invention. Compounds of the invention (middle panels) reduce fibrosis to at least the same extent as nintedanib (bottom panels), and significantly vs vehicle (top panels).

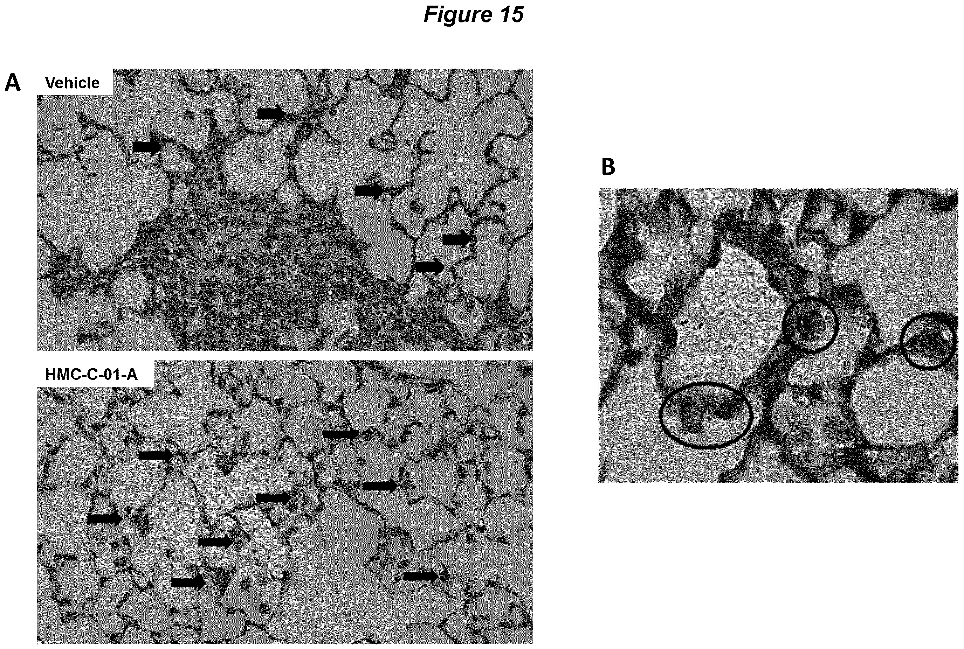

**Figure 15****.** H&E stained bleomycin-induced fibrotic lung tissue from mice treated with Vehicle, nintedanib and Complex I binder of the invention. (A) Compound elicits a cuboid phenotype in the AEC2 cells (lower panel, arrows), compared to vehicle which shows a flattened appearance (top panel, arrows). This indicates adaptive repair changes, which are accompanied by increased surfactant production ((B); magnified image with circles).

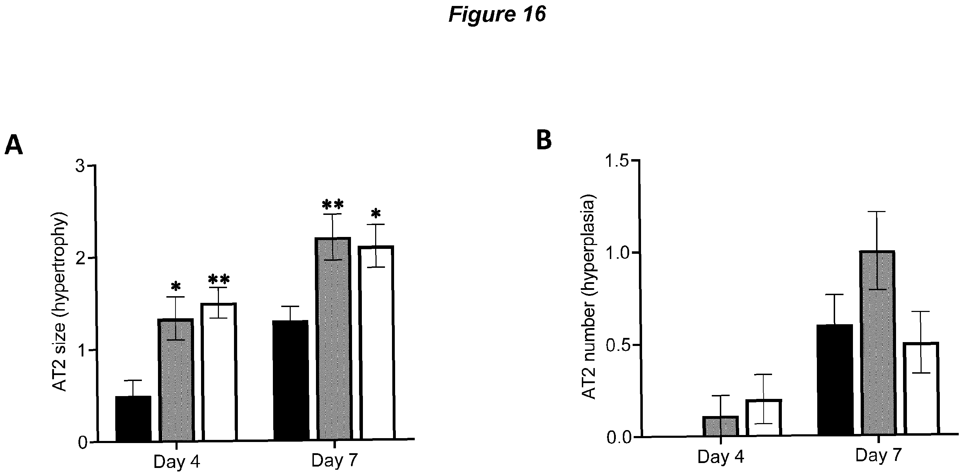

**Figure 16****.** Comparison of (A) AT2 cell size and (B) AT2 cell number in mice with bleomycin-induced lung fibrosis following treatment with vehicle control (black), 10 mg/kg/day ABD900 (grey bars) or 20 mg/kg/day ABD900 (white bars). ABD900 increases AT2 cell size (hypertrophy) at Day 4 and is maintained through to Day 7. ABD900 treatment did not lead to a statistically significant increase in AT2 number (hyperplasia) over 7 days. Data are mean ± s.e.m.. * p<0.05 ** p<0.01 vs time-matched vehicle.

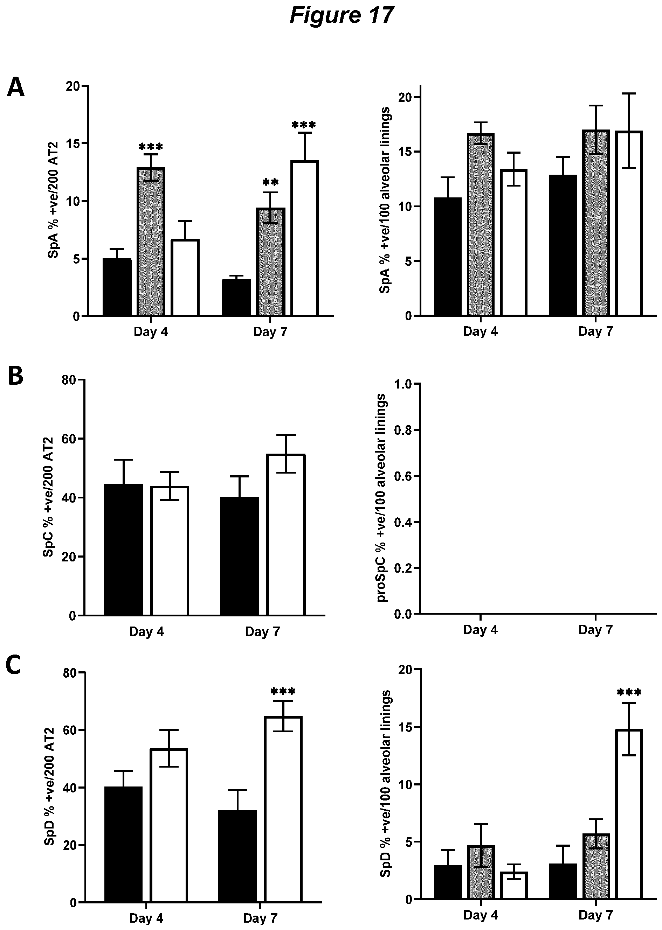

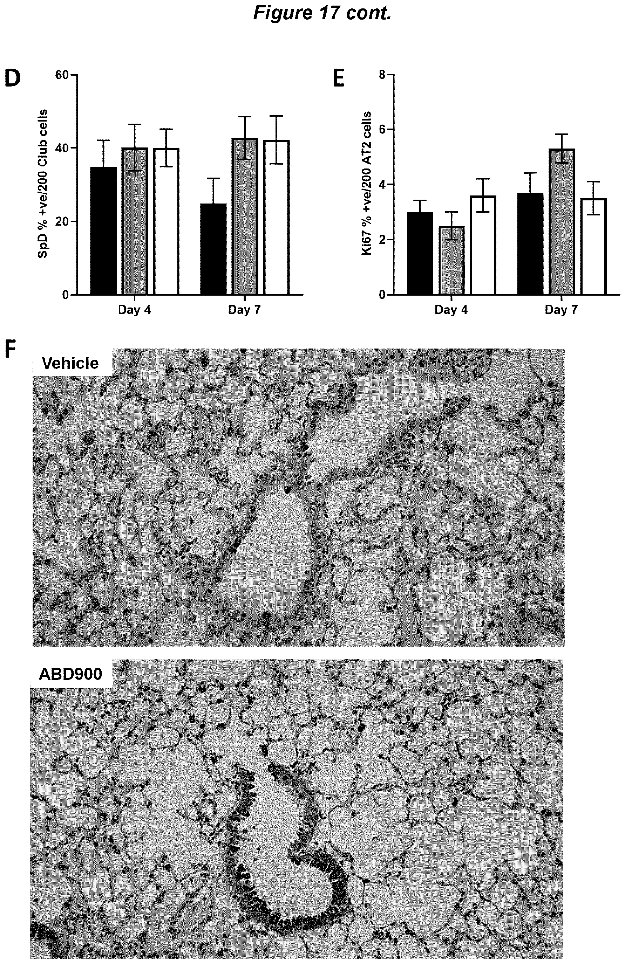

**Figure 17****.** Comparison of surfactant expression in lung of mice with bleomycin-induced lung fibrosis following treatment with vehicle control or an MCIM of the current invention, ABD900. (A) SP-A, (B) pSP-C and (C) SP-D scoring in AT2 cells (left panels) and alveolar lining (right panels), (D) SP-D scoring in Club cells, and (E) Ki67 in AT2 cells. Vehicle is shown in black bars and animals dosed with ABD900 at 10 mg/kg/day in grey bars or 20 mg/kg/day in white bars, respectively. Data are mean ± s.e.m.. (F) Representative images (160x magnification) show the increase in SP-D staining (black) in the lungs following treatment with ABD900 (20 mg/kg/day) for 7 days (lower panel) compared with vehicle (upper panel). ** p<0.01 *** p<0.005 vs time-matched vehicle.

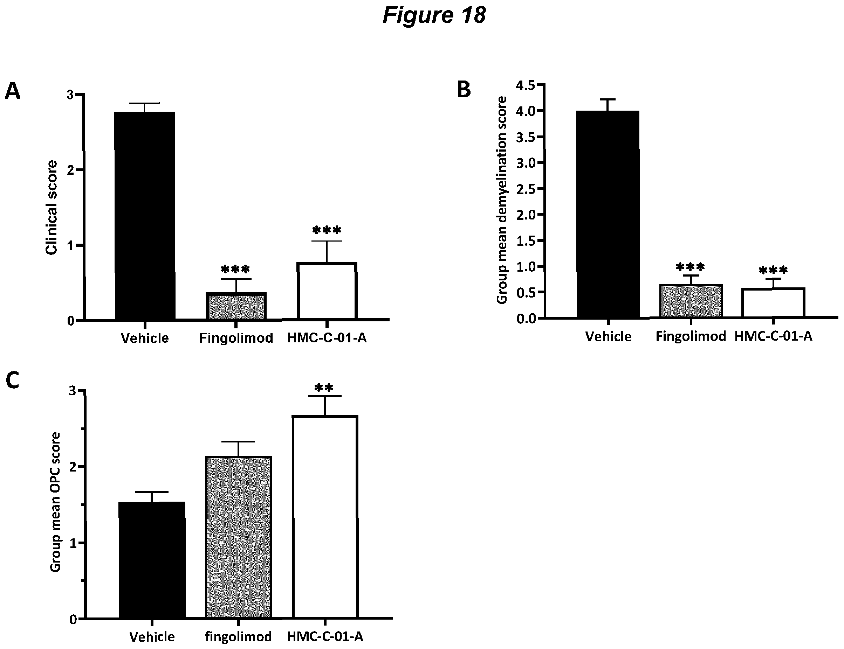

**Figure 18****.** Graphs showing the (A) clinical score, (B) mean demyelination score, and (c) mean oligodendrocyte precursor cell score in mice with EAE multiple sclerosis treated with vehicle, fingolimod or an MCIM compound. MCIM compounds of the invention controls disease and supports repair in the EAE multiple sclerosis disease model. The first panel shows that 10 mg/kg/day of a MCIM compound of the invention reduces clinical score in the EAE multiple sclerosis disease model. The second panel shows that an MCIM compound of the invention reduces demyelination in the EAE multiple sclerosis disease model. The third panel shows that a MCIM compound of the invention supports oligodendrocyte precursor cells (OPCs) to a greater extent than fingolimod in the EAE multiple sclerosis disease model. Data are mean ± s.e.m.. ** p<0.01 *** p<0.005 vs vehicle

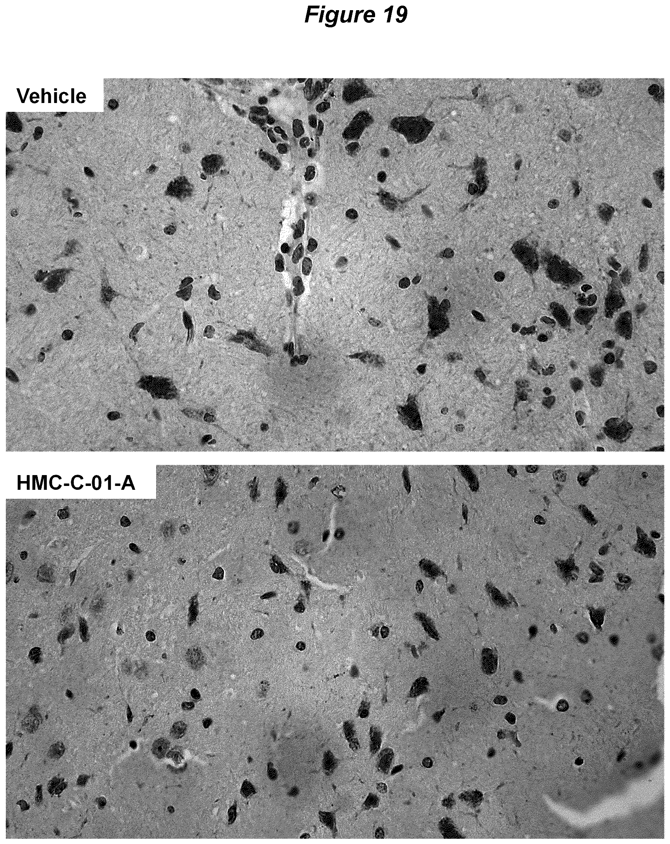

**Figure 19****.** Histological sections of mice neural tissue from mice with EAE MS treated with either vehicle control (left panel) or 10 mg/kg/day MCIM compound (right panel). MCIM compounds of the invention reduces inflammation and increases ramified (`resting') microglia in the MS model. Upper panel shows rounded microglia with evident process extrusions following administration of vehicle alone (negative control). Lower panel shows ramified microglia with reduced process extrusions following administration of a MCIM compound of the invention.

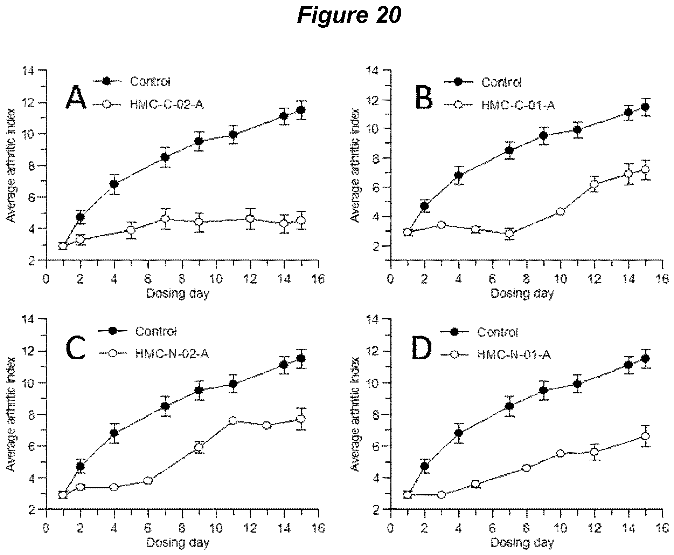

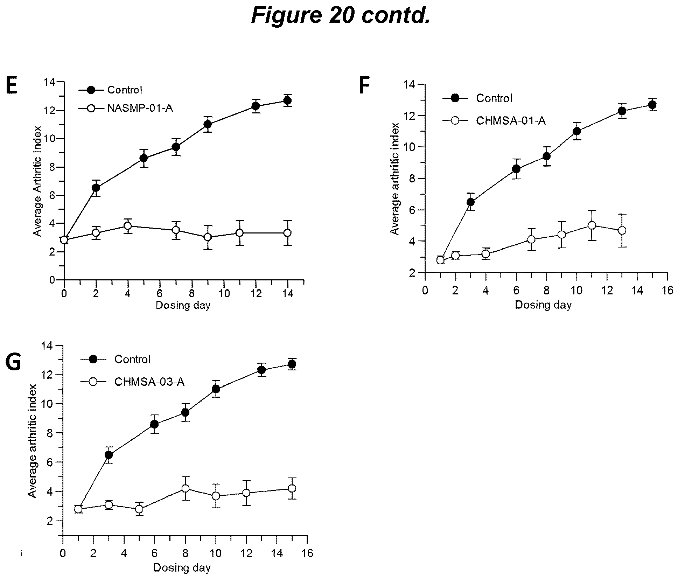

**Figure 20****.** Seven graphs each showing the average arthritic index as a function of time (dosing day) for test compound dosed at 10 mg/kg/day by oral gavage (open circles) and control (solid circles), for each of (A) HMC-C-02-A, (B) HMC-C-01-A, (C) HMC-N-02-A, (D) HMC-N-01-A, (E) NASMP-01-A, (F) CHMSA-01-A, (G) CHMSA-03-A.

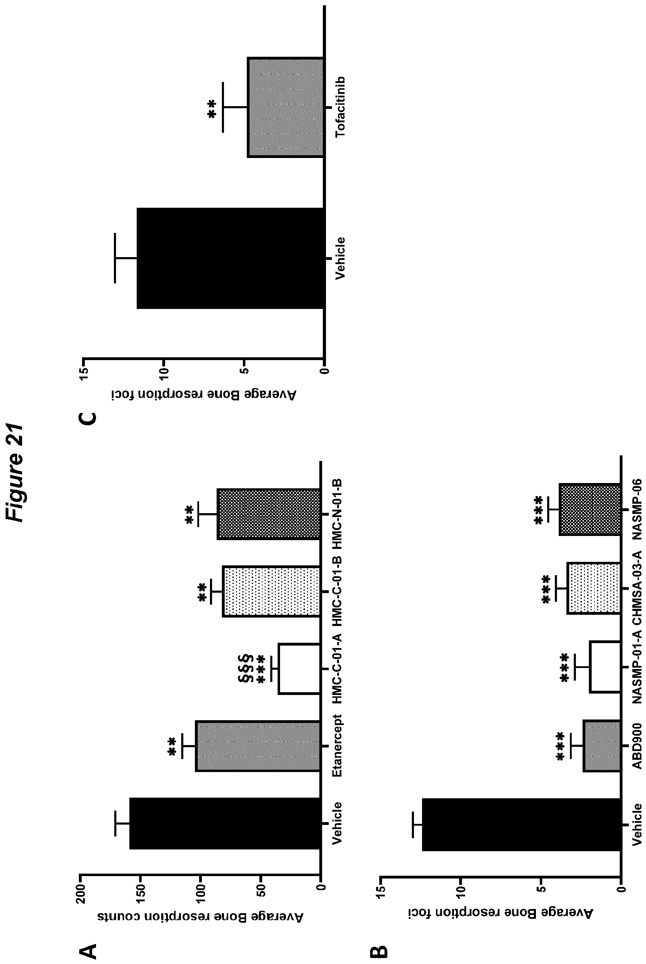

**Figure 21****.** Graphs showing the average bone resorption counts in mice with collagen-induced arthritis treated with (A) vehicle control, 3 mg/kg/day etanercept, or 10 mg/kg/day of either HMC-C-01-A, HMC-C-01-B, or HMC-N-01-B, (B) vehicle control, or 10 mg/kg/day of either ABD900, NASMP-01, CHMSA-03-A, or NASMP-06, and (C) vehicle control or tofacitinib, MCIM compounds of the invention protect against bone resorption in mice suffering from collagen-induced arthritis at levels comparable or exceeding approved treatments etanercept and tofactinib. Data are mean ± s.e.m.. ** p<0.01 *** p<0.005 vs vehicle §§§ p <0.005 vs etanercept.

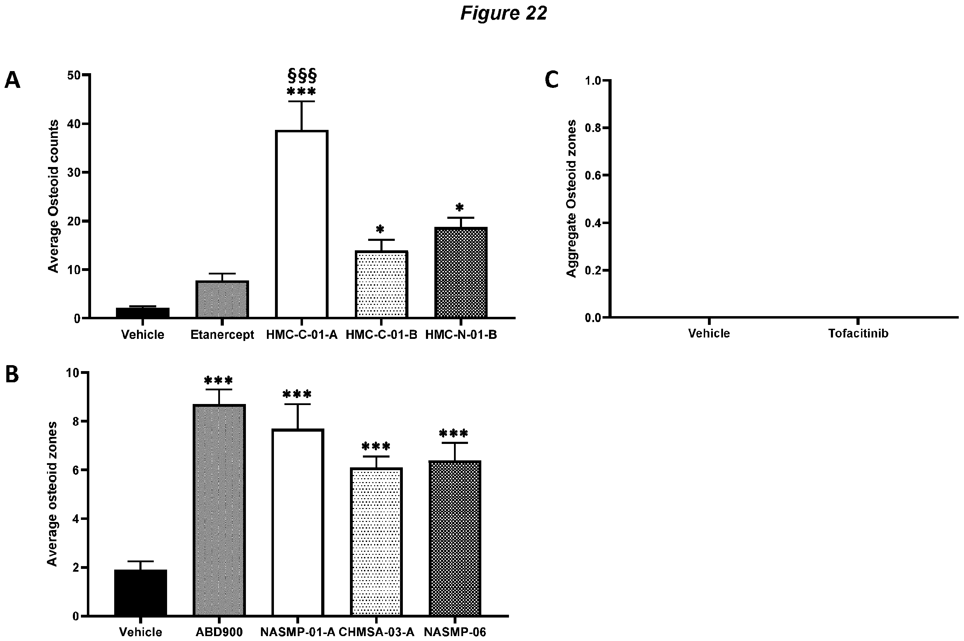

**Figure 22****.** Graphs showing the (A) average osteoid counts in mice with collagen-induced arthritis treated with vehicle control, 3 mg/kg/day etanercept, or 10 mg/kg/day of either HMC-C-01-A, HMC-C-01-B, or HMC-N-01-B, (B) average osteoid zones in mice with collagen-induced arthritis treated with vehicle control, or 10 mg/kg/day of either ABD900, NASMP-01 ,CHMSA-03-A, or NASMP-06, and (C) vehicle control or tofacitinib. MCIM compounds of the invention promote the formation of osteoid, an indicator of bone formation, in mice with established collagen-induced arthritis. The new bone formation in animals with existing bone erosion indicates a repair effect, occurring to a much greater extent than seen with the approved therapeutic drugs etanercept and tofacitinib. Data are mean ± s.e.m.. ** p<0.01 *** p<0.005 vs vehicle §§§ p <0.005 vs etanercept.

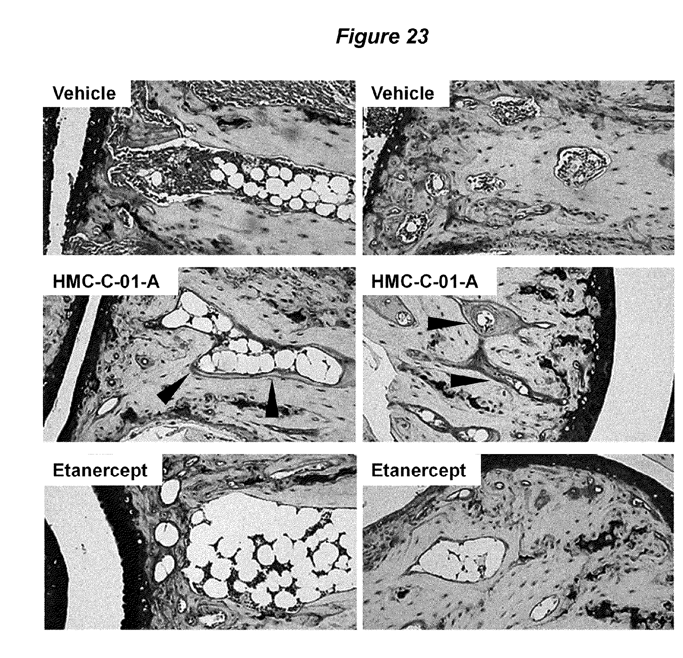

**Figure 23****.** Histological sections (160x magnification, stained with toluidine blue) of limbs from mice with collagen-induced arthritis treated with either vehicle control (top panels), 10 mg/kg/day MCIM compound (middle panels) or 3 mg/kg/day etanercept (bottom panels). MCIM compounds of the invention promote bone formation in established arthritis, indicating an adaptive repair response. Top panel shows bone from collagen-induced arthritic mice treated with vehicle showing no clear signs of bone formation. Middle panel shows bone from collagen-induced arthritis mice which have clear indications of osteoid (new bone) formation in a structured manner as indicated by the black arrows. In contrast, mice treated with etanercept show only low levels of reactive and sporadic new bone formation.

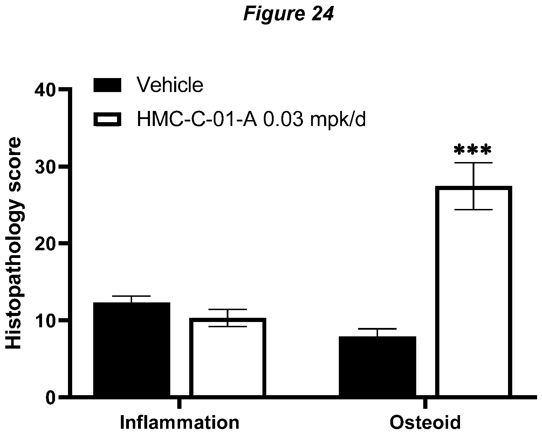

**Figure 24****.** Graph showing the relative inflammatory (left two bars) and osteoid (right two bars) scores in mice with collagen-induced arthritis treated with either vehicle control or a very low dose MCIM compound at 0.03 mg/kg per day for 14 days. MCIM compounds of the invention promote adaptive responses leading to repair (osteoid formation) in mice with established collagen-induced arthritis at doses which don't control inflammation, showing that the response is not a consequence of control of inflammation, but an independent, adaptive response. Data are mean ± s.e.m.. *** p<0.005 vs vehicle.

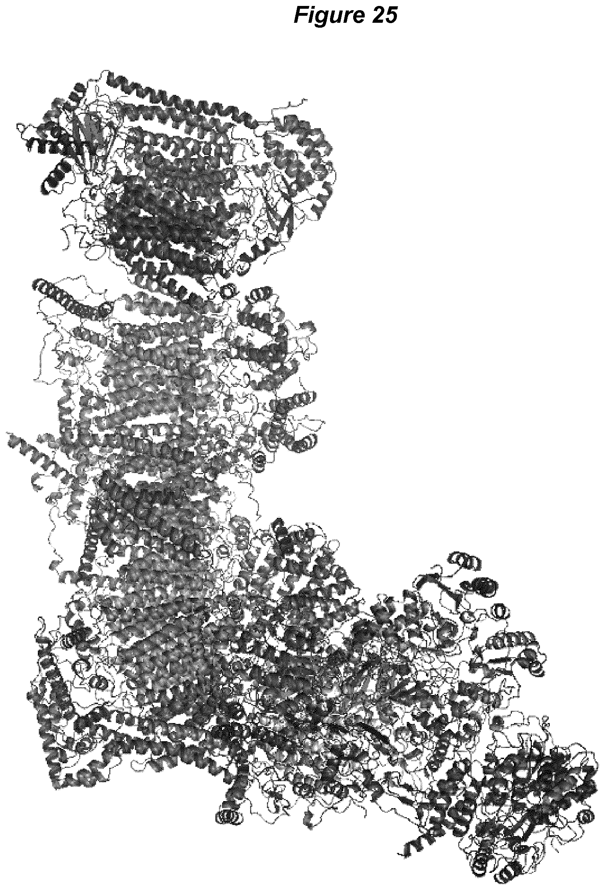

**Figure 25****.** Homology model of the complete Complex I constructed from publicly available structures for 5 different organisms. The putative targets were resolved in all 5 structures.

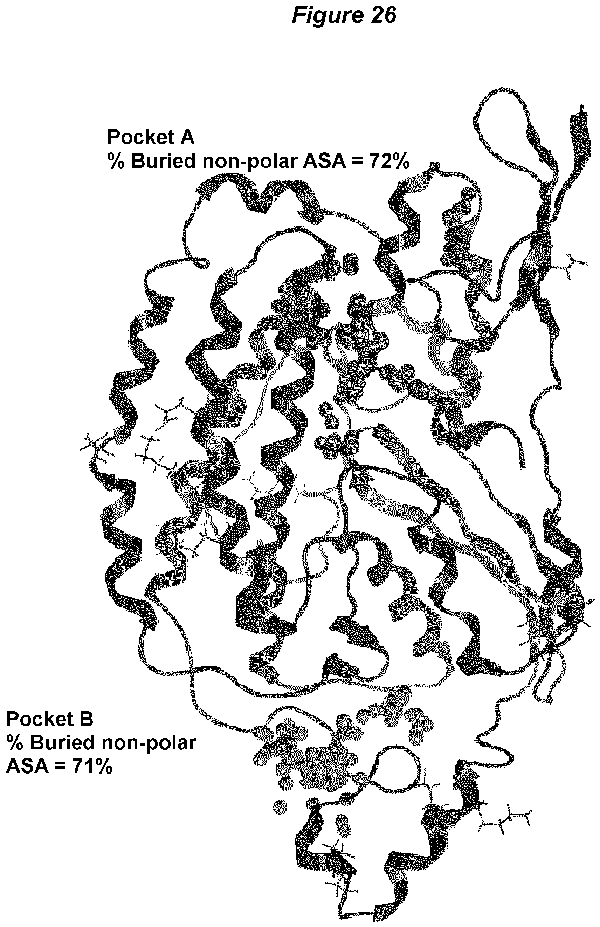

**Figure 26****.** *In silico* homology model of NDUFS2. Druggability assessment was performed using SiteMap and identified two binding pockets in the Complex I subunit NDUFS2 (spheres).

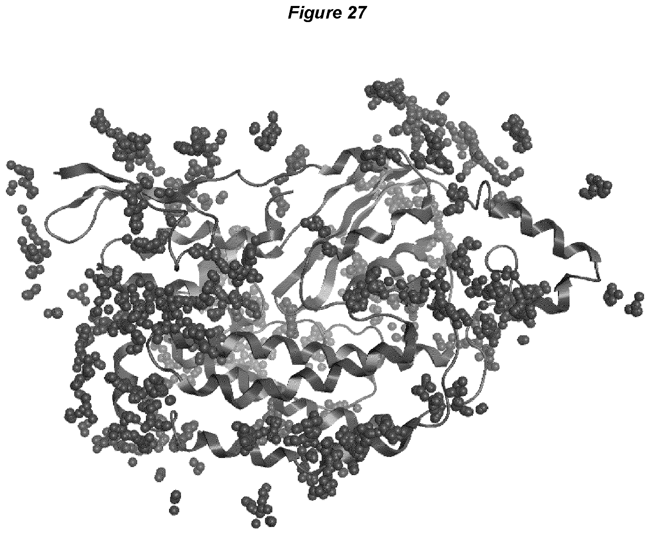

**Figure 27****.** *In silico* SiteFinder model of NDUFS2 when in Complex I. A narrow channel was identified for Q10 and drug-like compound binding in the Q-tunnel. Spheres are used to illustrate the space/channels around NDUFS2 when in the Complex I structure.

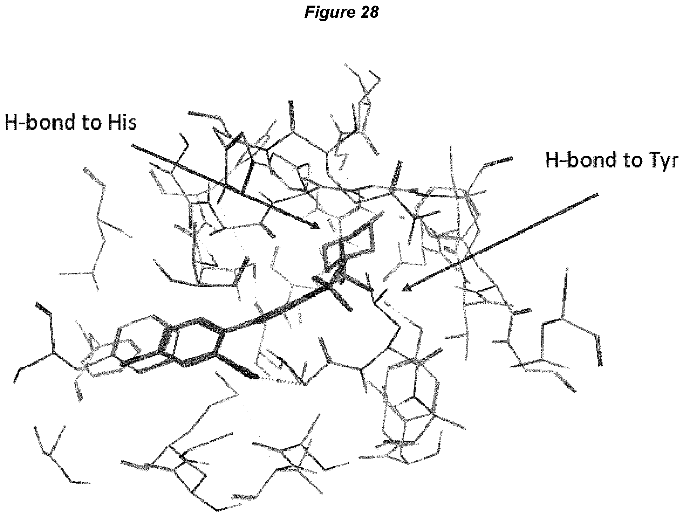

**Figure 28****.** *In silico* model of a MCIM compound of the invention docked in the Q-site of Complex I. This model reveals interactions between a Complex I inhibitor of the invention and NDUFS2 and additional interactions with the neighbouring Complex I subunit NDUFS7.
**Figure 29****.** 3D representation of a pharmacophore model built from *in silico* modelling of drug docking in Complex I.

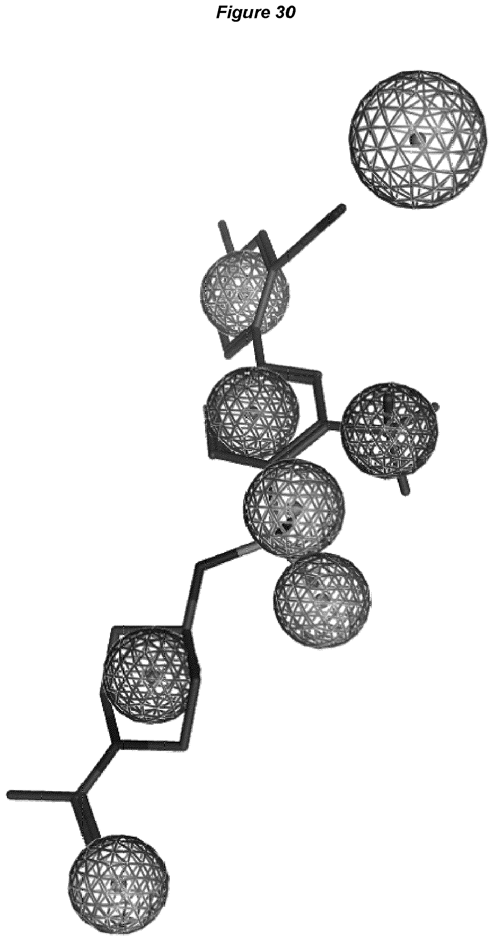

**Figure 30****.** Overlay of a MCIM compound of the invention on the ligand-protein pharmacophore model illustrating successful docking of the MCIM compound of the invention in the Q-tunnel of Complex I.

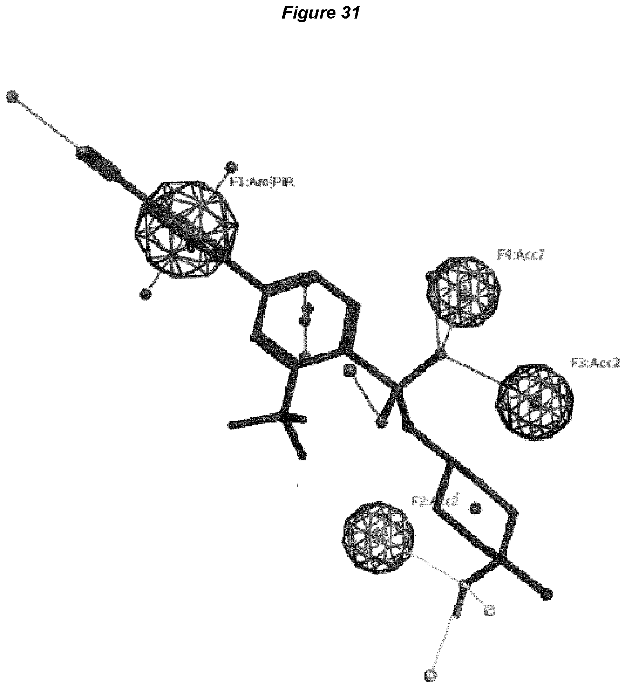

**Figure 31****.** Overlay of a MCIM compound of the invention, CHMSA-02-A, on the ligand-protein pharmacophore model (top panel) and the chemical structure of CHMSA-02-A (bottom panel). This illustrates successful docking of CHMSA-02-A in the Q-tunnel of Complex I.

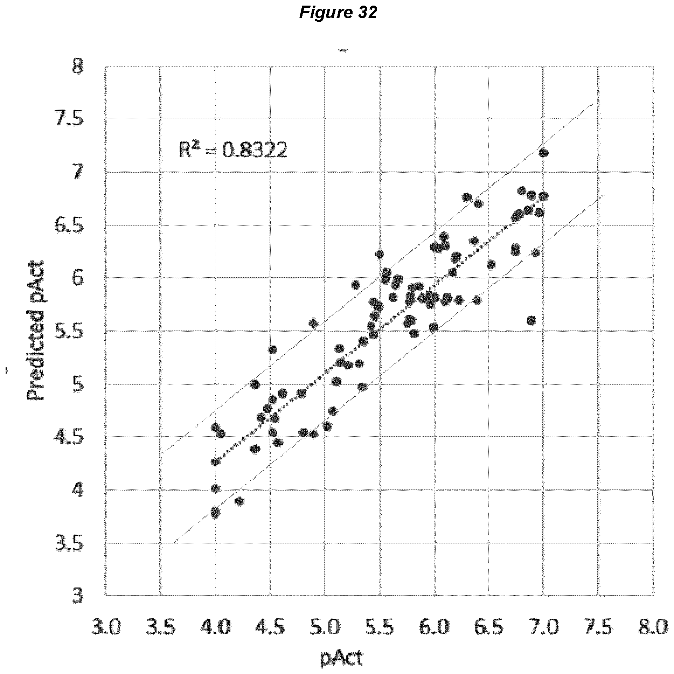

**Figure 32****.** Graph showing the quantitative structure-activity relationship (QSAR) model used to identify further Complex I binders of the invention and to predict their activity in vivo. Predicted pAct of Complex I binders of the invention correlates well with their experimentally validated pAct, with a coefficient of determination (R²) value of 0.8322 demonstrating that this QSAR model can be used to accurately identify Complex I binders of the invention.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

### Inflammatory and/or progressive diseases

As described herein, the mitochondrial Complex I modulator (MCIM) compound is suitable for use in treating inflammatory and/or progressive diseases.

The disease may be a chronic progressive disease, such as: interstitial lung disease (ILD), idiopathic pulmonary fibrosis (IPF); pulmonary fibrosis; liver fibrosis; nonalcoholic steatohepatitis (NASH), nonalcoholic fatty liver disease (NAFLD); kidney fibrosis; chronic kidney disease (CKD); cardiac fibrosis; ischaemia reperfusion injury; heart failure with reduced ejection fraction, heart failure with preserved ejection fraction; myelofibrosis; retroperitoneal fibrosis; atherosclerosis; myocardial infarction; stroke; neurodegenerative disease; multiple sclerosis; fronto-temporal dementia (FTD); amyotrophic lateral sclerosis (ALS), Alzheimer's disease (AD); osteoporosis, osteopenia; osteoarthritis; endometriosis; bone loss associated with endometriosis; neoplasia of bones (including, e.g., as a primary tumour or as metastases and including, e.g., bone cancer; osteosarcoma; or osteoma); cancer-associated bone disease (including, e.g., metastatic bone disease associated with, e.g., breast cancer, lung cancer, prostate cancer, or multiple myeloma; changes in bone mineralisation and density associated with cancer, including, e.g., hypercalcaemia associated with cancer); and bone metastases (including, e.g., osteolytic bone metastases).

The disease may be an autoimmune disease, such as: rheumatoid arthritis (RA); psoriatic arthritis; ankylosing spondylitis; spondyloarthritis; reactive arthritis; infectious arthritis; systemic lupus erythematosus; scleroderma; juvenile idiopathic arthritis; psoriasis; systemic lupus erythematosus; lupus nephritis; uveitis; systemic sclerosis; scleroderma; hepatitis; Sjogren's syndrome; inflammatory bowel disease; ulcerative colitis; Crohn's disease; multiple sclerosis; atherosclerosis; chronic obstructive pulmonary disease (COPD); uveitis; allergic disease (including, e.g., atopy, allergic rhinitis, atopic dermatitis, anaphylaxis, allergic bronchopulmonary aspergillosis, allergic gastroenteritis, hypersensitivity pneumonitis); type I diabetes; celiac disease; oophoritis; primary biliary cirrhosis; insulin-resistant diabetes; Behçet's disease; myasthenia gravis; autoimmune polyneuritis; pemphigus; rheumatic carditis; Goodpasture's syndrome; postcardiotomy syndrome; polymyositis; dermatomyositis; irritable bowel syndrome; pancreatitis; gastritis, chronic pulmonary inflammation; pulmonary alveolitis; polycystic kidney disease; cryopyrin-associated periodic syndrome (CAPS); Muckle-Wells Syndrome; Guillain-Barre syndrome; chronic inflammatory demyelinating polyneuropathy; dermatitis; atopic dermatomyositis; Graves' disease; autoimmune (Hashimoto's) thyroiditis; bronchitis; cystic fibrosis; pulmonary embolism; sarcoidosis; emphysema; respiratory failure; acute respiratory distress syndrome; BENTA disease; or polymyositis; SSC-ILD, hidradenitis suppurative, alopecia areata, atopic dermatitis.

### Evaluating disease repair in humans

Chronic autoimmune diseases are amenable to treatment with the Complex I modulator (MCIM) compound as described herein. Such diseases include RA, IBD, Ulcerative colitis (UC), multiple sclerosis (MS), Psoriatic arthritis (PsA), and psoriasis and fibrotic conditions such as pulmonary fibrosis, liver fibrosis and chronic kidney disease. As described herein, the MCIM compound can elicit tissue repair and disease healing.

In RA and PsA repair/healing can be clinically assessed using imaging methods such as MRI, computer tomography or ultrasonography, and/or by measuring functional responses. For example, the response to treatment in RA may be assessed via the American College of Rheumatology (ACR) Criteria, the Disease Activity Score (DAS) or by patient reported outcomes such as pain or physical function.

In IBD, repair/healing can be clinically assessed using gut transit time, occult blood, endoscopy, histopathology, electrolytes and/or by measuring biomarkers such as pANCA, ASCA, GP2, CUZD1, CHI3L1, GM-CSF, ACA, PS/PT, ALCA, ACCA, AMCA, OmpC, I2, CBir1, Laminarin, Chitin, IFI16, IL-1β, IL-6, IL-8, IL-9, IFN-γ, TNF, CCL2, IL-22, IL-2, and/or IL-6, as disclosed in Chen *et al* (2020) which is incorporated herein in its entirety by reference.

In MS, repair/healing can be clinically assessed using McDonald criteria, Doppler ultrasound, analysis of conduction defects and/or magnetic resonance imaging (MRI).

In psoriasis, repair/healing can be clinically assessed using Doppler ultrasound or by histological analysis of biopsy samples.

In pulmonary conditions, repair/healing can be clinically assessed using high-resolution computed tomography (HRCT), MRI, exacerbation frequency and/or by measuring biomarkers and/or pulmonary surfactant proteins. For example, in pulmonary fibrosis biomarkers which may be measured include ATF3, PPP1R15A, ZFP36, SOCS3, NAMPT, GADD45B, COL15A1, GIMPAP6, JAM2, LMO7, TSPAN13, LAMA3, GDF15, FGF-21, MUC1 and ANXA3, as disclosed in Maghsoudloo *et al* (2020).

In steatohepatitis such as NASH and NAFLD, repair/healing can be clinically assessed using imaging to measure liver distension, biopsy (Ishak score, Metavir score and/or Knodell score) and histopathology, liver function tests such as measures of albumin, total protein, alkaline phosphatase (ALP), aspartate aminotransferase (AST), gamma-glutamyl transferase (GGT), bilirubin, lactate dehydrogenase (LD) or prothrombin time (PT) and/or by measuring biomarkers. For example, in liver fibrosis biomarkers which may be measured include Type IV collagen, laminin, MMPs, TIMPs, YKL-40, P3NP, TGF-b1, MFAP-4, or combinatorial biomarkers such as APRI, AST/ALT ratio, ELF index and/or fibro index, among others (Nallagangula KS et al, 2018).

In chronic kidney disease, repair/healing can be clinically assessed using histological assessment of biopsy samples, kidney functional tests such as glomerular filtration rate (GFR), estimated GFR (eGFR) and/or by measuring biomarkers. For example, in kidney fibrosis biomarkers which may be measured include creatinine, neutrophil gelatinase-associated lipocalin (NGAL), kidney injury molecule-1 (KIM-1), N-acetyl-β-D-glucosaminidase (NAG), liver-type fatty acid binding protein (L-FABP), and uromodulin (UMOD) (Lousa, I et al, 2021).

### Pharmacophores

The term "pharmacophore" is used herein as defined in Wermuth, C.G., Ganellin, C.R., Lindberg, P., Mitscher, L.A.; Glossary of Terms Used in Medicinal Chemistry (IUPAC Recommendations 1998); Pure & Appl. Chem. 70:5 (1998) 1129-1143: A pharmacophore is an ensemble of aromatic steric and electronic features that is necessary to ensure the optimal supramolecular interactions with a specific biological target and to trigger (or block) its biological response. The ensemble of aromatic steric and electronic features represent so-called "pharmacophoric features". Typical pharmacophoric features include, for example, hydrogen bond donor, hydrogen bond acceptor, hydrophobic, aromatic, and positively and negatively ionized areas.

As used herein, the term "pharmacophore model" relates to a pharmacophore hypothesis for the binding interactions in a particular active site. A pharmacophore model is made up of a set of annotation points which are interrelated in 3D space. The annotation points show the location and type of biologically important atoms and groups, i.e., each annotation point relates to a pharmacophore feature of the model. Each annotation point is associated with a radius that describes the permissible variation in 3D space for the location of the given pharmacophoric feature.

As used herein, the phrase "conform to a pharmacophore model" means that a compound described herein binds to the target binding site (i.e., the NDUSF2 and/or NDUSF7 binding site) in a 3D conformation (i.e., "pose") whereby, 4 or more of the annotation points of the pharmacophore model described herein are occupied by corresponding features of the compound described herein, as determined the unified annotation scheme in *Molecular Operating Environment (MOE),* 2022.02 Chemical Computing Group ULC, 1010 Sherbooke St. West, Suite #910, Montreal, QC, Canada, H3A 2R7, 2022. In some embodiments, four or more of the annotation points of the pharmacophore model described herein are occupied by corresponding features of the compound described herein. In some embodiments, five or more, six or more, seven or more, eight or more, or all nine of the annotation points of the pharmacophore model described herein are occupied by corresponding features of the compound described herein. The conformance of a compound described herein with the pharmacophore features is as determined using the unified annotation scheme in *Molecular Operating Environment (MOE),* 2022.02 Chemical Computing Group ULC, 1010 Sherbooke St. West, Suite #910, Montreal, QC, Canada, H3A 2R7, 2022

Annotation points can be broadly divided into three categories: *atom, projected,* and *centroid.* Annotation points are determined for a given compound by the unified annotation scheme in *Molecular Operating Environment (MOE),* 2022.02 Chemical Computing Group ULC, 1010 Sherbooke St. West, Suite #910, Montreal, QC, Canada, H3A 2R7, 2022.

The annotation points used herein are described below.

### Atom

### Don: H-bond Donor

### Acc: H-bond Acceptor

*Atom* annotations are located directly on an atom of a molecule and typically indicate a function related to protein-ligand binding.

Don annotates an H-bond donor heavy atom. A Don annotation is added to all oxygen and nitrogen atoms with at least one (possibly implicit) hydrogen attached.

Acc annotates an H-bond acceptor heavy atom. O, S, and N elements can be hydrogen bond acceptors provided that they conform to the following rules:
1. Sulfur atoms are not acceptors except that sulfur in S=C groups and anionic sulfurs are acceptors and are given Acc annotations.
2. Nitrogen atoms are acceptors and given Acc annotations provided that they are not buried. A buried nitrogen is one of {=N=, -N#, >N=, >N<} or a non-3-ring conjugated nitrogen of the form {>N-π, >N-[C+], >N-B, >N-S=O, >N-P=O}.
3. Oxygen atoms are acceptors and are given Acc annotations provided that they are not buried and provided that they are not in certain exception groups. A buried oxygen is one of {=O=, -O#; >O=, >O<} or a non-3-ring conjugated oxygen of the form {>O-π, >O-B, >O-[C+]}. Non-buried oxygen atoms are acceptors unless they are part of the following exception groups:

| | |
|---|---|
| 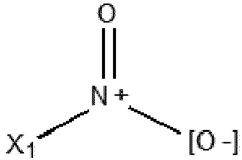 | 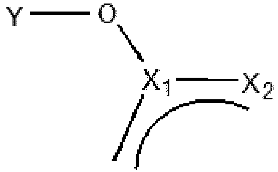 |
| 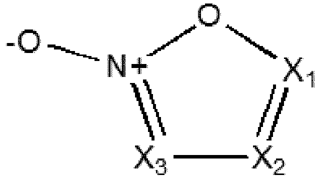 not Acc; X*i* = any | 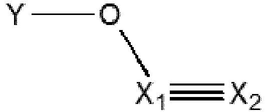 |
| | 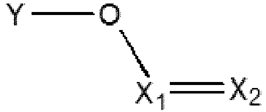 not Acc if any X*i* = het; Y ≠ H |
| 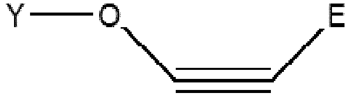 | |
| 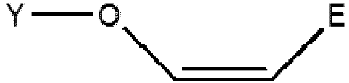 | 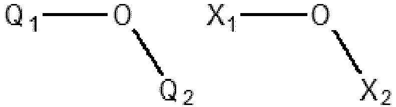 not Acc if both {Q1,Q2} are π |
| 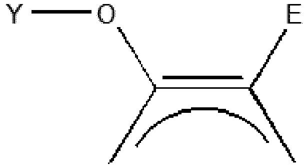 | |
| | not Acc if both {X1,X2} = het |
| not Acc if Y ≠ H, E = {C#N, N#C, >[N,S,P]=O, Q+, CFs} | |

### Projected

### Don2: Projected Donor

### Acc2: Projected Acceptor

*Projected* annotations are (typically) located along implicit lone pair or implicit hydrogen directions and are used to annotate the location of possible hydrogen bond or metal ligation partners, or possible R-group atom locations.

Projected **Don2** annotations are added according to the hybridization and the heavy atom coordination of the donor. In the following table the d denotes a **Don2** feature.

| **Table 1** | | |
|---|---|---|
| Projected donors | | |
| 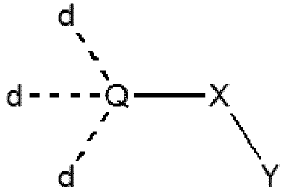 Q = 1° *sp*³, X >1-coord, Y ≠ H | 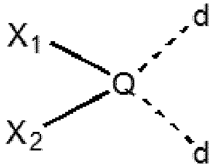 Q = 2° *sp*³ | 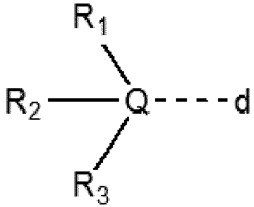 Q = 3°*sp*³ |
| 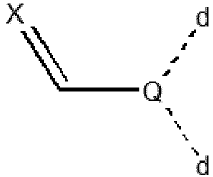 Q = 1° *sp*² | 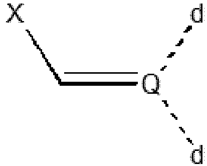 Q = 1° *sp*², X ≠ H | 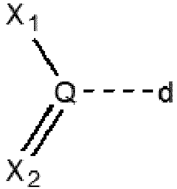 Q = 2° *sp*² |
| 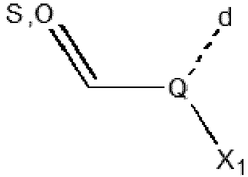 Q : 2° *sp*² | 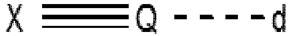 Q = 1° *sp* | |

Hydrogen bond **Acc2** projected annotations are added to those heavy atoms that qualify as H-bond acceptors (see above) and are given Acc annotations. The **Acc2** projected annotations are added in the same locations as those for the **Don2** projected annotations and according to the same rules. (That is, the donors and the acceptors are projected using the same angles and the same distances.) An atom that is both a Don and an Acc will be annotated with "Don2&Acc2" projected annotations.

Projected annotations such as **Don2,** and **Acc2** are located at potential heavy atom positions. For example, **Don2** indicates a potential hydrogen bond partner heavy atom. Realistically, this partner atom cannot have too much overlap with any of the atoms of the molecule generating the projected annotation. This condition depends on the particular conformation of a molecule and cannot be reliably predicted by topological means. Consequently, a *solvent exposure test* must be applied to validate any hits resulting from a Pharmacophore Search, i.e., a test to verify that applicable projected features are not covered by other parts of the conformation (that would prevent the putative projection atom from occupying the intended position).

### Centroid

### Aro Aromatic

### Hyd Hydrophobic

*Centroid* annotations (Aro, Hyd) are located at the geometric center of a subset of the atoms of a molecule.

**Aro** annotation centroids are used for aromatic and pseudo aromatic rings. The **Aro** annotation centroid is placed at the centroid of each aromatic ring (e.g. two centroids in naphthalene).

The definition of aromaticity is generous (a Daylight-style definition) in which each ring is treated in isolation and a Hückel 4n+2 rule is applied. C=O carbons count 0 electrons, otherwise C=R count as 1; N=X nitrogens count 1 and >N- nitrogens and -O- oxygens count 2 electrons. Thus, the following are treated as (pseudo) aromatic and are given an Aro annotation centroid in the center of the ring:

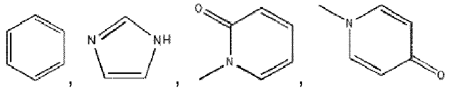

Hydrophobic atoms are annotated with **HydA** and hydrophobic centroids are annotated with Hyd. Hydrophobic groups are determined by graph theoretic algorithms.

The fundamental rules for deciding whether an atom is hydrophobic are summarized below:
1. Nitro nitrogen atoms (not in nitrate anions) are hydrophobic.
2. Divalent sulfur atoms with two heavy neighbors bonded only to carbon or sulfur are hydrophobic.
3. Halogens are hydrophobic.
4. Carbon atoms are hydrophobic except
   a) aliphatic carbons π bonded to non-carbon atoms; or
   b) π carbon atoms adjacent to univalent oxygen; or
   c) aromatic carbon adjacent to aromatic oxygen in 5-rings; or
   d) carbon atoms adjacent to two or more {N,O} atoms; or
   e) anionic carbons in c1cccc1;

The assignment of hydrophobic annotations proceeds by first applying the preceding hydrophobic atom typing rules but leaving out fluorine atoms on the grounds that they are small and should not affect annotation placement.

The Unified scheme provides an atom-centered hydrophobic annotation, **HydA** and a centroid hydrophobic feature Hyd. The **HydA** annotation is used for hydrophobic atoms that are deemed to have sufficiently high (potential) exposure to a potential receptor. This means that, for example, *sp*³ carbons with 4 heavy neighbors are not marked (since they are buried) and aromatic carbons with two heavy neighbors and two *ortho* substituents are not annotated.

The Hyd annotations are assigned by a procedure that groups connected hydrophobic atoms and assigns centroids weighted by an estimate of the likely exposed surface area of each hydrophobic atom; that is, the Hyd centroid will be placed closer to more exposed hydrophobic atoms in a hydrophobic group.

The following grouping algorithm is used:
**1. Strip Fluorines.** Remove all fluorines that are not bonded to {H,F} from further consideration. Note that estimates of exposed surface area use a fluorine suppressed molecule.
**2. Rings.** Find all 5-, 6-, 7-, and 8-member rings that are not composed of smaller rings. For each such ring, extract each contiguous stretch of hydrophobic atoms with at least three atoms that have a sufficiently high total exposed surface area, and generate a surface area weighted centroid annotation. Remove all annotated ring atoms from further consideration.
**3. Components.** Find all connected components (single-linkage clusters) among the remaining hydrophobes and remove from consideration those clusters with an exposed surface area sum deemed too small (less than a -CH2- group).
**4. Small Components.** For the remaining hydrophobic components with three or fewer atoms, generate an exposed surface area weighted centroid annotation.
**5. Large Components.** Identify the center of the component graph and generate a weighted centroid at the center including its neighbors. Remove the annotated atoms, splitting the component and apply the Small Components step and/or the Large Components step (recursively) to group the hydrophobes.

The compounds described herein conform to a pharmacophore model as described herein.

### Sequence Listing

Human NDUFS2 amino acid sequence SEQ ID NO: 1:
Human NDUFS7 amino acid sequence SEQ ID NO:2:

In some embodiments, tissue repair, disease regression, increase in reparative cells, decrease in destructive cells and/or reduction in cytokine production from peripheral mononuclear cells or lymphoid cells (such as T-, B- or NK-cells) can be determined as defined herein. For instance, in some embodiments, the compound reduces the levels of inflammatory mediators such as TNFα, eSEL, CD38, CD40, CD69, slgG, sIL-17A, sIL-17F, sIL-2 and/or sIL-6 that are produced by a target cell. In some embodiments, the compound reduces the levels of collagen type I and/or MMP1, and/or increases collagen type IV production. In some embodiments, the compound increases ETC efficiency, without an increase in biomass. In some embodiments, the compound decreases ETC efficiency. In some embodiments, the compound reduces cellular proliferation without reducing ATP concentration/cell and viability, dependent on the environmental composition such as in the absence of pyruvate. In some embodiments, the compound induces an adaptive/repair response under conditions of metabolic stress conditions. For instance, the compound modulates Complex I activity and attenuates high energy processes such as proliferation and/or differentiation and concurrently induces an adaptive/repair response by increasing the production of pro-angiogenic/repair factors such as VEGF to restore tissue metabolic homeostasis, particularly under metabolic stress conditions.

In some embodiments, the compound reduced cell viability, particularly in cell types which are heavily dependent on Complex I metabolism and lack the metabolic flexibility to adapt. Each of these effects on tissue repair, disease regression, increase in reparative cells, decrease in destructive cells and/or reduction in cytokine production from pro-inflammatory myeloid cells can be determined by comparing the effect in the presence and absence of the compound.

In some embodiments, lung fibroblasts are used as a target cell to determine the effect of the compound. For example, cellular adaptation may be determined in human primary lung fibroblasts, e.g. by measuring vascular endothelial growth factor (VEGF) secretion. (VEGF can be induced in a cell that is not receiving enough oxygen or nutrients to support ATP production. This can indicate how metabolic signalling pathways interact and integrate with angiogenic signalling events and repair). In some embodiments, VEGF secretion can be measured by plating primary human lung fibroblasts at 2×10³ cells/well in 96-well plates in 100 µL DMEM complete medium with: 1 g/L glucose and 110 mg/L pyruvate; or 1 g/L glucose without pyruvate (each supplemented with 1% penicillin-streptomycin and 10% heat inactivated foetal bovine serum), and incubating in a humidified 37°C incubator with 5% CO₂ for 24 hours. The test compound is prepared as a 10x final concentration solution in culture medium and added to final concentrations before further incubating the cells at 37°C / 5% CO₂ for 24 hours. VEGF secretion is measured in the cell supernatants using Quantikine^{®} ELISA Human VEGF kits according to the manufacturer's instructions. Absorbance at 450 nm is measured on a BMG Plate reader (CLARIOstar plus) using pathlength correction. Background absorbance is measured at 540 nm. Preferably, treatment with the test compound results in an increase in VEGF secretion in the absence of pyruvate, but not in the presence of pyruvate as a metabolic substrate for the cell. This indicates the induction of an adaptive/repair response under conditions of metabolic stress conditions via the attenuation of highly energy-consuming processes such as proliferation and concurrent production of pro-angiogenic/repair factors such as VEGF to restore tissue metabolic homeostasis.

In some embodiments, the compound increases ramified (`resting') microglia and/or increases mean oligodendrocyte progenitor cell (OPC) score in MS subjects, compared with MS subjects treated with a negative control.

In some embodiments, a histopathological assessment can be used to determine the effect of the compound on lung fibrosis: Histopathological assessment of the biological effects of the compounds on fibrosis may be performed on lung sections stained with Masson's Trichome (MT) to assess fibrosis using the Ashcroft score, and Haematoxylin and Eosin (H&E) to assess parameters of vascular inflammation, bronchiolar inflammation, neutrophil inflammation, lymphoid infiltration, monocyte/macrophage infiltration, pneumonitis, apoptosis/necrosis, AT2 cell hypertrophy and AT2 cell hyperplasia. The histology outputs are assessed on a scale of 0-5 as follows:
0: normal. 1: minimal, focal. 2: moderate, focal. 3: moderate, multi-focal or diffuse. 4: marked, focal. 5: marked, multi-focal or diffuse. In some embodiments, the compound reduces the average score compared with subject treated with a negative control.

In some embodiments, to assess fibrosis in the lung, lung sections can be graded from 0 (normal lung) to 8 (total fibrous obliteration of the section) as described in Hubner *et al* (2008). The Ashcroft score is assessed on a scale of 0-8 as follows:
0: Normal lung. 1: Minimal fibrous thickening of alveolar or bronchiolar walls. 2. 3 Moderate thickening of walls without obvious damage to lung architecture. 4. 5: Increased fibrosis with definite damage to lung structure and formation of fibrous bands or small fibrous masses. 6. 7: Severe distortion of structure and large fibrous areas; "honeycomb lung" is placed in this category. 8: Total fibrous obliteration of the field.

In some embodiments, a histopathological assessment can be used to determine the effect of the compound on IBD: Ileo-caecal issue sections can be stained with Haematoxylin and Eosin (H&E) and parameters of inflammation, mucosal erosion, epithelial hyperplasia, epithelial metaplasia, mucus cell metaplasia, and fibroplasia are assessed on a scale of 0-5 as follows:
0: normal. 1: minimal, focal. 2: moderate, focal. 3: moderate, multi-focal or diffuse. 4: marked, focal. 5: marked, multi-focal or diffuse. In some embodiments, the compound reduces the average score compared with subject treated with a negative control.

In some embodiments, a histopathological assessment can be used to determine the effect of the compound on multiple sclerosis (MS). For this assessment of MS, spinal cord sections can be stained with Masson's Trichome (MT) to assess fibrosis using the Ashcroft score, and Haematoxylin and Eosin (H&E) to assess parameters of inflammation, demyelination and pyknosis. The following scoring system is used to assess these parameters:
Grade 0: Normal, no pathology. Grade 1: minimal, Single focal lesion in one section. Grade 2: moderate, Single focal lesion in 2+ sections. Grade 3: moderate, multi-focal lesions in one section. Grade 4: multi-focal lesions in 2+ sections. Grade 5: marked, diffuse pathology

In some embodiments, sections can be stained using A2B5 immunohistochemistry to assess oligodendrocyte progenitor cells. Cells are counted to assess this parameter.

In some embodiments, a histopathological assessment can be used to determine the effect of the compound on arthritis: For this assessment of arthritis, the following signs are monitored in digits or limbs of each subject three times per week and summed to generate the Arthritic Index (Al). (The maximum AI for one animal is 16):
0: no visible effects of arthritis. 1: oedema and/or erythema of 1 digit. 2: oedema and/or erythema of 2 digits. 3: oedema and/or erythema of more than 2 digits. 4: severe arthritis of entire paw and digits.

In some embodiments, the compound reduces the average score compared with subject treated with a negative control.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

### Examples

### EXAMPLE 1 - BioMAP profiling

A high-throughput integrative biology platform (BioMAP ^{®}) was used to profile the effect of MCIM compounds on multiple disease-associated regulatory pathways. BioMAP^{®} has been developed as a method to assess efficacy, safety and the mechanism of action of drugs in multiple human cell types stimulated with inflammatory challenges as described in US6656695, which is incorporated herein in its entirety. The BioMAP system reflects human disease pathology and has the ability to detect and distinguish the effects of approved drugs and investigational human therapeutic compounds. BioMAP technology enables rapid determination of efficacy, side effects and mechanism of action of drug candidates.

BioMAP^{®} provides an unbiased, target-agnostic and data-driven approach to understanding compound or combination therapy impact on human disease models and translational biomarkers. The system is validated with clinically approved drugs and known test agents. The principle of the assay is to test compounds in human primary cell-based disease systems, and compare the data with a Reference Database of over 4,500 compounds. The profile of a compound can be compared against a reference compound to see if the biological activity of the test item is differentiated from the reference.

The activity of MCIM compounds was determined in two BioMAP systems; Fibrosis panel and Diversity Plus.

Twelve primary human cell and co-culture assays were used to assess the effects of MCIM compounds on clinically relevant protein biomarkers of inflammation, cell growth, and fibrosis as part of the quality controlled BioMAP^{®} Diversity PLUS, commercially available service (Eurofins DiscoverX Corporation, Freemont, CA, USA; for full details https://www.discovero.com). Briefly, the BioMAP^{®} panels consist of human primary cell-based systems designed to model different aspects of the human body in an in vitro format. The 12 cell assays utilised in the Diversity PLUS panel allow characterisation of test agent responses in an unbiased way across a broad set of systems modelling various human disease states compared to historical controls. BioMAP^{®} panels are constructed with primary cell types from healthy human donors, with stimuli (such as cytokines or growth factors) added to capture relevant signalling networks that naturally occur in human tissue or pathological conditions.

MCIM Compounds were tested in these assays at four concentrations: 4000 nM, 1300 nM, 400 nM & 150 nM. Human primary cells employed in the BioMAP^{®} systems were used at passage 4 or earlier, derived from multiple donors (n = 2-6), commercially purchased and handled according to the recommendations of the manufacturers. Human blood derived CD14 + monocytes were differentiated into macrophages in vitro before being added to the LPS system (Eurofins DiscoverX Corporation).

The human cell types and stimuli used in each assay system were as follows: 3 C system [human umbilical vein endothelial cells (HUVEC) + (IL-1β, TNFα and IFNy)], 4H system [HUVEC + (IL-4 and histamine)], lipopolysaccharide (LPS) system [peripheral blood monocyte cells (PBMC) and HUVEC + LPS (TLR4 ligand)], Sag system [peripheral blood mononuclear cells, PBMC and HUVEC + TCR ligands], BT system [CD19 + B cells and PBMC + (α-IgM and TCR ligands)], BF4T system [bronchial epithelial cells and human neonatal dermal fibroblasts, HDFn, + (TNFα and IL-4)], BE3C system [bronchial epithelial cells + (IL-1β, TNFα and IFNy)], CASM3C system [coronary artery smooth muscle cells + (IL-1β, TNFα and IFNγ)], HDF3CGF system [HDFn + (IL-1β, TNFα, IFNγ, EGF, bFGF and PDGF-BB)], KF3CT system [keratinocytes and HDFn + (IL-1β, TNFα, IFNy and TGFβ)], MyoF system [differentiated lung myofibroblasts + (TNFα and TGFβ)], SAEMyoF system [small airway epithelial cells and differentiated lung myofibroblasts + (TNFα and TGFβ)], ReMyoF system [renal epithelial cells and differentiated lung myofibroblasts + (TNFα and TGFβ)] and IMphg system [HUVEC and M1 macrophages + Zymosan (TLR2 ligand)].

Assays were derived from either single cell types or co-culture systems. Adherent cell types were cultured in 96 or 384-well plates until confluence, followed by the addition of PBMC (Sag and LPS systems). The BT system consisted of CD19 + B cells co-cultured with PBMC and stimulated with a BCR activator and low levels of TCR stimulation. Test agents prepared in either DMSO (small molecules; final concentration ≤ 0.1%) or PBS (biologics) were added at the indicated concentrations 1 h before stimulation, and cells remained in culture for 24 h or as otherwise indicated [48 h, MyoF system; 72 h, BT system (soluble readouts); 168 h, BT system (secreted IgG)]. Each assay plate contained negative controls (e.g., non-stimulated conditions) and vehicle controls (e.g., 0.1% DMSO) appropriate for each system. Direct ELISA was used to measure biomarker levels of cell-associated and cell membrane targets. Soluble factors from supernatants were quantified using either HTRF^{®} detection, bead-based multiplex immunoassay, or capture ELISA. Overt adverse effects of (compounds) on cell proliferation and viability (cytotoxicity) were detected by sulforhodamine B (SRB) staining for adherent cells, and alamarBlue^{®} reduction for cells in suspension. For proliferation assays, individual cell types were cultured at subconfluence and measured at time points optimised for each system (48 h: 3 C and CASM3C systems; 72 h: BT and HDF3CGF systems; 96 h: Sag system). Cytotoxicity for adherent cells was measured by SRB (24 h: 3 C, 4H, LPS, Sag, BF4T, BE3C, CASM3C, HDF3CGF, KF3CT, and IMphg systems; 48 h: MyoF system), and by alamarBlue^{®} staining for cells in suspension (24 h: Sag system; 42 h: BT system) at the time points indicated.

Biomarker measurements in treated samples were divided by the average of the control samples (at least 6 vehicle controls from the same plate) to generate a ratio that was then log10 transformed. Significance prediction envelopes were calculated using proprietary historical vehicle control data at a 95% confidence interval. Biomarker activities were annotated when two or more consecutive concentrations change in the same direction relative to vehicle controls were outside of the significance envelope and had at least one concentration with an effect size > 20% (log10 ratio> 0.1). Biomarker key activities were described as modulated if these activities increase in some systems but decrease in others. Cytotoxic conditions were noted when total protein levels decreased by more than 50% (log10 ratio of SRB or alamarBlue^{®} levels < -0.3) and were indicated by a thin black arrow above the X-axis. A compound was considered to have broad cytotoxicity when cytotoxicity was detected in 3 or more systems. Concentrations of test agents with detectable broad cytotoxicity were excluded from biomarker activity annotation and downstream benchmarking, similarity search and cluster analysis. Antiproliferative effects were defined by an SRB or alamar Blue^{®} log 10 ratio value < -0.1 from cells plated at a lower density and were indicated by grey arrows above the X-axis. Cytotoxicity and antiproliferative arrows only require one concentration to meet the indicated threshold for profile annotation.

| **Table 2.** | | | |
|---|---|---|---|
| Key activities regulated by various MCIM compounds in BioMAP experiments. | | | |
| | ABD599 | ABD900 | HMC-C-01-A |
| System | All | Sag and BT | Sag, BT, MyoF, SAEMyoF, ReMyoF |
| Inflammation | Decreased Eotaxin-3, VCAM-1, P-selectin, E-selectin, MCP-1, IL8, IP-10, ITAC, and sTNFα | decreased Esel, IL8, sTNFα | decreased Esel and sTNFα |
| | | | decreased M-CSF, ITAC, MCP-1, sIL6 |
| Immune modulation | Decreased HLA-DR, CD38, CD40, CD69, slgG, sIL-17A, sIL-17F, sIL-2, sIL-6, and sIL-10 | decreased CD69, slgG, sIL-17F, sIL-2, sIL-6 | decreased CD38, CD40, CD69, slgG, sIL-17A, sIL-17F, sIL-2 and sIL-6 |
| Tissue remodeling | Increased PAI-1 and Collagen-IV; | | Decreased Coll I, MMP1, MMP9 |
| | decreased uPAR, tPA, and MMP1 | | Increased Coll IV |
| Myofibroblast activation | | | Decreased aSMA |

Figure 2 shows the BioMAP profile of several MCIM compounds. (A) ABD599 tested in the full bioMAP profile shows decreased inflammation and immune modulation, and tissue remodelling with increased collagen IV levels. (B) HMC-C-01-A shows anti-inflammatory and immune modulation activity in the BT and Sag systems and (C) HMC-C-01-A in the fibrosis panels increases Collagen type IV and (D) BioMAP Sag and BT profiles of ABD900.

The data demonstrate that MCIM compounds have a distinct phenotypic profile with multi-modal actions, having specific effects in different cell types following stimulation with different inflammatory mediators. In particular, the MCIM compounds modulate immune activities by reducing the levels of inflammatory mediators such as TNFα, e-Selectin, CD38, CD40, CD69, slgG, sIL-17A, sIL-17F, sIL-2 and sIL-6. In addition, the MCIM compounds show potential for tissue remodelling as indicated by the decrease in Col I and MMP1. Particularly of note, the MCIM compounds increase production of Coll IV, a critical basement membrane collagen which is involved in tissue repair and remodelling activities.

### EXAMPLE 2 - Mitochondrial phenotype assessment in osteoclasts

The ability of mitochondria to undergo fusion and fission processes is essential to mitochondrial function and cellular health. Qualitative and/or quantitative changes in the mitochondrial reticulum are also observed under pathological conditions that are caused by inherited mutations in mitochondrial DNA or in nuclear OXPHOS genes and suggest a tight relationship between mitochondrial structure and function. In addition, several lines of evidence suggest that the damage response of injured cells can be ameliorated by the presence of healthy mitochondria (Jin,*et al*, 2019, which is hereby incorporated by reference in its entirety). Studying mitochondrial form and function may therefore yield important insights into the potential of cells and tissues to recover from damage. Osteoclasts are a highly energetic cell type sensitive to changes in mitochondrial metabolism that are suitable for evaluating such relationships.

Peripheral blood mononuclear cells were isolated from human whole blood by differential centrifugation over Ficoll-Paque PLUS (GE Healthcare Biosciences). CD14+ monocytes were purified from the freshly isolated PBMCs using the CD14+ selection kit (StemCells. UK) by positive magnetic selection according to manufacturer's instructions . Cells were differentiated to osteoclasts by adding 1×10⁶ ml-1 cells in complete minimum essential medium-alpha supplemented with 10% heat inactivated fetal bovine serum (FBS, Invitrogen,UK),2mM glutamine (Invitrogen,UK) 20U ml-1 penicillin, 100ug/ml streptomycin (Sigma ALdrich, UK) along with 25ng ml-1 recombinant human M-CSF (Peprotech, UK) and 25 ng ml-1 RANKL for 6 days. On th^{e} 6th day, the cells were treated with test compound (final concentration 0.03-1 uM, 0,05 % DMSO) or a control, rotenone (100 nM).

Adherent cells were fixed in 2.5% glutaraldehyde in 0.1 M cacodylate buffer, pH 7.4, for 1 h, post fixed with 1% osmium tetroxide (Electron Microscopy Science), dehydrated in a graded series of ethanol, and embedded in Epon (Electron Microscopy Science). The embedded samples were sectioned by an ultramicrotome (Ultracut E, Richert-Jung, Leica Microsystem). Thin sections (90 nm thick) were collected on 300 mesh nickel grids and stained with uranyl acetate (Electron Microscopy Science) and lead citrate. Samples were observed by using a Zeiss EM 109 apparatus (Zeiss). Images were captured using a Nikon digital camera Dmx 170 1200F and ACT-1 software.

Representative images are shown in Figure 3. (A) Control cells show a heterogenous and dynamic population of mitochondria with a good balance of fusion and fission (mitochondria (M) and endoplasmic reticulum (ER)). (B) cells treated with the archetypal Complex I inhibitor, rotenone show increased numbers of abnormal mitochondria which were more rounded and had condensed cristae with evidence of fragmentation, and evidence of lysosomes (L) close by. (C) 0.03 µM ABD900 showed increased tubular mitochondrial with evidence of extrusion budding, consistent with an adaptive change in mitochondrial structure to increase mitochondrial area without an overt increase in organelle biomass. Dumbbell morphology, consistent with the formation of electron transport chain (ETC) super-complexes, were also observed. (D) 0.1 µM ABD900 showed a similar profile with evidence of cristae refraction. (E) 0.3 µM ABD900 shows filamentous extensions in many mitochondria consistent with an attempt to enlarge cristae volume. (F) 1 µM ABD900 shows a heterogenous mitochondrial population with rounded morphology and condensed cristae.

The results demonstrate that, in contrast to the classical Complex 1 blocker, rotenone, the MCIM compounds of the invention elicit a mitochondrial phenotype consistent with differentiation based on maturity. For example, older mitochondria, which are usually cleared by mitophagy, are retained as part of an integrated stress response. To increase ETC efficiency, without an overt increase in biomass, structural adaptation occurs to maintain cellular free energy.

### EXAMPLE 3 - Cellular metabolism and viability

The *in vitro* effects of test compounds on cellular metabolism and viability were determined by incubation with human primary lung fibroblasts followed by measurement of cellular ATP concentrations and cell counts.

ATP is an organic compound which can be produced by several cellular processes such as glycolysis and oxidative phosphorylation. However, if levels of oxygen, or substrates to fuel oxidative phosphorylation are insufficient, cells can reprogramme their metabolism towards glycolysis or other pathways to maintain their availability of ATP. Depending on the environment of the cell, such changes in metabolism may be accompanied by adaptive changes in gene expression. For example, under certain culture conditions, cells may upregulate an 'adaptive response' gene known as vascular endothelial growth factor (VEGF); VEGF encodes a proangiogenic protein (VEGF), whose function to induce new blood vessel formation to seek out new sources of oxygen and nutrients. This is critical to eliciting a functional repair response. By modulating the activity of Complex I, the compounds of the invention modulate ATP production, and thereby induce a cellular adaptation response.

The effects of the compounds on cellular adaptation was assessed in vitro in human primary lung fibroblasts (HLF). Cells were cultured with different metabolic substrates- glucose, L-glutamine, and pyruvate- to explore potential changes according to the microenvironment of the cell. Cells cultured in these conditions were treated with compound at various concentrations, and intracellular ATP concentrations, nucleus counts, and VEGF secretion were measured to quantify cell viability, metabolism, and indications of adaptive response.

Human primary lung fibroblasts were plated at a concentration of 2 × 10³ cells/well in 96-well plates in 100 µL DMEM complete media (5.5 mM glucose, 2 mM L-glutamine, and 1 mM pyruvate) containing 1% penicillin-streptomycin and 10% heat inactivated foetal bovine serum. Cells were incubated at 37°C / 5% CO₂ overnight to allow cell attachment. On the day of the treatment, cell culture medium was removed, and the cells were washed three times with Gibco^{™} DMEM, no glucose, no L-glutamine, no pyruvate, no phenol red + 1% penicillin-streptomycin and 10% heat inactivated foetal bovine serum (termed 'basal' culture medium). Fresh cell culture supplemented with 5.5 mM glucose alone, 5.5 mM glucose and 2 mM L-glutamine, or 5.5 mM glucose, 2 mM L-glutamine, and 1 mM pyruvate was added to the wells. Test compounds were prepared as 10x final concentration solutions in each appropriate culture medium. Compounds were added to the cultures at 1x final concentration and incubated at 37°C / 5% CO₂ for 1 or 3 days. On day 1, VEGF secretion was measured from cell culture supernatants; 72 hours post treatment, cells were assayed for nucleus counts and ATP generation.

To assess nucleus counts, formaldehyde was added to a final concentration of 4%. Following a further 20-minute incubation at room temperature, the medium was aspirated, the cells were washed twice with 200 µL TBS-T (1x TBS + 0.1% Tween 20) and the nuclei were stained with 50 µL of PBS containing 10% heat inactivated foetal bovine serum, 0.1% Triton-X-100 and 2 µM Hoechst dye. Following a 30-minute incubation at room temperature, protected from light, the cells were washed twice with TBS-T, and cells were counted in 100 µL PBS solution using an ImageXpress Pico system with stitched plate acquisition (4x magnification), DAPI channel (50ms exposure, -3 digital confocal setting).

To assess ATP concentration, 50 µL of reconstituted ATPlite substrate solution (ATPlite 1step Luminescence Assay System, Perkin Elmer) was added to the cells. After a 5-minute incubation at room temperature on a plate shaker, luminescence was measured on a BMG Plate reader (PHERAstar) using LUM plus module, gain 3000, OR 96/384 aperture spoon (type A3).

The average values across the concentrations tested were then plotted and the half-maximal inhibitory concentration (IC₅₀) for the effect on ATP or nuclei counts was calculated by fitting the data to a four-parameter IC₅₀ equation using GraphPad Prism software (v9). ATP readout per nuclei count was calculated by dividing ATPlite luminescence readout values by the Hoechst-stained nuclei count number per test concentration.

To assess VEGF secretion, cell supernatants of three replicate wells were combined and VEGF secretion was measured following the Quantikine^{®} ELISA Human VEGF kit manufacturer instructions. Absorbance at 450 nm was measured on a BMG Plate reader (CLARIOstar plus) using pathlength correction. Background absorbance was measured at 540 nm.

Data were normalised to a VEGF standard curve and expressed as the average of the control wells in pg/mL. Data were plotted and the IC₅₀ for the effect on VEGF secretion was calculated by fitting the data to a four-parameter IC₅₀ equation using GraphPad Prism software (v9).

The results are shown in Figure 4.

Figure 4A consists of 3 panels showing intracellular ATP (left panel), nucleus counts (middle panel) and ATP readout per cell (right panel) after 72 hrs incubation with MCIM compound. In cells cultured in glucose-supplemented media (squares), there was no effect of the compound on intracellular ATP levels, nuclei count or ATP readout per cell. In cells cultured in media supplemented with glucose and glutamine (open circles), there was an increase in intracellular ATP levels and nucleus counts given vehicle compared to cells cultured with glucose alone. Treatment of cells cultured in medium supplemented with glucose and glutamine with the MCIM compound reduced intracellular ATP levels and nucleus counts, in a concentration-dependent manner, with no effect on ATP levels per cell. When the basal culture media supplemented with glucose, L-glutamine was enriched with pyruvate (filled circles), the effect of the compound on intracellular ATP and nucleus counts seen in the medium supplemented with glucose and L-glutamine alone was eliminated.

The results show that in cells cultured in basal medium containing glucose, the MCIM compound had showed no cytotoxicity and had no effect on cell viability. When the cells were cultured in medium supplemented with glucose and L-glutamine, there was an increase in intracellular ATP levels and nucleus counts reflecting that L-glutamine is a precursor amino acid essential for cellular proliferation. The reduction in intracellular ATP and nucleus counts seen in the cells cultured with glucose and L-glutamine, and lack of effect on ATP levels per cells shows that the cells were adapting to the metabolic effects of Complex I modulation by the compound by limiting highly energy-demanding processes, such as cellular proliferation, in order to maintain their intracellular levels of ATP. In addition, the reversal of this effect with the medium was supplemented further with pyruvate (glucose + L-glutamine + pyruvate condition) shows that the adaptive response observed (reduction of cell proliferation) is dependent on nutrient availability to the cell.

Figure 4B shows that in complete medium, there is no effect of the MCIM compound on VEGF secretion (filled circles (●),), but in the absence of pyruvate in the media (open circles (○)), the compound elicited a concentration-dependent increase in VEGF secretion (half-maximal inhibitory concentration (IC₅₀) = 112 nM).

The results show that by modulating Complex I, in the absence of an excess of substrate, cells trigger an adaptive response to cause growth and repair by upregulating VEGF. The absence of an effect when pyruvate was present in the medium, shows that the adaptive repair response is also dependent on nutrient availability.

In summary, the results show that treatment with MCIM compounds of the invention induces an adaptive response to enable cells to maintain their supply of ATP. When cells are treated with MCIM compound, they attempt to restore tissue homeostasis by reducing energy-intensive activities such as proliferation and increasing production of growth factors such as VEGF. This occurs without any effect on cell viability.

### EXAMPLE 4

Classical Complex I inhibitors often cause cytotoxicity and cell death and despite reports of anti-tumour effects for compounds such as IACS-010579 the known inhibitors have not found use as approved therapeutics, largely due to mechanism-based toxicity (Yap, T *et* al, 2023). As such, identifying a suitable approach for complex I inhibition that provides a benefit without toxicity has proved to be a challenge. An alternative approach which does not elicit adverse effects may have potential benefits for the treatment of a variety of progressive diseases. In this study we evaluated whether the MCIM compounds of the invention showed differences in their cellular behaviour compared with known Complex I inhibitors, IACS-010579 and rotenone.

Human primary lung fibroblasts were plated at a concentration of 5 × 10³ cells/well in 96well plates in 100 µL DMEM complete media (5.5 mM glucose, 2 mM L-glutamine, and 1 mM pyruvate) containing 1% penicillin-streptomycin and 10% heat-inactivated foetal bovine serum. Cells were incubated overnight at 37°C / 5% CO₂ to allow cell attachment. On the day of the treatment, cell culture medium was removed, and the cells were washed three times with Gibco^{™} DMEM, no glucose, no L-glutamine, no pyruvate, no phenol red + 1% penicillin-streptomycin and 10% heat inactivated foetal bovine serum (termed `basal' culture medium). Fresh cell culture media (without pyruvate) was added to the wells. Test compounds were prepared as 10x final concentration solutions in pyruvate-free medium. Following 24-hours compound treatment (in 5% CO_{2,}37°C cell incubator), spent medium was removed from all wells and were thoroughly washed thrice with basal cell culture medium. For washout assay condition, 100 µL of pyruvate-free media was added per well. For No Washout, 90 µL of pyruvate-free media and 10 µL of 10x final assay concentration prepared test agents (or assay media) were added per well. Recovery of cell proliferation was measured after 24-hours incubation at 37°C / 5% CO₂ with either pyruvate-free media (without re-addition of the compound) or re-addition of compounds in pyruvate-free media.

To assess nucleus counts, formaldehyde was added to a final concentration of 4%. Following a further 20-minute incubation at room temperature, the medium was aspirated, the cells were washed twice with 200 µL TBS-T (1x TBS + 0.1% Tween 20) and the nuclei were stained with 50 µL of PBS containing 10% heat inactivated foetal bovine serum, 0.1% Triton-X-100 and 2 µM Hoechst dye. Following a 30-minute incubation at room temperature, protected from light, the cells were washed twice with TBS-T, and cells were counted in 100 µL PBS solution using an ImageXpress Pico system with stitched plate acquisition (4x magnification), DAPI channel (50 ms exposure, -3 digital confocal setting).

When cells grow and divide (termed proliferation), cells progress through the cell cycle, a tightly regulated process that consists of two main activities: DNA replication and mitosis/cell division. As a method of accurately quantifying cellular proliferation rate, the pyrimidine analogue, BrdU can be utilised to measure DNA replication. BrdU can be incorporated into the newly synthesized DNA in place of thymidine. Following incubation of proliferating cells with BrdU for 20 hours, labelling solution was removed thoroughly, and cells were fixed with 70 µL of fix solution for 30 min at room temperature. Fix solution was aspirated, and anti-BrdU-Eu antibodies were added for 2 hours at room temperature in the dark. Wells were washed thrice with wash solution and 70 µL of DELFIA inducer per well was added and incubated for 30 min, in the dark. Eu-fluorescence was measured in a time-resolved manner using the PHERAstar FSX (Ex337/ Em620 nm). Data were plotted and a curve was fitted using a 4-parameter equation with GraphPad Prism software.

The results are shown in Figure 5. The figure shows 4 panels: cell proliferation inhibition measured by BrdU incorporation (A) and nucleus counts (B), and effects of compound washout on cell proliferation by BrdU incorporation (C) and nucleus counts (D) at the highest tested concentration. In panels A and B, ABD900 is shown in filled circles (●), rotenone is shown in grey squares ( 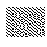 ) and IACS-010759 in open circles (O). In panel C-D, cells without washout are shown in solid black (IACS-010759) and grey (Rotenone) fill, and washout in open black (IACS-010759) and grey (rotenone) bars. For ABD900, washout cells are shown with an open fill pattern and cells not washed out are shown in a checkerboard fill. Data are mean ± s.e.m..

The results show that typical Complex I inhibitors such as IACS-010759 and rotenone reduce cell proliferation (BRdU incorporation and nucleus counts) when cells are cultured in pyruvate-restricted conditions. For known Complex 1 inhibitors such as IACS-010759 and rotenone, there is no recovery from these effects when the compounds are washed out. However, for the MCIM compound, there is a recovery in the proliferative capacity (BrdU incorporation and nucleus counts) of the cells after the compound is washed out. Overall, the data together show that the MCIM compounds of the invention display different cellular effects to the known, archetypal, inhibitors of Complex I.

### EXAMPLE 5 - Enhanced therapy in IBD model

DSS-induced colitis is a widely used model of IBD (Chassaing *et al,* 2015, which is hereby incorporated by reference in its entirety). Eight- to nine-week-old female C57BI/6 mice were used for all procedures. Animals were housed in groups of 10 and were maintained at 21°C ± 2°C on a 12-hour light/dark cycle with food and water ad libitum. Dextran sulphate (DSS) was prepared by dissolving DSS in water to a final concentration of 1.5%. All mice were given ad libitum access to the DSS-containing water for 6 hours prior to dosing with vehicle control, 300 mg/kg sulfadiazine, 3 mg/kg etanercept or 10 mg/kg MCIM test compound by oral gavage, once daily for 8 days. The mice started to develop signs and symptoms of colitis within 1 day.

For assessment of colitis, mouse body weight, stool consistency and presence/absence of blood in stool were monitored. Depending on the severity of the change in each of these observed parameters, the mice were assigned a score based on the criteria in Table 3. The disease scores were summed to generate the Disease Activity Index (DAI) (the maximum DAI for one animal is 9). The data are presented as mean s.e.m. across the group, and statistical analysis was performed using a two-way ANOVA with multiple comparisons (GraphPad Prism v 9.2.0). *p<0.05, ***p <0.005 vs vehicle, ^{§§§}p <0.005 vs sulfasalazine, ^{aaa}p<0.005 vs etanercept.

| **Table 3** | | | | | |
|---|---|---|---|---|---|
| Disease scores associated with observed phenotypes in mice with DSS-induced colitis. | | | | | |
| Parameter | Score | | | | |
| | 0 | 1 | 2 | 3 | 4 |
| Stool consistency | Separate hard lumps | Sausage like with cracks | soft blobs | watery, no solid pieces | N/A |
| Fecal blood | No blood in stool | Blood in stool | N/A | N/A | N/A |
| Body Weight loss | ≤ 1% | 1≤5% | 5≤10% | 10≤15% | > 15% |

Figure 6 shows the mean disease activity index for mice with DSS-induced colitis following treatment with vehicle, 300 mg/kg/d sulfasalazine, 3 mg/kg/d etanercept or 10 mg/kg/d MCIM compound, HMC-C-01-A. These data indicate that the MCI compounds described herein show excellent *in vivo* activity in preventing the progression of established DSS-induced colitis. Furthermore, the data shows that the MCIM compound has greater efficacy than both prior art treatments, sulfasalazine and etanercept.

For histopathological assessment of the biological effects of MCIM compounds in colitis, on Day 9, the colon from the rectum to the ileo-caecal junction was removed and the length recorded. The faeces were then removed and the weight of the colon recorded. The colon was preserved in 10% neutral buffered formalin and processed to paraffin blocks.

Tissue sections were then stained with Haematoxylin and Eosin (H&E) and parameters of inflammation, mucosal erosion, epithelial hyperplasia, epithelial metaplasia, mucus cell metaplasia, and fibroplasia were assessed on a scale of 0-5 as follows:
0: normal
1: minimal, focal
2: moderate, focal
3: moderate, multi-focal or diffuse
4: marked, focal
5: marked, multi-focal or diffuse

The data were analysed by generating an average histopathology score across each treatment group. Test groups were compared to the vehicle and positive control groups using two-way ANOVA with correction for multiple comparisons (Prism 9.2.0). Data shown as mean ± s.e.m.. * p<0.05, ** p<0.01, *** p<0.005 vs vehicle. ^{aaa} p<0.005 vs sulfasalazine. § p<0.05, §§ p<0.01, §§§ p<0.005 vs etanercept. The data are summarised in Figures 6-10.

Figure 6 shows one graph, showing the average disease activity index for vehicle control, 300 mg/kg/day sulfasalazine, 3 mg/kg/day etanercept and 10 mg/kg/day HMC-C-01-A. Figure 7 shows two graphs, each of average mucosal erosion score for (A) vehicle control, 300 mg/kg/day sulfasalazine and 10 mg/kg/day ABD900and (B) vehicle control, 3 mg/kg/day etanercept and 10 mg/kg/day HMC-C-01-A. Figure 8 shows two graphs, each of average glandular loss score for each of: (A) vehicle control, 300 mg/kg/day sulfasalazine and 10 mg/kg/day ABD900 and (B) vehicle control, 3 mg/kg/day etanercept and 10 mg/kg/day HMC-C-01-A. Figure 9 shows 2 graphs, each of average epithelial hyperplasia score for each of: (A) vehicle control, 300 mg/kg/day sulfasalazine and 10 mg/kg/day ABD900 and (B) vehicle control, 3 mg/kg/day etanercept and 10 mg/kg/day HMC-C-01-A. Figure 10 shows 2 graphs, each of average fibroplasia score for each of: (A) vehicle control, 300 mg/kg/day sulfasalazine and 10 mg/kg/day ABD900, (B) vehicle control, 3 mg/kg/day etanercept and 10 mg/kg/day HMC-C-01-A.

Figure 11 shows representative histological cross sections of colon taken from mice with DSS-induced colitis treated with vehicle, 3 mg/kg/day etanercept or 10 mg/kg/day HMC-C-01-A, respectively. In vehicle treated mice, there is clear ulceration (top right panel, arrow) and a general loss of tissue architecture of the colon as demonstrated by visible oedema/inflammation and erosion (top left panel, arrow). Mice treated with etanercept display a general conservation of tissue architecture but still display a moderate degree of inflammation and oedema (middle panels, arrows). In contrast, the colon of mice treated with HMC-C-01-A have conserved tissue architecture and no visible signs of inflammation or oedema. Surprisingly, treatment with HMC-C-01-A stimulates an adaptive repair of the colon which is not seen with Etanercept treatment (bottom panel, arrows). The histological section from mice treated with HMC-C-01-A demonstrates that this reaction is organised and localised within the lamina propria and aligned along the basal layer, with expansion/maintenance of basement membrane and maintenance of crypt architecture.

Together, these data indicate that HMC-C-01-A and ABD900 compounds have excellent activity in preventing the progression of established colitis and can stimulate repair of damaged tissues. Administration of MCIM compounds inhibited the key histological outcome of mucosal erosion/ ulceration, as shown in Figure 7. Importantly, administration also increased hyperplasia of epithelium, suggesting induction of a repair response. This is supported by the unique finding of fibroplasia in the MCIM compound treated groups (Figure 10A and 10B).

Overall, Figures 6 to 11 show the effects of the compounds in a model of gastrointestinal disease. The results show that the compounds reduce disease signs and symptoms and protect the underlying tissue damage to a greater extent than approved drugs, sulfasalazine or anti-TNF biologic, etanercept, and that the compounds promote repair responses of epithelial hyperplasia, mucus cell metaplasia and fibroplasia to a greater extent than sulfasalazine and etanercept.

### EXAMPLE 6 - Mouse bleomycin-induced lung fibrosis

Eight- to ten-week-old female C57BI/6 mice were used for all procedures. Animals were housed in groups of 4 and were maintained at 22°C ± 3°C on a 12-hour light/dark cycle with food and water ad libitum. Bleomycin was prepared by dissolving in saline at 1.5 mg/mL. All mice were administered intranasally with 3 mg/kg bleomycin. Seven days after bleomycin administration, vehicle control, nintedanib or MCIM compound administration was initiated. Nintedanib was administered twice daily (b.d.) by oral gavage at a total daily dose of 30 mg/kg/day for 14 days. MCIM compounds were administered once daily (q.d.) by oral gavage at a total daily dose of either 10 or 20 mg/kg/day for 14 days.

On the 14^{th} day, lungs were collected after perfusion. The left lung lobe was assessed for fibrosis by measuring total lung hydroxyproline levels. The right lung lobes were fixed and sectioned for histopathology.

For histopathological assessment of the biological effects of MCIM compounds on fibrosis, lung sections were stained with Masson's Trichome (MT) to assess fibrosis using the Ashcroft score, and Haematoxylin and Eosin (H&E) to assess parameters of vascular inflammation, bronchiolar inflammation, neutrophil inflammation, lymphoid infiltration, monocyte/macrophage infiltration, pneumonitis, apoptosis/necrosis, alveolar type 2 (AT2) cell hypertrophy and AT2 cell hyperplasia, as described in Hubner RH *et al* (2008) which is hereby incorporated by reference in its entirety. The latter parameters were assessed on a scale of 0-5 as follows:
0: normal
1: minimal, focal
2: moderate, focal
3: moderate, multi-focal or diffuse
4: marked, focal
5: marked, multi-focal or diffuse

To assess fibrosis in the lung, 10 successive fields of view of a lung section was visually graded from 0 (normal lung) to 8 (total fibrous obliteration of the area) as described in Hubner *et al* (2008). The Ashcroft score is assessed on a scale of 0-8 as follows:
0: Normal lung
1: Minimal fibrous thickening of alveolar or bronchiolar walls
2
3 Moderate thickening of walls without obvious damage to lung architecture
4
5: Increased fibrosis with definite damage to lung structure and formation of fibrous bands or small fibrous masses
6
7: Severe distortion of structure and large fibrous areas; "honeycomb" lung" is placed in this category
8: Total fibrous obliteration of the field

The data were analysed by generating an average histopathology score across each treatment group. Test groups were compared to the vehicle and positive control groups using two-way ANOVA with multiple comparisons (Prism 9.2.0). Data shown as mean ± s.e.m.. * p<0.05, ** p<0.01, *** p<0.005 vs vehicle. The data are summarised in Table 4 and Table 5.

| **Table 4** | | | | | | | |
|---|---|---|---|---|---|---|---|
| Inhibition of Fibrosis and support of repair | | | | | | | |
| Compound | Dose (mg/kg/ day) | Lung hydroxyproline, ug/g | | Ashcroft Score | | Vascular Inflammation | |
| Study | | Vehicle value | Treatment value | Vehicle value | Treatment value | Vehicle value | Treatment value |
| Nintedanib | 30 b.d. | 109 ± 15 | 82 ± 10*** | 4.4 ± 0.7 | 3.3 ± 0.8 | 2.3 ± 0.3 | 1.7 ± 0.4 |
| Nintedanib | 30 b.d. | 56 ± 16 | 26 ± 8*** | 5.1 ± 0.6 | 1.3 ± 0.3** | 3.0 ± 0.2 | 1.1 ± 0.2*** |
| ABD900 | 20 q.d. | 109 ± 15 | 86 ± 16** | 4.4 ± 0.7 | 2.8 ±0.8 | 2.3 ± 0.3 | 1.2 ±0.4 |
| HMC-C-01-A | 10 q.d. | 56 ± 16 | 36 ± 6* | 5.1 ± 0.6 | 1.6± 0.4* | 3.0 ± 0.2 | 1.4 ± 0.3*** |

| **Table 5** | | | | | |
|---|---|---|---|---|---|
| Inhibition of Fibrosis and support of repair | | | | | |
| Compound | Dose (mg / kg / day) | AT2 cell size | | AT2 cell number | |
| Study | | Vehicle value | Treatment value | Vehicle value | Treatment value |
| Nintedanib | 30 b.d. | | | 1.9 ± 0.3 | 1.6 ± 0.2 |
| Nintedanib | 30 b.d. | 1.2 ± 0.1 | 1.6 ± 0.2 | 1.1 ±0.1 | 1.1 ± 0.1 |
| ABD900 | 20 q.d. | Not measured | Not measured | 1.9 ± 0.3 | 3.3 ± 0.2***^{,§§§} |
| HMC-C-01-A | 10 q.d. | Not measured | 3.6 ± 0.2***^{,§§§} | 1.1 ± 0.1 | 3.4 ± 0.2***^{,§§§} |

Figure 12 shows 4 graphs, showing the anti-fibrotic effects of the MCIM compounds and control drug nintedanib in mice with bleomycin-induced lung fibrosis. Quantification of lung hydroxyproline levels is an established and widely used method for biochemical quantification of collagen content in the lung, with an increase in hydroxyproline being indicative of an increase in collagen deposition and fibrosis. The effect on lung hydroxyproline levels following treatment with ABD900 (A), HMC-C-01-A (B), or nintedanib (A) was measured. As demonstrated in Figures 12A and B, MCIM compounds reduce hydroxyproline levels in mice with established lung fibrosis indicating a decrease in total collagen and fibrosis in these mice.

Effects of MCIM compounds on lung fibrosis was also assessed using an established scoring system known as the Ashcroft score first described in Ashcroft et al., 1988, which is incorporated herein in its entirety and refined by Hubner *et al* (2008). The Ashcroft score grades lung fibrosis on a scale of 0 to 8, described above. Following treatment with nintedanib or ABD900 (Figure 12C) or HMC-C-01-A (Figure 12D), histological analysis was performed and Ashcroft scores generated. ABD900 can lead to a modest reduction in the Ashcroft score, comparable to nintedanib. However, HMC-C-01-A can significantly reduce the Ashcroft score of mice with bleomycin-induced lung fibrosis compared to mice treated with vehicle.

At the early phases (Days 4 to 7) the bleomycin model shows a pneumonitis (lung inflammation) with the beginnings of fibroplasia (the development of fibrous tissue). During this phase, alveolar epithelial Type 2 cells (AT2), the 'stem cells' of the lung, activate to maintain alveolar sac patency by augmenting a surfactant response, and initiate differentiation to AT1 cells to maintain alveolar wall structure and spatial organisation with respect to the pulmonary microvasculature. AT2 differentiation is a highly organised process which occurs in the so-called 'transitional zone' at the ends of the terminal bronchioles, and in the alveolar pores. In these areas, a variety of cell forms (a stem cell reservoir) can be seen which reflects the differentiation responses of AT2, AT1 and transitional `club' cell forms. The AT2 can de-differentiate to club cells, and club cells can differentiate into AT2 and directly into AT1 cells. Hence, repair, in the context of AT2 responses, is a complex equilibrium between these three transitional forms.

In this study we assessed AT2 cell hypertrophy and hyperplasia. The hyperplasia was constrained and localised to areas of disorganised Type I alveolar epithelial cells (AT1) and in areas adjacent to transitional zones. The hypertrophy was associated with maintenance of a 'vacuolated' phenotype in AT2 and distinct from the flattened 'basaloid' forms associated with active pneumonitis/ fibroplasia. Figure 13 shows 2 graphs illustrating the effects of HMC-C-01-A on AT2 cell number (A) and AT2 cell size (B) in mice with bleomycin-induced lung fibrosis. Compared to mice receiving nintedanib or vehicle control, mice treated with HMC-C-01-A have a significant increase in AT2 cell hyperplasia (Figure 13A) and hypertrophy (Figure 13B) indicating that HMC-C-01-A promotes an adaptive response in the lung, consistent with repair of the tissue.

Figures 14 and 15 show H&E stained sections of lung in mice treated with either vehicle control, 30 mg/kg/day nintedanib (Ofev) or 10 mg/kg/day HMC-C-01-A.

Together, the above data indicate that the compounds show excellent activity in inhibiting the key fibrosis outcomes of lung hydroxyproline levels and Ashcroft Score. Importantly, administration also elicited an effect consistent with protection of the alveolar epithelial response, indicative of an adaptive repair phenotype, with preservation of AT2 cell size and number. Nintedanib was effective in ameliorating fibrosis but showed no effect on AT2 cells and thus no protection of the alveolar epithelial response.

In summary, the data in Figures 12-15 shows that overall HMC-C-01-A outperforms nintedanib in preventing inflammation in the lungs of mice with bleomycin-induced lung fibrosis and preventing fibrotic lesions developing. Surprisingly, this data also demonstrates that the compounds promote repair responses of alveolar epithelial cell hypertrophy and hyperplasia which are not changed compared with vehicle controls by nintedanib.

### EXAMPLE 7- Surfactant staining in bleomycin model

To maintain alveolar sac integrity, AT2 produce a range of surfactants, which in turn play a role in regulating hydration of the alveolar wall surface. Different surfactants serve different roles this regard, and in controlling surface tension of the alveolar wall and thus facilitating patency of the airspaces. For example, surfactant protein C (SpC) is a continually synthesized 'surveillance' surfactant, important in regulating the absorption of lipid films in the alveolar air-liquid interface, which covers a significant area of the AT1 surface. SpC expression and area are reduced in a range of lung pathologies, such as pulmonary fibrosis or ARDS. By contrast, surfactant protein A (SpA) is a 'fast response' surfactant induced in stress conditions, such as infection and, as such, is a useful register of AT2 'health'. Reduced SpA levels are correlate with degeneration of the AT2 population. Like SpA, surfactant protein D (SpD) is involved in the acute response of the alveolar sac to injury and possessed anti-microbial and anti-inflammatory effects. SpD is both secreted and re-absorbed by the AT2, which allows the AT2 to `sample' the local microenvironment and thus allow early detection of injury. This surfactant has a wider expression that either SpA or SpC and is expressed on a range of progenitor lineages in the transitional zone stem cell niche, notably on the pluripotent club cell. Therefore, by monitoring surfactant protein expression in the lung we can monitor the repair responses of the lung in relation to the stem cell niche.

In this study, bleomycin was used to induce fibrosis and mice were treated with ABD900 as described above once daily for 4 or 7 days. Lungs were collected, inflated with 0.5mL 10% Neutral Buffered Formalin and then fixed in 10% Neutral Buffered Formalin for 48 hours. Following sectioning, slides were blocked to prevent non-specific binding, followed by immunostaining for the proliferation marker, Ki67, surfactant protein A (SP-A), pro-surfactant protein C (pSP-C) and surfactant protein D (SP-D), and counterstaining with Harris Haematoxylin and Eosin. 30 randomly selected fields were examined at x20 magnification using a 400 x 400 um grid with 50 µm Mertz lines for counting according to the following grading system.

| **Grade** | **Definition** |
|---|---|
| 0 | No abnormalities |
| 1 | Minimal change; focal |
| 2 | Moderate change, focal |
| 3 | Moderate change, multi-focal or diffuse across >3 fields |
| 4 | Marked change, focal |
| 5 | Marked change, multi-focal or diffuse across > 3 fields |

For AT2 cells, the data were analysed by generating an average histopathology score across each treatment group. For the AT2 staining, the % of positive cells in 200 AT2 cells was counted. For the epithelial lining, the % of positive alveoli (showing > 30% surface staining) in 100 alveoli was counted. The count grid was from the largest terminal bronchiole as the mid-point, then overlaying transects moving clockwise out. Any grid with greater than 25% blood vessel or air space (or edge of tissue) was rejected.

The data are presented as mean s.e.m. across the group. Statistical analysis was performed using a two-way ANOVA with multiple comparisons (GraphPad Prism v 9.2.0). *p<0.05, ***p <0.005 vs vehicle.

The AT2 cell results are shown in Figure 16, which contains two panels: (A) AT2 size and (B) AT2 number (right). Vehicle is shown in black bars and animals dosed with ABD900 at 10 mg/kg/day in grey bars or 20 mg/kg/day in white bars.

The surfactant staining results are shown in Figure 17. The figure shows 10 panels: (A) SpA, (B) pSpC and (C) SpD scoring in AT2 cells (left) and alveolar lining (right), (D) SpD scoring in Club cells, and (E) Ki67 in AT2 cells. Vehicle is shown in black bars and animals dosed with ABD900 at 10 mg/kg/day in grey bars or 20 mg/kg/day in white bars. Data are mean ± s.e.m.. (F) Representative images (160x magnification) show the increase in SpD staining (black) in the lungs following treatment with ABD900 (20 mg/kg/day) for 7 days (lower panel) compared with vehicle (upper panel).

The results show that the compound increases AT2 cell size (hypertrophy) at Day 4 and is maintained through to Day 7. Trends to increased AT2 number (hyperplasia) did not reach statistical significance on Day 7. The compound significantly increased SpA expression in AT2 at Day 4, with the increase seen in both dose groups on Day 7. There was no effect of treatment on SpA expression on the alveolar lining cells at either time point. There was no effect of treatment on pro-SpC expression in AT2 cells or the alveolar lining at either time point. AT2 SpD expression was increased at 10mg/kg ABD900 at Day 4 and at both doses at Day 7. In addition, SpD expression was significantly increased on the alveolar lining at Day 7 in the 20mg/kg ABD900 group, and the pattern of expression of SpD was distinct from the other surfactant proteins, with high expression seen on club cells in the transitional zones, and expression being similar across all study groups at both time points. There were no statistically significant changes in Ki67 expression between vehicle and test groups at either the Day 4 or Day 7 timepoints, indicating that the primary efficacy phenotype was a result of differentiation rather than proliferation.

Overall, the data together show that the compound inhibits the development of AT2 cells with a basoloid form, which is indicative of degeneration or senescence. The protection of the AT2 is not due to a proliferative response, as shown by the lack of effect on the proliferation marker, Ki67. Rather, and together with the enhanced expression of SpA and SpD in the treatment groups, the data supports that the compound is causing a protection and enhancement of AT2 and preservation of the transitional niche (AT2 - AT1 - club cell). In addition, the expression of SpD in club cells suggests a protection of the functional integrity of the transitional zone progenitor niche, which is a critical component of lung repair. Importantly, the lack of effect on proSpC indicates that the compound is maintaining homeostasis of the epithelial lining barrier.

### EXAMPLE 8 - EAE model of Multiple sclerosis (MS)

Seven- to eight-week-old female C57BI/6 mice were used for all procedures. Animals were housed in groups of 10, and were maintained at 22°C ± 3°C on a 12-hour light/dark cycle with food and water ad libitum. MOG₃₅₋₅₅ was prepared by dissolving in saline at 1 mg/mL and mixed in an equal ratio with complete Freund's adjuvant (CFA). Pertussis toxin was prepared by dissolving pertussis toxin to 1 ug/ml in saline. All mice were administered subcutaneously with 0.1 g MOG35-55/CFA emulsion, and again two hours later with 0.2 ug pertussis toxin. A further 0.2 ug pertussis toxin was administered the following day. Clinical signs emerged after 7 days. Dosing was initiated after 13 days. All compounds were administered once daily by oral gavage for 27 days.

Clinical signs of disease were monitored daily according to the following scoring system.
0: No obvious signs of motor dysfunction in mice compared to non-immunized control
0.5: Distal tail limpness
1: Limp or floppy tail
2: Limp tail and weakness in hind legs
3: Limp tail and complete paralysis of hind legs (most common)
   OR Limp tail with paralysis of one front and one hind leg
   OR All of the following:
      severe head tilting
      walking only along the edges of the cage
      pushing against the cage wall
      spinning when picked up by the tail.
4: Limp tail, complete hind leg and partial front leg paralysis
   Mouse is minimally moving around cage but appears alert and feeding. Euthanasia is recommended after mouse scores 4 for 2 consecutive days. A score of 5 is entered when mouse is euthanized.
5: Complete hind and complete front leg paralysis, no movement around cage OR mouse is spontaneously rolling in cage OR mouse found dead due to paralysis

On the 27^{th} day, the brain was removed to 5 ml of 10% neutral buffered formalin and the spinal column was preserved in 50 ml 10% neutral buffered formalin and samples were sectioned for histopathology.

For histopathological assessment of the biological effects of MCIM compounds on disease, spinal cord sections were stained with Masson's Trichome (MT) to assess fibrosis using the Ashcroft score, and Haematoxylin and Eosin (H&E) to assess parameters of inflammation, demyelination and pyknosis. Sections were stained using A2B5 to assess oligodendrocyte progenitor cells. The following scoring system was used to assess these parameters:
Grade 0: Normal, no pathology
Grade 1: minimal, Single focal lesion in one section
Grade 2: moderate, Single focal lesion in 2+ sections
Grade 3: moderate, multi-focal lesions in one section
Grade 4: marked, multi-focal lesions in 2+ sections
Grade 5: marked, diffuse pathology

The data were analysed by generating an average histopathology score across each treatment group. Test groups were compared to the vehicle and positive control groups using two-way ANOVA with multiple comparisons (Prism 9.2.0). *p<0.05, **p <0.01, ***p <0.005 vs vehicle. The data are summarised in Figure 18.

Figure 18A also demonstrates that MCIM compounds of the invention reduce the clinical score in the EAE multiple sclerosis disease model. In addition, Figure 18B shows that a MCIM compound of the invention reduces demyelination in the EAE multiple sclerosis disease model. Figure 18C also demonstrates that a MCIM compound of the invention increases oligodendrocyte precursor cells (OPCs) to a greater extent than fingolimod in the EAE multiple sclerosis disease model. These data demonstrate that MCIM compounds of the invention reduce inflammation and tissue damage in EAE model mice and also promote repair of damaged tissue. Microglia are the first line of defence following brain injury and respond rapidly to any type of brain injury. Microglia are typically highly ramified cells, with the ramifications capable of detecting stress signals in the local environment. Upon detection of such signals, the microglia are capable of responding to the stress (e.g. injury) in order to protect and/or promote repair of the damaged tissue. Microglia with a ramified phenotype are therefore neuroprotective. In chronic disease, microglia become less ramified and thus the normal neuroprotective function is impaired.

To determine if the MCIM compounds of the invention could promote neuroprotection, brain sections were taken from mice with EAE treated with vehicle control or an MCIM compound of the invention and the state of the microglia investigated.

An MCIM compound of the invention reduces inflammation and increases ramified (`resting') microglia in sections from mice with EAE. Figure 19, top panel, shows rounded microglia with evident process extrusions following administration of vehicle alone (negative control). In contrast, mice treated with HMC-C-01-A (bottom panel) shows ramified microglia with reduced process extrusions. These data suggest that by eliciting a more ramified microglia phenotype, MCIM compounds cause an adaptation to the neuronal environment and increase the potential for the normal neuroprotective response of microglia to function.

The results described above show that the compounds reduce inflammation and demyelination as well as fingolimod, and promote OPC cells, and a change in microglial phenotype as an indicator of a selective, adaptive repair responses.

### EXAMPLE 9 - Mouse collagen-induced arthritis

Seven- to eight-week-old male DBA/1j mice were used for all procedures. Animals were housed in groups of 10 and were maintained at 21°C ± 2°C on a 12-hour light/dark cycle with food and water ad libitum. Complete Freund's adjuvant (CFA) was prepared by emulsifying bovine type II collagen at 4 mg/mL with a 4 mg/mL suspension of Mycobacterium tuberculosis H37Ra in Incomplete Freund's adjuvant (IFA) (0.85 mL paraffin oil and 0.15 mL mannide monooleate) in a 1:1 (v/v) ratio. All mice were immunised subcutaneously with 200 µg of bovine type II collagen in CFA. 21 days later, all mice were immunised subcutaneously with 100 µg of bovine type II collagen in IFA. The mice started to develop signs and symptoms of arthritis following the 'booster' immunisation.

For macroscopic assessment of arthritis, the following signs were monitored in each paw of each mouse three times per week and summed to generate the Arthritic Index (Al) (the maximum AI for one animal is 16):
0 = no visible effects of arthritis.
1 = oedema and/or erythema of 1 digit.
2 = oedema and/or erythema of 2 digits.
3 = oedema and/or erythema of more than 2 digits.
4 = severe arthritis of entire paw and digits.

Figure 20 shows 7 graphs, each of average arthritic index as a function of time (dosing day) for test compound dosed at 10 mg/kg/day by oral gavage (open circles (O)) and control (solid circles (●)), for each of: (A) HMC-C-02-A, (B) HMC-C-01-A, (C) HMC-N-02-A, (D) HMC-N-01-A, (E) NASMP-01-A, (F) CHMSA-01-A, (G) CHMSA-03-A. These data indicate that the MCIM compounds described herein show excellent oral *in vivo* activity in preventing the progression of established, severe arthritis.

Animals were sorted into treatment groups with a mean arthritic index of 2.5 and then dosed once daily for 14 days with compound by oral gavage. On Day 14, animals were sacrificed, and limbs were fixed in 10% neutral buffered formalin.

The fixed limbs were processed into paraffin blocks and sectioned, and then stained using Toluidine Blue.

For histopathological assessment of the biological effects of MCIM compounds in arthritis, bone resorption was assessed by a direct count per bone of areas showing evident Howship's lacunae or active osteolytic foci. To assess the frequency of new bone formation, a total count of cancellous bone osteoid zones was performed.

The data were analysed by generating an aggregate score for each parameter in each treatment group. Test groups were compared to vehicle group by ANOVA using the Kruskal Wallis test statistic with exact P value comparisons. The data are summarised in the Table 6. Data shown as sum from all limbs per animal per group. * p<0.05, ** p<0.01, *** p<0.005 vs vehicle. §§§ p<0.005 vs etanercept.

| **Table 6** | | | | | |
|---|---|---|---|---|---|
| Histological assessment of bone resorption and formation. | | | | | |
| | | Bone resorption (sum) | | Bone formation (sum) | |
| Compound | Dose (mg / kg / day) | Vehicle value | Treatment value | Vehicle value | Treatment value |
| Etanercept | 3 | 150 | 78** | 21 | 78 |
| Tofacitinib | 10 | 117 | 48** | 0 | 0 |
| HMC-C-01-A | 3 | 150 | 30*** | 21 | 387***,^{§§§} |
| ABD900 | 10 | 124 | 24*** | 19 | 87*** |
| NASMP-01 | 10 | 124 | 20*** | 19 | 77*** |
| CHMSA-03-A | 10 | 124 | 34*** | 19 | 61*** |
| HMC-C-01-B | 10 | 150 | 60*** | 21 | 139 |
| HMC-N-01-B | 10 | 150 | 30*** | 21 | 188* |
| CHMSA-01-A | 10 | 124 | 39*** | 19 | 64*** |

The data for several of the compounds are also illustrated in Figure 21 and Figure 22.

Figure 21 and 22, shows a decrease in bone resorption with approved drugs, etanercept (Figure 21A) and tofacitinib (Figure 21C) and various MCIM compounds (Figure 21A and B). Figure 22A shows a modest increase in bone formation in mice treated with etanercept compared to vehicle control. In contrast, mice treated with HMC-C-01-A, HMC-C-01-B, and HMC-N-01-B have significantly increased bone formation compared to vehicle control and etanercept (Figure 22A). Figure 23 shows the appearance of the osteoid/new bone formed in response to treatment with vehicle (top panel), an approved disease modifying anti-rheumatic drug (etanercept; bottom panel) and compound HMC-C-01-A (middle panel). Figure 23, middle panel, demonstrates the new bone formed in response to treatment with HMC-C-01-A has a regular appearance with conservation of the tide-mark (arrows). This indicates that the bone formed is responsive to pressure and has structural integrity in contrast to the reactive and sporadic deposits generated with etanercept (bottom panel) or tofacitinib (data not shown).

Figure 24 shows that improvements in osteoid are achieved in mice treated with ABD900 without control of inflammation. This indicates a direct remodelling effect that is not dependant on control of inflammation.

Figures 20-24 show the effects of the compounds in a model of joint inflammation and bone loss. The results show that the MCIM compounds reduce total bone resorption, and local focal areas of bone resorption as well as, or better than, anti-biologic drugs such as etanercept or Janus kinase (JAK) inhibitors such as tofacitinib. In addition, the MCIM compounds trigger an adaptive repair response resulting in an increase in new bone deposition (osteoid), both in terms of osteoid counts and the areas of osteoid formation.

Together, the above data indicate that the compounds show excellent oral *in vivo* activity in preventing the progression of bone loss in established, severe arthritis, but importantly that they increase bone formation, indicating repair, in established arthritis.

### EXAMPLE 10 - Cellular thermal shift assay (CETSA) and multiplexes quantitative mass spectrometry

Following the observation that MCIM compounds can affect mitochondrial morphology and modulate cellular metabolism it was next investigated whether the reparative properties of the MCIM compounds could be attributed to binding to/modulation of mitochondrial proteins and/or complexes. To this end, MCIM compounds were assessed using a cellular thermal shift assay (CETSA) coupled with quantitative mass spectroscopy (MS) to determine what pathways are modulated by the MCIM compounds.

Thp-1 cells were incubated in the presence of 2 µM MCIM compound (ABD900) or DMSO (vehicle control) for 4 hours. Following the incubation period, samples were heated to one of the following temperatures: 40.0, 42.9, 46.0, 49.6, 53.2, 56.8, 60.8, 64.0, 67.1, 70.0°C. Each test condition was performed in duplicate.

Following heating, cells were lysed, and the samples digested with trypsin. The digested soluble peptide fractions corresponding to individual temperatures were then labelled with a different isobaric tag, using the TMT10plex system as described in Bantscheff, M., et al (2007), Bantscheff, M., et al (2012) and Franken, H., et al (2015), each of which are incorporated by reference in their entirety. Labelling of fractions from individual temperatures with an individual TMT1 OPlex tag allows the samples to be pooled and analysed by mass spectroscopy in a single run.

Following TMT10Plex labelling and pooling, the samples were fractionated using hydrophilic strong anion exchange (hSAX) (24 fractions per sample) and Liquid Chromatography with tandem mass spectrometry (LC-MS/MS) performed. The LC-MS/MS data was analysed to identify proteins whose thermal stability was shifted in the presence of the MCIM compound using the TPP R Package (Franken, H., et al (2015)) with a procedure described in Savitski., MM et al (2014). Generally, upon compound binding, proteins become more stable and thus more resistant to thermal denaturation. Therefore, a shift in thermal stability of a protein in the described CETSA assay can indicate direct binding of a compound to the protein with shifted thermal stability. Alternatively, a shift on thermal stability may indicate the protein is involved in a downstream event from the bound protein, for example, a post-translation modification as a result of an altered metabolic or signalling pathway.

Briefly, criteria for target candidate selection were as follows:
- Min p-value <0.4 (Benjamini-Hochberg corrected)
- DTm of Run 1 (R1) and Run 2 (R2) have same sign
- DTm (drug vs DMSO) > DTm (DMSO R1 vs DMSO R2)
- Minimum slope less than -0.06

Using the above criteria, 105 proteins were identified as showing a thermal shift when incubated with the MCIM compound indicating that they were either stabilised or destabilised. Of these 105 proteins, 73 were classified as high confidence and 32 as medium confidence hits.

Functional protein interactions and/or associations of the 105 identified candidate proteins were retrieved using the freely available STRING software (https://string-db.org). STRING analysis identified candidates involved in oxidative phosphorylation (e.g. NDUFA6 and SDHB), mitochondrial function and the ER to Golgi apparatus interface.

In addition, thermal shifts were observed in proteins involved in adaptive stress responses (e.g. YME1L1, OXSR1, MKNK1) and other proteins which play a role in NF-κB signalling (e.g. OXSR1, RASA1 and BIRC2).

It was therefore concluded, in line with the data in Example 3, that incubation with MCIM compounds alters the metabolic activity of cells specifically through modulation of oxidative phosphorylation, as indicated by the thermal shift of NDUFA6 (a Respiratory Complex 1 component). It was hypothesised that such modulation of Complex 1 and the oxidative phosphorylation pathway would alter NFκB and adaptive response pathways in cells, as indicated by the thermal shift observed for proteins such as YMEL1L1, OXSR1, MKNK1, RASA1 and BIRC2.

### EXAMPLE 11 - Photoaffinity labelling (PAL) and quantitative stable isotope labelling by amino acids in cell culture (SILAC)

To investigate the binding partner(s) of the MCIM compounds, and the cellular activities modulated by them, Photoaffinity labelling (PAL) and quantitative stable isotope labelling by amino acids in cell culture (SILAC) was performed. A clickable linker probe MCIM compound was generated to do this.

This identified NDUFS2, a subunit of Mitochondrial Complex I, as a binder of the clickable linker probe MCIM compound. Together, the CETSA and PAL/SILAC-MS results strongly indicate that MCIM compounds can modulate oxidative phosphorylation and stress response pathways by binding and modulating the activity of the Mitochondrial Complex I.

### EXAMPLE 12 - Computational modelling to identify binding sites in NDUFS2

Given that both CETSA and PAL/SILAC-MS methods identified Complex I subunits as targets of MCIM compounds, an *in silico* computer modelling approach was used to determine the binding site of MCIM compounds with the Complex I subunits.

Initially, homology models were built from publicly available structures of mitochondrial Complex I from five organisms, including human (Table 7). The putative targets were fully resolve in 5 structures. Mitochondrial Complex 1 used for the following modelling approaches is show in Figure 25.

| **Table 7** | | | | | | | |
|---|---|---|---|---|---|---|---|
| Publicly available Complex I structures. FMN = Flavin mononucleotide. | | | | | | | |
| **Protein** | **Size** | **Type** | **Method** | **Liganded?** | | **Ref** | **Protein database ID** |
| | | | | Y/N | Site | | |
| Y. Lipolytica | 466 KD | Fungal | X-Ray | N | | Zickerman et al Science, 2015, 247, 44-49 | 4WZ7 |
| T. Thermophilus | | Bacterial | X-Ray | FMN | NADH-FMN | Efremov et al Nature, 2010, 465, 441-445 | 3M9S |
| T. Thermophilus | 536 KD | Bacterial | X-Ray | FMN, Y | NADH-FMN, Quinone | Sazanov et a Nature, 2013, 2013, 494, 443-450 | 4HEA |
| T. Thermophilus | 570 KD | Bacterial | X-Ray | NADH, FMN | NAD-FMN | Sazanov et al JBC, 2009, 284, 29773 | 3IAM |
| Bos Taurus | 517 KD | Mammalian | Cryo-EM | N | | Hirst et al Nature, 2014, 515, 80-84 | 4UQ8 |
| Bos Taurus | 891 KD | Mammalian | Cryo-EM | NADH, FMN | NADH-FMN | Hirst et al Nature, 2016, 536, 354 | 5LDW, 5LDX, 5LC5 |
| Ovis Aries | 978 KD | Mammalian | Cryo-EM | N | | Sazanov et al Nature, 2016, 538, 406 | 5LNK |
| Ovis Aries | 1.28 MD | Mammalian | Cryo-EM | N | | Sazanov et al Nature, 2016, 537, 644 | 5J4Z, 5J7Y, 5J8K |
| Homo Sapiens | | Mammalian | Cryo-EM | NADP, FMN | NADH-FMN | Guo et al Cell, 2017, 170, 1247-1257 | 5XTD, 5XTH, 5XTI |

The homology modelling revealed a lid pocket in the NDUFS2 subunit of Complex I which is in contact with the Q-tunnel. In four of the models the lid pocket was seen to be in an "open" conformation while in the remaining model it was in a "closed" conformation.

A homology model of the "open" confirmation was constructed and then SiteFinder was used to map NDUFS2 in the open and closed conformations. In particular, SiteFinder (Halgren T. A., 2009) was used to build a model of the "drugability" of NDUFS2, as measured by the volume of buried non-polar available surface area (ASA; Figure 26). This model identified two binding sites on NDUFS2. The first binding site ("Pocket A") is located on the lid pocket in the open conformation and is represented on Figure 26 by a cluster of spheres. Pocket A has a percentage buried non-polar available surface area of 72%. The second binding site ("Pocket B") is located at the "back" of the mitochondrial Complex I, relative to the position of the mitochondrial Complex I subunits NDUFS7 and ND1 and is represented by a second cluster of spheres. Pocket B has a percentage buried non-polar available surface area of 71%.

Further modelling of Q10, the natural ligand of the Q-tunnel, within the Q-site of Complex 1 using SiteFinder indicated that the space for binding in this pocket is limited (Figure 27). The spheres in Figure 27 illustrate the space and channels around NDUFS2 when NDUFS2 is associated with Complex 1. This modelling approach also revealed a further ligand binding site at the junction between NDUFS2 and NDUFS7 (Figure 28). In particular, the MCIM compound is predicted to interact with His38 and Tyr141 of NDUFS2.

Using these protein models it was determined that the optimal binding site of each of ABD900, HMC-C-01-A and HMC-N-01-A was the NDUFS2 lid pocket (as illustrated in Figure 26, "Pocket A"), specifically the lid pocket in the open conformation (Glide score ~6), which is close to the Q-site. Even more particularly, the inventors were able to predict which amino acid residues in NDUFS2 contributed to the binding of the compounds:
- H-bonding with backbone carbonyl of Gly85, backbone amine of Leu95 and carboxylic acid of Asp193
- H-bonding to Tyr141 and His38
- π- π stacking with Phe458 and His88

In conclusion, analysis of the Q-site of NDUFS2 indicates that this site needs to be in the open confirmation to accommodate Q10 or small drug-like compounds.

### Example 13 - Virtual Screening to assess structure-activity relationship against the NDUFS2 pocket

As discussed in Example 12, is has been determined that the optimal binding site for MCIM compounds is in the lid pocket of NDUFS2 which is in close proximity to the Q-tunnel. From the data in Example 12, a ligand-protein pharmacophore model was built which identified 9 pharmacophoric features (Figure 29 and Tables 8A to 8C). Table 8-A describes the relationship between the type of pharmacophoric feature and the permissible variation in 3D space for the location of the given pharmacophoric feature. Table 8-B shows a distance matrix describing the 3D relationship between the centre of each pharmacophoric feature. Table 8-C describes the angle between each triplet combination of pharmacophoric features, wherein column "Y" describes the vertex of each angle. Using the parameters described in Tables 8-A to 8-C a visual representation of the pharmacophore model has been produced using the unified annotation scheme in *Molecular Operating Environment* (MOE) software tool (Figure 29). Using *Molecular Operating Environment (MOE),* the parameters can also be used to determine if a molecule conforms to the pharmacophore model - i.e. if 4 or more of the annotation points of the pharmacophore model are occupied by a corresponding annotation point located on a test molecule.

| **Table 8-A** Pharmacophore feature annotations, and radii | | |
|---|---|---|
| *Feature Number* | *Feature Annotation* | *Radius (Å)* |
| F1 | Acc | 1.2 |
| F2 | Acc | 1.2 |
| F3 | Acc | 1.2 |
| F4 | Acc2 | 1.4 |
| F5 | Aro | 1.4 |
| F6 | Hyd | 1.4 |
| F7 | Hyd | 1.4 |
| F8 | Hyd | 1.4 |
| F9 | Don2 | 1.4 |

| **Table 8-B** Pharmacophore feature distances (Å) | | | | | | | | | | |
|---|---|---|---|---|---|---|---|---|---|---|
| *Dist. (Å)* | *Feature Number* | *F1* | *F2* | *F3* | *F4* | *F5* | *F6* | *F7* | *F8* | *F9* |
| *Feature Number* | *Feature Ann* | Acc | Acc | Acc | Acc2 | Aro | Hyd | Hyd | Hyd | Don2 |
| *F1* | Acc | 0.00 | 2.64 | 8.50 | 8.16 | 8.11 | 4.07 | 4.77 | 3.13 | 4.82 |
| *F2* | Acc | 2.64 | 0.00 | 7.21 | 9.80 | 8.48 | 4.60 | 3.85 | 3.85 | 3.46 |
| *F3* | Acc | 8.50 | 7.21 | 0.00 | 16.55 | 14.19 | 10.57 | 4.37 | 10.95 | 9.58 |
| *F4* | Acc2 | 8.16 | 9.80 | 16.55 | 0.00 | 6.76 | 6.87 | 12.41 | 6.05 | 8.88 |
| *F5* | Aro | 8.11 | 8.48 | 14.19 | 6.76 | 0.00 | 4.12 | 9.96 | 6.41 | 5.82 |
| *F6* | Hyd | 4.07 | 4.60 | 10.57 | 6.87 | 4.12 | 0.00 | 6.26 | 3.31 | 3.22 |
| *F7* | Hyd | 4.77 | 3.85 | 4.37 | 12.41 | 9.96 | 6.26 | 0.00 | 7.12 | 5.90 |
| *F8* | Hyd | 3.13 | 3.85 | 10.95 | 6.05 | 6.41 | 3.31 | 7.12 | 0.00 | 3.77 |
| *F9* | Don2 | 4.82 | 3.46 | 9.58 | 8.88 | 5.82 | 3.22 | 5.90 | 3.77 | 0.00 |

| **Table 8-C** Pharmacophore feature angles between features X-Y-Z (°), | | | |
|---|---|---|---|
| X | *Y* | Z | *Angle (°)* |
| F1 | F2 | F3 | 110.39 |
| F1 | F2 | F4 | 45.38 |
| F1 | F2 | F5 | 72.94 |
| F1 | F2 | F6 | 61.46 |
| F1 | F2 | F7 | 92.58 |
| F1 | F2 | F8 | 53.87 |
| F1 | F2 | F9 | 103.51 |
| F1 | F3 | F4 | 6.61 |
| F1 | F3 | F5 | 30.48 |
| F1 | F3 | F6 | 21.30 |
| F1 | F3 | F7 | 22.60 |
| F1 | F3 | F8 | 11.59 |
| F1 | F3 | F9 | 30.18 |
| F1 | F4 | F5 | 65.03 |
| F1 | F4 | F6 | 29.85 |
| F1 | F4 | F7 | 12.32 |
| F1 | F4 | F8 | 18.96 |
| F1 | F4 | F9 | 32.52 |
| F1 | F5 | F6 | 7.99 |
| F1 | F5 | F7 | 28.34 |
| F1 | F5 | F8 | 21.01 |
| F1 | F5 | F9 | 35.98 |
| F1 | F6 | F7 | 49.67 |
| F1 | F6 | F8 | 48.90 |
| F1 | F6 | F9 | 81.99 |
| F1 | F7 | F8 | 20.48 |
| F1 | F7 | F9 | 52.40 |
| F1 | F8 | F9 | 88.17 |
| F2 | F3 | F4 | 15.65 |
| F2 | F3 | F5 | 27.53 |
| F2 | F3 | F6 | 20.78 |
| F2 | F3 | F7 | 26.84 |
| F2 | F3 | F8 | 5.82 |
| F2 | F3 | F9 | 17.47 |
| F2 | F4 | F5 | 58.21 |
| F2 | F4 | F6 | 24.99 |
| F2 | F4 | F7 | 14.75 |
| F2 | F4 | F8 | 6.62 |
| F2 | F4 | F9 | 20.61 |
| F2 | F5 | F6 | 14.35 |
| F2 | F5 | F7 | 22.35 |
| F2 | F5 | F8 | 25.41 |
| F2 | F5 | F9 | 18.14 |
| F2 | F6 | F7 | 37.84 |
| F2 | F6 | F8 | 55.34 |
| F2 | F6 | F9 | 48.68 |
| F2 | F7 | F8 | 22.45 |
| F2 | F7 | F9 | 34.01 |
| F2 | F8 | F9 | 54.05 |
| F3 | F4 | F5 | 58.15 |
| F3 | F4 | F6 | 22.97 |
| F3 | F4 | F7 | 5.71 |
| F3 | F4 | F8 | 18.05 |
| F3 | F4 | F9 | 27.40 |
| F3 | F5 | F6 | 24.17 |
| F3 | F5 | F7 | 5.20 |
| F3 | F5 | F8 | 47.65 |
| F3 | F5 | F9 | 29.69 |
| F3 | F6 | F7 | 5.20 |
| F3 | F6 | F8 | 87.80 |
| F3 | F6 | F9 | 63.56 |
| F3 | F7 | F8 | 143.73 |
| F3 | F7 | F9 | 137.11 |
| F3 | F8 | F9 | 59.09 |
| F4 | F5 | F6 | 73.80 |
| F4 | F5 | F7 | 93.94 |
| F4 | F5 | F8 | 54.64 |
| F4 | F5 | F9 | 89.42 |
| F4 | F6 | F7 | 142.01 |
| F4 | F6 | F8 | 61.79 |
| F4 | F6 | F9 | 118.85 |
| F4 | F7 | F8 | 17.94 |
| F4 | F7 | F9 | 41.28 |
| F4 | F8 | F9 | 127.89 |
| F5 | F6 | F7 | 146.38 |
| F5 | F6 | F8 | 118.68 |
| F5 | F6 | F9 | 104.33 |
| F5 | F7 | F8 | 39.90 |
| F5 | F7 | F9 | 31.63 |
| F5 | F8 | F9 | 63.74 |
| F6 | F7 | F8 | 27.72 |
| F6 | F7 | F9 | 30.48 |
| F6 | F8 | F9 | 53.57 |
| F7 | F8 | F9 | 55.96 |

Using the *Molecular Operating Environment (MOE),* 2022.02 Chemical Computing Group ULC, 1010 Sherbooke St. West, Suite #910, Montreal, QC, Canada, H3A 2R7, **2022** MOE docking using the pharmacophore algorithm for ligand placement and the GBVI/WSA dG scoring function, 117 compounds were assessed for their ability to dock in the predicted binding site using the pharmacophore model described above. To be treated as a successful docking in the predicted binding site, it is required that a molecule hits at least 4 features of the pharmacophore model shown in Figure 29 and has a half-maximal inhibitory concentration (IC₅₀) ≤ 1 µM.

Figure 30 shows a MCIM compound that conforms to the pharmacophore model and satisfies 7 out of the 9 annotation points determined to be important for binding to Complex I. Surprisingly, it was also found that out of the 117 compounds assessed for their ability to bind in the pharmacophore model, only 13 compounds had an IC₅₀ > 1 µM, which indicates that these compounds would fail to dock in the predicted binding sites. The remaining 104 compounds hit at least 4 of the pharmacophore features and are predicted to have an IC₅₀ ≤ 1 µM. Figure 31 shows representative compound CHMSA-02-A, which fulfils the pharmacophore model and is predicted to have a pAct (-Log(IC₅₀) of ~7.

### EXAMPLE 14 - Ultra-high throughput virtual screening (uHTVS)

Following building of the pharmacophore model, the inventors next wanted to identify additional NDUFS2 binders using this model.

The 3D model of the entire Complex 1 described in Example 12 (Figure 25) was validated by docking Q10 and active MCIM compounds. This allowed establishment of the bioactive conformation of the MCIM compounds when docked in the Q-tunnel of Complex I. This further allowed a structure-based hypothesis to be generated and to rationalise the structure-activity relationship (SAR) of the compounds and to build the pharmacophore model described in Example 13 and a QSAR model (Figure 29) for activity prediction. The QSAR model is a linear regression model which includes docking scores and parameters related to ligand energies and electrostatics.

For uHTVS, a library of compounds was screened against the pharmacophore model described in Example 13. The model identified 37.6 million compounds from the library of compounds which generally hit 3-6 of the pharmacophore features. Of these 37.6 million compounds, those up to a molecular weight of ~350 Da were then virtually docked in the 3D Complex I model without imposing pharmacophore constraints. 67,000 compounds were predicted to dock in Complex I and were retained for further screening.

The retained compounds were then filtered through a more theoretically rigorous docking method and evaluated with a QSAR model to predict biological activity of each compound (Figure 29). The GBVI/WSA ΔG forcefield-based scoring function (Naim *et al.* 2007) on the MOE dock (*Molecular Operating Environment (MOE),* 2022.02 Chemical Computing Group ULC, 1010 Sherbooke St. West, Suite #910, Montreal, QC, Canada, H3A 2R7) was used. Using the screening process described, 756 compounds were identified as potential drug candidates for targeting NDUFS2/NDUFS7 of Complex 1.

### References

A number of publications are cited above in order to more fully describe and disclose the invention and the state of the art to which the invention pertains. Full citations for these references are provided below. The entirety of each of these references is incorporated herein.
Ashcroft, T., Simpson, JM.,. and Timbrell, V. (1988) Simple method of estimating severity of pulmonary fibrosis on a numerical scale. J Clin Pathol. 41(4):467-70.
Badylak SF, Weiss DJ, Caplan A, Macchiarini P. Engineered whole organs and complex tissues. Lancet. 2012;379:943-952
Bantscheff, M., et al (2007) Quantitative mass spectrometry in proteomics: a critical review, Anal Bioanal Chem 389(4):1017-31
Bantscheff, M., et al (2012) Quantitative mass spectrometry in proteomics: critical review update from 2007 to the present. Anal Bioanal Chem 404(4):939-65
Bridges HR, Fedor JG, Blaza JN, Di Luca A, Jussupow A, Jarman OD, Wright JJ, Agip AA, Gamiz-Hernandez AP, Roessler MM, Kaila VRI, Hirst J. Structure of inhibitor-bound mammalian complex I. Nat Commun. 2020 Oct 16;11(1):5261.
Berardi S, Corrado A, Maruotti N, Cici D, Cantatore FP. Osteoblast role in the pathogenesis of rheumatoid arthritis. Mol Biol Rep. 2021 Mar;48(3):2843-2852. doi: 10.1007/s11033-021-06288-y. Epub 2021 Mar 27. PMID: 33774802; PMCID: PMC8060181.
Berthiaume F, Maguire TJ, Yarmush ML. Tissue engineering and regenerative medicine: History, progress, and challenges. Annu. Rev. Chem. Biomol. Eng. 2011;2:403-430
Cai S, Zhao M, Zhou B, Yoshii A, Bugg D, Villet O, Sahu A, Olson GS, Davis J, Tian R. Mitochondrial dysfunction in macrophages promotes inflammation and suppresses repair after myocardial infarction. J Clin Invest. 2022 Dec 8:E159498. doi: 10.1172/JCI159498. Epub ahead of print. PMID: 36480284.
Chassaing B, Aitken JD, Malleshappa M, Vijay-Kumar M. Dextran sulfate sodium (DSS)-induced colitis in mice. Curr Protoc Immunol. 2014 Feb 4;104:15.25.1-15.25.14. doi: 10.1002/0471142735.im1525s104. PMID: 24510619; PMCID: PMC3980572.
Chen P, Zhou G, Lin J, Li L, Zeng Z, Chen M, Zhang S. Serum Biomarkers for Inflammatory Bowel Disease. Front Med (Lausanne). 2020 Apr 22;7:123. doi: 10.3389/fmed.2020.00123. PMID: 32391365; PMCID: PMC7188783.
Chung, I., et al (2021) Cork-in-bottle mechanism of inhibitor binding to mammalian complex I. Science Advances, 14 May 2021, Vol 7, Issue 20
Diller, R.B.; Tabor, A.J. (2022) The Role of the Extracellular Matrix (ECM) in Wound Healing: A Review. Biomimetics 7, 87. https://doi.org/10.3390/biomimetics7030087
Eming SA, Martin P, Tomic-Canic M. Wound repair and regeneration: mechanisms, signaling, and translation. Sci Transl Med. 2014 Dec 3;6(265):265sr6. doi: 10.1126/scitranslmed.3009337. PMID: 25473038; PMCID: PMC4973620.
Fu XB. Repair cell first, then regenerate the tissues and organs. Mil Med Res. 2021 Jan 15;8(1):2. doi: 10.1186/s40779-021-00297-5. PMID: 33451321; PMCID: PMC7809804.
Franken, H., et al (2015) Thermal proteome profiling for unbiased identification of direct and indirect drug targets using multiplexed quantitative mass spectrometry. Nat. Protoc. 10(10): 1567-193.
Gutiérrez-Fernández, J., Kaszuba, K., Minhas, G.S. et al. Key role of quinone in the mechanism of respiratory complex I. Nat Commun 11, 4135 (2020).
Halgren T. A. Identifying and characterizing binding sites and assessing druggability. J. Chem. Inf. Model. 2009, 49, 377-389.
Hubner R-H et al (2008) Standardized quantification of pulmonary fibrosis in histological samples. Biotechniques, 44(4), 5-7-511
Izreig, S., et al (2020) Repression of LKB1 by miR-17~92 Sensitizes MYC-Dependent Lymphoma to Biguanide Treatment. Cell Reports Medicine 1(2)
Jin, S., Cordes, N. ATM controls DNA repair and mitochondria transfer between neighboring cells. Cell Commun Signal 17, 144 (2019)
Joh Y, Choi WS. Mitochondrial Complex I Inhibition Accelerates Amyloid Toxicity. Dev Reprod. 2017 Dec;21(4):417-424.
Kaspar S, Oertlin C, Szczepanowska K, Kukat A, Senft K, Lucas C, Brodesser S, Hatzoglou M, Larsson O, Topisirovic I, Trifunovic A. Adaptation to mitochondrial stress requires CHOP-directed tuning of ISR. Sci Adv. 2021 May 26;7(22):eabf0971. Doi: 10.1126/sciadv.abf0971. PMID: 34039602; PMCID: PMC8153728.Kurelac Ivana, et al. NDUFS3 knockout cancer cells and molecular docking reveal specificity and mode of action of anti-cancer respiratory complex I inhibitors. Open Biol. 12: 220198 (2022)
Krafts KP. (2010) Tissue repair: The hidden drama. Organogenesis. 6(4):225-33. doi: 10.4161/org.6.4.12555. PMID: 21220961; PMCID: PMC3055648.
LaMoia et al. (2022) Metformin, phenformin, and galegine inhibit complex IV activity and reduceglycerol-derived gluconeogenesis. PNAS 119(10); e2122287119.
Paul, W., and Sharma, CP., (2021),Chapter 1 - Tissue and organ regeneration: An introduction, Editor(s): Chandra P. Sharma, Regenerated Organs, Academic Press, 2021, Pages 3-9,
Li M, Hou Q, Zhong L, Zhao Y, Fu X. Macrophage Related Chronic Inflammation in Non-Healing Wounds. Front Immunol. 2021 Jun 16;12:681710. doi: 10.3389/fimmu.2021.681710. PMID: 34220830; PMCID: PMC8242337.
Liehn EA, Postea O, Curaj A, Marx N. Repair after myocardial infarction, between fantasy and reality: the role of chemokines. J Am Coll Cardiol. 2011 Nov 29;58(23):2357-62. doi: 10.1016/j.jacc.2011.08.034. PMID: 22115639.
Zerina Lokmic, James Musyoka, Timothy D. Hewitson, Ian A. Darby; Chapter three - Hypoxia and Hypoxia Signaling in Tissue Repair and Fibrosis (Editor: Kwang W. Jeon), International Review of Cell and Molecular Biology, Academic Press, Volume 296, 2012
Lousa I, Reis F, Beirão I, Alves R, Belo L, Santos-Silva A. New Potential Biomarkers for Chronic Kidney Disease Management-A Review of the Literature. Int J Mol Sci. 2020 Dec 22;22(1):43. doi: 10.3390/ijms22010043. PMID: 33375198; PMCID: PMC7793089.
Maghsoudloo, M., Azimzadeh Jamalkandi, S., Najafi, A. et al. Identification of biomarkers in common chronic lung diseases by co-expression networks and drug-target interactions analysis. Mol Med 26, 9 (2020). https://doi.org/10.1186/s10020-019-0135-9
Matthay MA, Zemans RL, Zimmerman GA, Arabi YM, Beitler JR, Mercat A, et al. Acute respiratory distress syndrome. Nat Rev Dis Prim. 2019;5(1):18.
Naim et al.; J. Chem. Inf. Model. 47 (2007) 122-133
Nallagangula KS, Nagaraj SK, Venkataswamy L, Chandrappa M. Liver fibrosis: a compilation on the biomarkers status and their significance during disease progression. Future Sci OA. 2017 Oct 5;4(1):FSO250. doi: 10.4155/fsoa-2017-0083. PMID: 29255622; PMCID: PMC5729599.
Peyrin-Biroulet, L (2020) Gastroenterology & Hepatology. 16(4) April 2020.
Rennert RC, Rodrigues M, Wong VW, Duscher D, Hu M, Maan Z, Sorkin M, Gurtner GC, Longaker MT. Biological therapies for the treatment of cutaneous wounds: Phase III and launched therapies. Expert Opin. Biol. Ther. 2013; 13:1523-1541.
Savitski, MM., et al (2015) Tracking cancer drugs in living cells by thermal profiling of the proteome. Science 346(6205).
Savu DI, Moisoi N. Mitochondria - Nucleus communication in neurodegenerative disease. Who talks first, who talks louder? Biochim Biophys Acta Bioenerg. 2022 Oct 1;1863(7):148588. doi: 10.1016/j.bbabio.2022.148588. Epub 2022 Jun 30. PMID: 35780856.
Schiller, J.; Zickermann, V. (2022) Binding of Natural Inhibitors to Respiratory Complex I.
Pharmaceuticals, 15, 1088. https://doi.org/10.3390/ph15091088
Tsogbadrakh B, et al (2018) HL156A, a novel pharmacological agent with potent adenosine-monophosphate-activated protein kinase (AMPK) activator activity ameliorates renal fibrosis in a rat unilateral ureteral obstruction model. PLoS One. 30;13(8):e0201692. doi: 10.1371/journal.pone.0201692. PMID: 30161162; PMCID: PMC6116936.
Searfoss GH, Paisley BM, Goldstein KM, Baker TK, Willy JA. The Integrated Stress Response Regulates Cell Health of Cardiac Progenitors. Toxicol Sci. 2019 Jan 1 ;167(1):202-210. doi: 10.1093/toxsci/kfy229. PMID: 30215789.
Sommer K, Wiendl M, Müller TM, Heidbreder K, Voskens C, Neurath MF, Zundler S. Intestinal Mucosal Wound Healing and Barrier Integrity in IBD-Crosstalk and Trafficking of Cellular Players. Front Med (Lausanne). 2021 Mar 23;8:643973. doi: 10.3389/fmed.2021.643973. PMID: 33834033; PMCID: PMC8021701.
Takeo M, Lee W, Ito M. Wound healing and skin regeneration. Cold Spring Harb Perspect Med. 2015 Jan 5;5(1):a023267. doi: 10.1101/cshperspect.a023267. PMID: 25561722; PMCID: PMC4292081.
Way SW, Popko B. Harnessing the integrated stress response for the treatment of multiple sclerosis. Lancet Neurol. 2016 Apr;15(4):434-43. doi: 10.1016/S1474-4422(15)00381-6. Epub 2016 Feb 10. PMID: 26873788; PMCID: PMC4792730.
Wright R, Truelove SR. Serial rectal biopsy in ulcerative colitis during the course of a controlled therapeutic trial of various diets. Am Dig Dis. (1966); 11 :847-857
Xiong, N., Long, X., Xiong, J., Jia, M., Chen, C., Huang, J., Ghoorah, D., Kong, X., Lin, Z. and Wang, T. Mitochondrial complex I inhibitor rotenone-induced toxicity and its potential mechanisms in Parkinson's disease models. Critical Reviews in Toxicology, 42:7. 11 May 2012.
Yap, T.A., Daver, N., Mahendra, M. et al. Complex I inhibitor of oxidative phosphorylation in advanced solid tumors and acute myeloid leukemia: phase I trials. Nat Med 29, 115-126 (2023). https://doi.org/10.1038/s41591-022-02103-8
Yoga, EG., et al (2021) Ubiquinone Binding and Reduction by Complex I-Open Questions and Mechanistic Implications. Front. Chem., 30 April 2021
Zieris A, Prokoph S, Levental KR, Welzel PB, Grimmer M, Freudenberg U, Werner C. FGF-2 and VEGF functionalization of starPEG-heparin hydrogels to modulate biomolecular and physical cues of angiogenesis. Biomaterials. 2010;31:7985-7994.

For standard molecular biology techniques, see Sambrook, J., Russel, D.W. Molecular Cloning, A Laboratory Manual. 3 ed. 2001, Cold Spring Harbor, New York: Cold Spring Harbor Laboratory Press.

## Claims

1. A mitochondrial complex I modulator (MCIM) compound, for use in the treatment of an inflammatory and/or progressive disease in a subject in need thereof, wherein the treatment comprises administering the compound to the subject to achieve tissue repair and/or disease control or regression.

2. The compound for the use according to claim 1, wherein the tissue repair and/or disease regression comprises an increased repair score and/or increased wound healing.

3. The compound for the use according to claim 1 or claim 2, wherein the tissue repair and/or disease regression comprises an increased cell count of reparative cells.

4. The compound for the use according to any one of the preceding claims, wherein the tissue repair and/or disease regression induces a restoration of anatomically normal tissue architecture.

5. The compound for the use according to claim 3, wherein the reparative cells comprise epithelial cells and/or mucus cells, and the inflammatory and/or progressive disease is an inflammatory bowel disease (IBD).

6. The compound for the use according to claim 3, wherein the reparative cells comprise alveolar type 1, type 2 cells and/or club cells, and the inflammatory and/or progressive disease is an interstitial lung disease and/or pulmonary fibrosis.

7. The compound for the use according to claim 3, wherein the reparative cells comprise oligodendrocyte precursor cells (OPCs), and the inflammatory and/or progressive disease is multiple sclerosis (MS).

8. The compound for the use according to claim 3, wherein the reparative cells comprise osteoblasts (OBs) and/or non-transformed fibroblasts, and wherein the inflammatory and/or progressive disease is RA.

9. The compound for the use according to any one of the preceding claims, wherein the tissue repair and/or disease regression comprises a reduction in cytokine production from proinflammatory myeloid cells.

10. The compound for the use according to any one of the preceding claims, wherein the tissue repair and/or disease regression comprises increased bone formation and/or decreased bone resorption, wherein the inflammatory and/or progressive disease is RA.

11. The compound for the use according to any one of the preceding claims, wherein the binding of the compound to complex I modulates complex I activity, wherein complex I modulation is determined by detecting a reduction in cellular O₂ consumption without a reduction of cell viability.

12. The compound for the use according to any one of the preceding claims, wherein the binding of the compound to complex I modulates complex I activity, leading to a reversible reduction in cell proliferation.

13. The compound for the use according to any one of the preceding claims, wherein the compound interacts with complex I at a binding site outside the Q tunnel.

14. The compound for the use according to any one of the preceding claims, wherein the binding site comprises one or more amino acid residues from NDUSF2 and/or NDUSF7.

15. The compound for use according claim 14, wherein the compound interacts with one or more amino acid residues in NDUFS2 selected from His92, Gly85, Tyr141, His88, Leu95, Asp193 and Phe458.

16. The compound for use according to claim 15, wherein the compound interacts with at least one amino acid residues in NDUFS2 selected from Tyr141, His92 and Asp139.

17. The compound for use according to any one of the preceding claims, wherein a 3D conformation of the compound as annotated by *Molecular Operating Environment (MOE)* 2022 unified annotation scheme comprises 4 or more pharmacophore features which conform to the pharmacophore model represented in Figure 29, and Tables 8-A, 8-B and 8-C.

18. The compound for use according to any of the preceding claims, wherein the compound is a compound, or a pharmaceutically acceptable salt, hydrate, or solvate thereof, of the following formula: 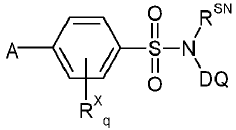 as defined in claim 1 of WO2010/032009.

19. The compound for use according to claim 18, wherein the compound is a compound selected from compounds of the following formulae, or a pharmaceutically acceptable salt, hydrate, or solvate thereof: 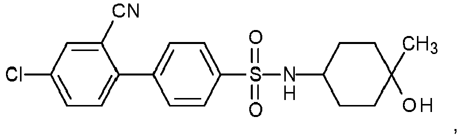 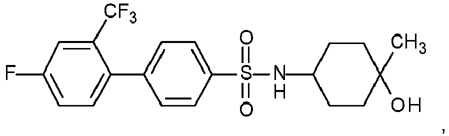 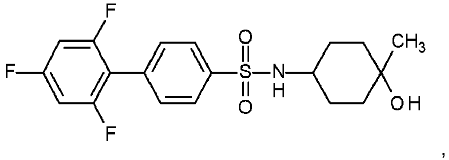 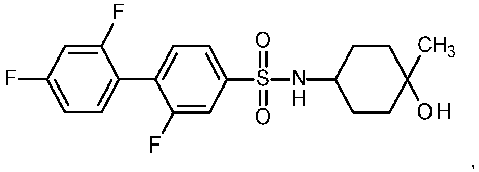 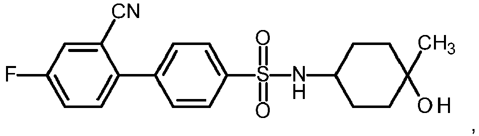 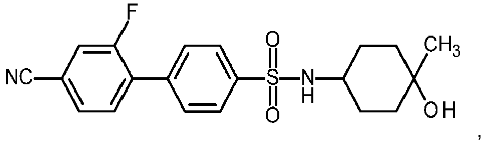 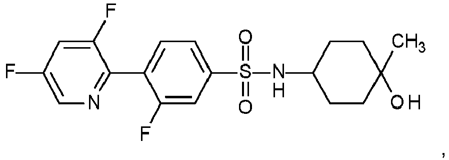 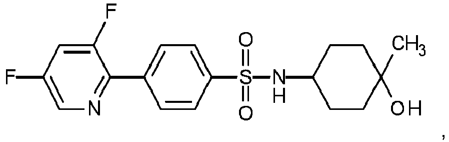 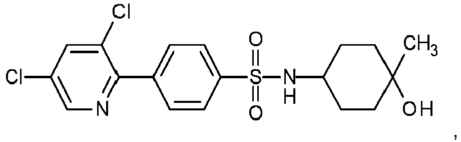 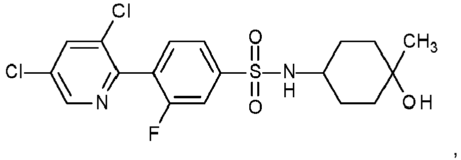 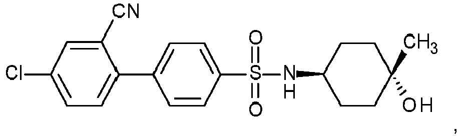 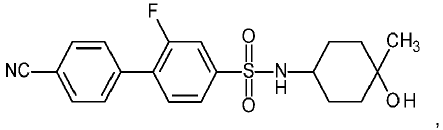 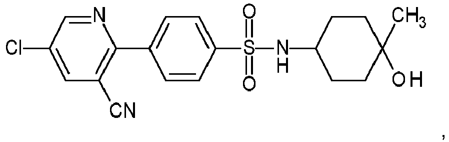 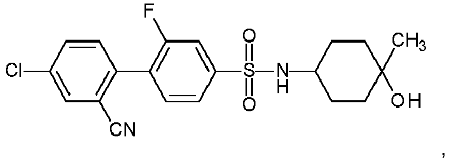 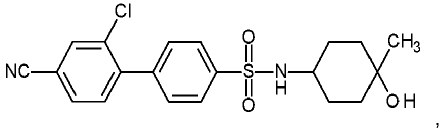 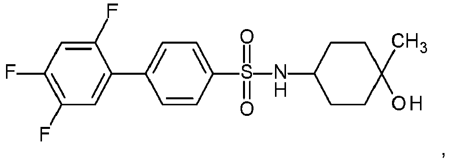 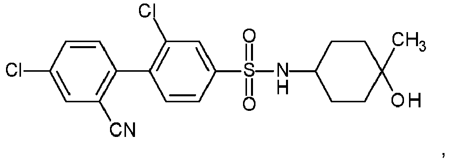 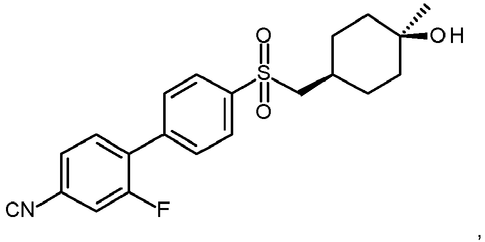 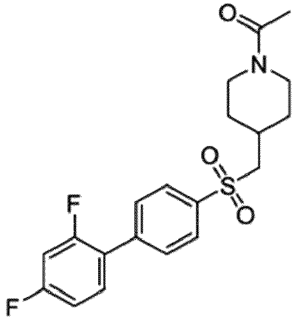 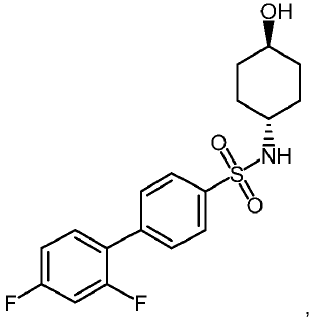 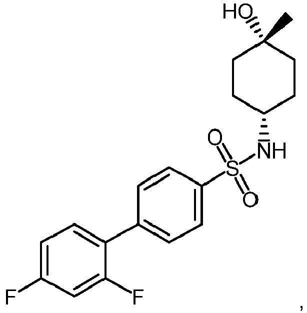 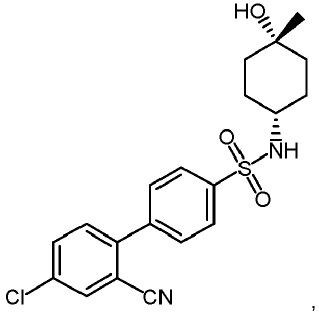 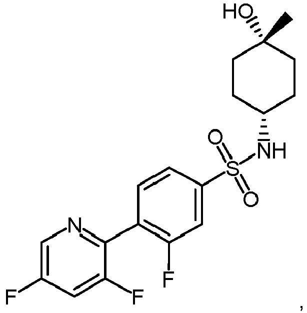 and

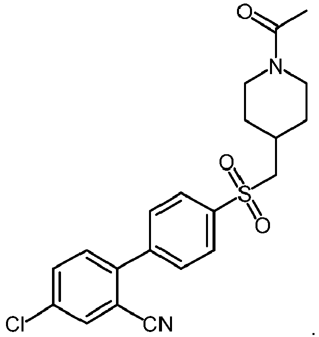

20. The compound for use according to any of the preceding claims, wherein the compound is a compound, or a pharmaceutically acceptable salt, hydrate, or solvate thereof, of the following formula: 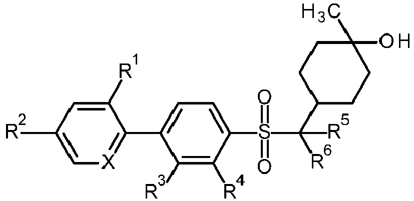 as defined in claim 1 of WO2020/035560 A1.

21. The compound for use according to any of the preceding claims, wherein the compound is a compound, or a pharmaceutically acceptable salt, hydrate, or solvate thereof, of the following formula: 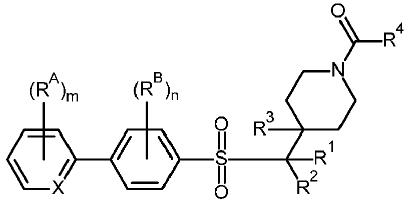 as defined in claim 1 of WO2020/212581 A1.
